# EUROPÄISCHE PATENTSCHRIFT

(11) **EP 2 686 303 B1**
(45) Veröffentlichungstag und Bekanntmachung des Hinweises auf die Patenterteilung: **20.01.2016**
(21) Anmeldenummer: 12707819.4
(22) Anmeldetag: 12.03.2012
(51) Int. Cl.: C07D 231/14, C07D 231/16, C07D 231/18, C07D 401/12, C07C 255/61, A01N 43/56, A01P 5/00, A01P 7/00, C07C 255/46, C07C 237/38

(54) **N-(3-CARBAMOYLPHENYL)-1H-PYRAZOL-5-CARBOXAMID- DERIVATE UND IHRE VERWENDUNG ZUR BEKÄMPFUNG VON TIERISCHEN SCHÄDLINGEN**
N-(3-CARBAMOYLPHENYL)-1H-PYRAZOLE-5-CARBOXAMIDE DERIVATIVES AND THEIR USE FOR COMBATING ANIMAL PESTS
DÉRIVÉS DE N-(3-CARBAMOYLPHÉNYL)-1H-PYRAZOLE-5-CARBOXAMIDE ET LEUR UTILISATION POUR COMBATTRE LES PARASITES ANIMAUX

(30) Priorität: 18.03.2011 EP 11158838; 18.03.2011 US 201161454134 P; 25.11.2011 EP 11190693
(43) Veröffentlichungstag der Anmeldung: 22.01.2014
(73) Patentinhaber: Bayer Intellectual Property GmbH, 40789 Monheim am Rhein (DE)
(72) Erfinder: MAUE, Michael, 40764 Langenfeld (DE); ADELT, Isabelle, 42781 Haan (DE); HEIL, Markus, 42799 Leichlingen (DE); JESCHKE, Peter, 51467 Bergisch Gladbach (DE); KAPFERER, Tobias, 4054 Basel (CH); MÜHLTHAU, Friedrich, August, 65779 Kelkheim-Fischbach (AT); SUDAU, Alexander, 40764 Langenfeld (DE); MALSAM, Olga, 51503 Rösrath (DE); LÖSEL, Peter, 51371 Leverkusen (DE); VOERSTE, Arnd, 50674 Köln (DE); GÖRGENS, Ulrich, 40882 Ratingen (DE)
(74) Vertreter: BIP Patents
(86) Internationale Anmeldenummer: PCT/EP2012/054299
(87) Internationale Veröffentlichungsnummer: WO 2012/126766

(56) Entgegenhaltungen:
- EP-A1- 1 911 751
- WO-A2-2010/051926

## Beschreibung

Die vorliegende Anmeldung betrifft neue Halogen-substituierte Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen herbizid wirksam sind (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Des Weiteren ist bekannt, daß bestimmte Halogen-substituierte Verbindungen insektizid wirksam sind (EP1911751).

Ferner ist bekannt, dass bestimmte Halogen-substituierte Verbindungen Cytokin-inhibitorische Aktivitäten aufweisen (WO 00/07980).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Halogen-substituierte Verbindungen, sowie deren *N*-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Ähnliche Verbindungen sind bereits aus WO 2010/051926 bekannt geworden.

Die erfindungsgemäßen Halogen-substituierten Verbindungen sind durch die allgemeine Formel (I) 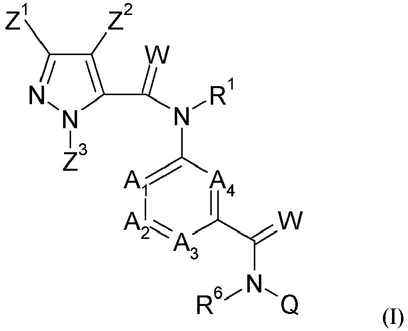 definiert, in denen
- R¹: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoₗycarbonyl, Cyan-C₁-C₂-alkyl, Aryl(C₁-C₃)-alkyl, Heteroaryl(C₁-C₃)-alkyl,
die chemische Gruppierung
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthlo, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₁-C₆alkylamino, *N*-C₁-C₆-Alkylaminocarbonyl, *N*-C₃-C₆-Cycloalkylaminocarbonyl oder (C₁-C₃-Alkoxy)carbonyl, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- R⁶: für Wasserstoff, gegebenenfalls substituiertes C₁-C₆-Alkyl, Aryl(C₁-C₃)-alkyl, Heteroaryl(C₁-C₃)-alkyl, C₂-C₆-Alkenyl, C₂-C₆-Alkinyl, C₃-C₆-Cycloalkyl, (C₁-C₃-Alkyl)-C₃-C₆-cycloalkyl und (C₃-C₆-Cycloalkyl)-C₁-C₃-alkyl, C₁-C₆-Alkylcarbonyl, C₁-C₆-Alkoxycarbonyl steht;
- Q: für 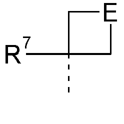 steht;
- E: für eine Bindung, -CH₂-, S, SO, SO₂, -S-CH₂-, -SO-CH₂-, -SO₂-CH₂-, -CH₂-S-CH₂-, -CH₂-SO-CH₂-, -CH₂-SO₂-CH₂-, -S-CH₂-CH₂-, -SO-CH₂-CH₂-, -SO₂-CH₂-CH₂-, -NR⁶-CH₂-, -CH₂-NR⁶-CH₂- steht;
- R⁷: für Cyano und C(=S)NH₂ steht;
- Z¹: für ein gegebenenfalls substituiertes C₁-C₆-Halogenalkyl und C₃-C₆-Cycloalkyl, C₃-C₆-Halogencycloalkyl, und
- Z²: für Halogen, Cyano, Nitro oder ein gegebenenfalls substituiertes C₁-C₆-Halogenalkyl, C₁-C₆-Alkylthio, C₁-C₆-Halogenalkylthlo, C₁-C₆-Alkylsulfinyl, C₁-C₆-Halogenalkylsulfinyl, C₁-C₆-Alkylsulfonyl, C₁-C₆-Halogenalkylsulfonyl, und
- Z³: für Wasserstoff oder ein gegebenenfalls substituiertes C₁-C₆-Alkyl, C₁-C₄-Alkenyl, C₁-C₄-Alkinyl, C₁-C₆-Halogenalkyl stehen;

Bevorzugt sind Verbindungen der Formel (I) 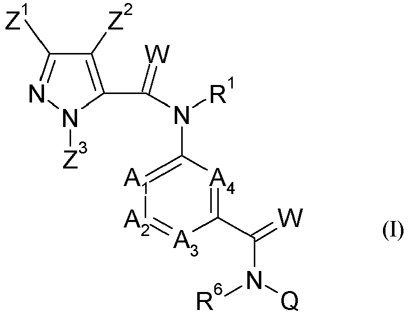 definiert, in denen
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemische Gruppierung
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R² und R⁵: unabhängig voneinander für Wasserstoff, Methyl, Fluor und Chlor stehen und
- R³ und R⁴: unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl stehen; wobei
- W: für Sauerstoff steht,
- R⁶: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
- Q: für 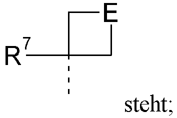
- E: für eine Bindung und -CH₂- steht;
- R⁷: für Cyano und C(=S)NH₂ steht;
- Z¹: für Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1-methyl-cyclopropyl, und
- Z²: für Halogen, Cyan, Nitro, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylthio, Methylsulfinyl, Methylsulfonyl, Ethylthio, Ethylsulfinyl, Ethylsulfonyl, Trifluormethylthio, Trifluormethylsulfinyl, Trifluormethylsulfonyl und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, Ethinyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl stehen;

Weitere besonders bevorzugte Verbindungen sind Verbindungen der allgemeinen Formel (I), in denen
- Z¹: für Trifluormethyl, 1-Chlor-cyclopropyl, 1-Fluor-cyclopropyl oder Pentafluorethyl steht,
- Z²: für Trifluormethyl, Nitro, Methylthio, Methylsulfinyl, Methylsulfonyl, Fluor, Chlor, Brom oder Iod steht,
- Z³: für Methyl, Ethyl, n-Propyl oder Wasserstoff steht,
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht,
- A¹, A² und A⁴: für CH steht,
- A₃: für CR⁴ und
- R⁴: für Fluor, Chlor, Brom oder Iod steht
- R⁶: für Wasserstoff, Methyl, Ethyl, n-Propyl, Isopropyl, n-Butyl, Isobutyl, s-Butyl, t-Butyl steht,
- W: für Sauerstoff steht und
- Q: für 1-Cyancyclopropyl steht.

Insbesondere sind weitere Verbindungen bevorzugt, in denen
- Z¹: für Trifluormethyl, 1-Chlor-cyclopropyl, 1-Fluor-cyclopropyl oder Pentafluorethyl steht,
- Z²: für Trifluormethyl, Chlor oder steht,
- Z³: für Methyl steht,
- R¹: für Wasserstoff, Methyl, Ethyl, steht,
- A¹ A² und A⁴: für CH steht,
- A₃: für CR⁴ und
- R⁴: für Chlor steht
- R⁶: für Wasserstoff, Methyl, Ethyl steht,
- W: für Sauerstoff steht und
- Q: für 1-Cyancyclopropyl steht.

Die Erfindung umfasst ferner neue Verbindungen der allgemeinen Formeln (IVa), (IVb), (Va), (Vb) als bevorzugte Ausgangsverbindungen für die Synthese der Verbindungen der allgemeinen Formel (I).

Die Verbindungen der allgemeinen Formeln (IVa) und (IVb) sind bevorzugte Ausführungsformen für die Vorstufen der allgemeinen Formel (IV) gemäß beispielsweiße Reaktionsschemata 1, 2 und 3. Die Herstellung der Verbindungen der allgemeinen Formel (I) erfolgt unter anderem bevorzugt mit diesen Verbindungen. Üblicherweise werden die Verbindungen der allgemeinen Formel (IVb) durch Reduktion in die Verbindungen (IVa) überführt.

Die Verbindungen (IVa) und (IVb) sind durch die folgenden allgemeinen Formeln definiert, 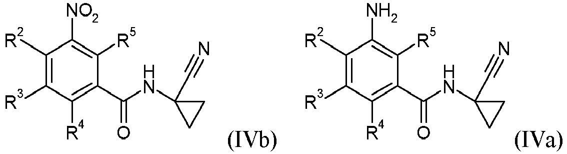 in denen
- R², R³, R⁴ und R⁵: unabhängig voneinander für Wasserstoff, Halogen, Cyan, Nitro, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthlo, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₁-C₆alkylamino, *N*-C₁-C₆-Alkylaminocarbonyl, *N*-C₃-C₆-Cycloalkylaminocarbonyl oder (C₁-C₃-Alkoxy)carbonyl stehen.

Bevorzugt sind Verbindungen der allgemeinen Formeln (IVa), (IVb), in denen
- R² und R⁵: für Wasserstoff oder Halogen stehen, und
- R³ und R⁴: für Wasserstoff, Halogen, Cyan, Nitro, C₁-C₆-Alkyl, C₁-C₆-Halogenalkyl, C₃-C₆-Cycloalkyl, C₃-C₆-Halogencycloalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl stehen.

Besonders bevorzugt sind Verbindungen der allgemeinen Formeln (IVa), (IVb), in denen
- R²: für Wasserstoff oder Fluor, und
- R³: für Wasserstoff, Fluor, Chlor, Brom, Iod, Cyan, Nitro, Methyl, Ethyl, n-Propyl i-Propyl, n-Butyl, i-Butyl, s-Butyl, t-Butyl, Cyclopropyl, 1-Chlor-cyclopropyl, 1-Fluor-cyclopropyl, Methylthio, Trifluormethylthio, Methylsulfinyl, Trifluormethylsulfinyl, Methylsulfonyl, Trifluormethylsulfonyl, und
- R⁴: für Wasserstoff, Fluor, Chlor, Brom, Iod, Cyan, Nitro, Methyl, Ethyl, n-Propyl i-Propyl, n-Butyl, i-Butyl, s-Butyl, t-Butyl, Cyclopropyl, 1-Chlor-cyclopropyl, 1-Fluor-cyclopropyl, Methylthio, Trifluormethylthio, Methylsulfinyl, Trifluormethylsulfinyl, Methylsulfonyl, Trifluormethylsulfonyl, und
- R⁵: für Wasserstoff stehen.

Ganz besonders bevorzugt sind Verbindungen der allgemeinen Formeln (IVa), (IVb), in denen
- R² und R⁵: für Wasserstoff, und
- R³: für Wasserstoff, Fluor, Chlor, Brom, Iod, Cyan, Methyl, Ethyl, Methylthio, Trifluormethylthio, Methylsulfinyl, Trifluormethylsulfinyl, Methylsulfonyl, Trifluormethylsulfonyl, und
- R⁴: für Fluor, Chlor, Brom, Iod, Methyl, und
- R⁵: für Wasserstoff stehen.

Die Verbindungen der allgemeinen Formeln (Va) und (Vb) sind bevorzugte Ausführungsformen für die Vorstufen der allgemeinen Formel (V) gemäß beispielsweiße Reaktionsschemata 1, 2 und 8. Die Herstellung der Verbindungen der allgemeinen Formel (I) erfolgt unter anderem bevorzugt mit diesen Verbindungen.

Die Verbindungen (Va) und (Vb) sind durch die folgenden allgemeinen Formeln definiert, in denen 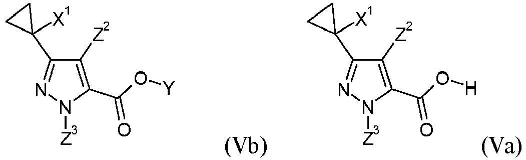
- X¹: für Halogen, Cyan und C₁-C₄-Haloalkyl, und
- Z²: für Halogen, Cyano, Nitro oder ein gegebenenfalls substituiertes C₁-C₆-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, und
- Z³: für Wasserstoff oder ein gegebenenfalls substituiertes C₁-C₆-Alkyl, C₁-C₄-Alkenyl, C₁-C₄-Alkinyl, C₁-C₆-Halogenalkyl, und
- Y: für ein gegebenenfalls substituiertes C₁-C₆-Alkyl stehen.

Bevorzugt sind Verbindungen der allgemeinen Formeln (Va) und (Vb), in denen
- X¹: für Fluor, Chlor, Brom, Cyan und C₁-C₂-Haloalkyl, und
- Z²: für Halogen, Cyan, Nitro, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylthio, Methylsulfinyl, Methylsulfonyl, Ethylthio, Ethylsulfinyl, Ethylsulfonyl, Trifluormethylthio, Trifluormethylsulfinyl, Trifluormethylsulfonyl, und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, Ethinyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, und
- Y: steht für ein gegebenenfalls substituiertes C₁-C₆-Alkyl stehen.

Besonders bevorzugt sind Verbindungen der allgemeinen Formeln (Va) und (Vb), in denen
- X¹: für Fluor, Chlor, Trifluormethyl oder Pentafluorethyl, und
- Z²: für Trifluormethyl, Nitro, Methylthio, Methylsulfinyl, Methylsulfonyl, Fluor, Chlor, Brom oder Iod, und
- Z³: für Methyl, Ethyl, n-Propyl oder Wasserstoff, und
- Y: für Methyl, Ethyl, n-Propyl, i-Propyl, n-Butyl oder t-Butyl stehen.

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, Halogenalkyloxy, Halogenalkylthio, Halogenalkylsulfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;

Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen, wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylthio" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt für Alkylthiogruppen mit 1 bis 4 Kohlenstoffatomen. Die erfindungsgemäßen Alkylthiogruppen können mit einem oder mehreren, gleichen oder verschiedenen Resten substituiert sein.

Beispiele für Halogenalkylthioalkyle, d.h. mit Halogen substituierte Alkylthiogruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht "*N,N-*Dialkylamino-carbonyl" für geradkettiges oder verzweigtes *N,N-*Dialkylaminocarbonyl mit vorzugsweise 1 bis 6 Kohlenstoffatomen oder 1 bis 4 Kohlenstoffatomen im Alkylteil, wie beispielsweise *N,N*-Dimethylamino-carbonyl, N.N-Diethylamino-carbonyl, *N,N-*Di(n-propylamino)-carbonyl, *N,N-*Di-(isopropylamino)-carbonyl und *N,N-*Di-(s-butylamino)-carbonyl. Die erfindungsgemäßen *N,N-*Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N,N-*Dialkylamino-carbonyl, substituiertes Amino, wie Acylamino, Mono- und *N,N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und *N,N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N,N*-Dialkenylamino-carbonyl, Mono- und Dialkinylaminocarbonyl, Mono- und *N,N-*Dialkenylamino, Mono- und *N,N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise

Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N*-Mono-alkyl-amino, *N,N-*Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoylamino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N-*Diethylamino, *N,N*-Di-n-propylamino, *N,N*-Diisopropylamino oder *N,N*-Dibutylamino), *N*-Mono- oder *N,N*-Dialkoxyalkylaminogruppen (z.B. *N*-Methoxymethylamino, *N*-Methoxyethylamino, *N,N*-Di-(methoxymethyl)-amino oder *N,N*-Di-(methoxyethyl)-amino), *N*-Mono- und *N,N*-Diarylamino, wie gegebenenfalls substituierte Aniline, Acylamino, *N,N-*diacylamino, *N*-Alkyl-*N*-arylamino, *N*-Alkyl-*N-*acylamino sowie gesättigte *N-*Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mir gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylthio, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl" Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N'*-Dibenzylethylen-diammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können t-Amine, wie beispielsweise manche der erfindungsgemäßen Verbindungen, *N*-Oxide bilden, welche ebenfalls erfindungsgemäße Salze darstellen.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der allgemeinen Formel (I) können mit anderen insektiziden, nematiziden akariziden oder antimikrobiellen Wirkstoffen gemischt oder gemeinsam angewendet werden. In diesen Mischungen oder gemeinsamen Anwendungen treten synergistische Wirkungen auf, d.h. die beobachtete Wirkung dieser Mischungen oder gemeinsamen Anwendungen ist höher als die Summe der Wirkungen der Einzelwirkstoffe innerhalb dieser Anwendungen. Beispiele für solche Misch- bzw. Kombinationspartner sind:
(1) Acetylcholinesterase (AChE) Inhibitoren, wie beispielsweise
   Carbamate, z.B. Alanycarb (II-1-1), Aldicarb (II-1-2), Bendiocarb (II-1-3), Benfuracarb (II-1-4), Butocarboxim (II-1-5), Butoxycarboxim (II-1-6), Carbaryl (II-1-7), Carbofuran (II-1-8), Carbosulfan (11-1-9), Ethiofencarb (II-1-10), Fenobucarb (II-1-11), Formetanate (II-1-12), Furathiocarb (II-1-13), Isoprocarb (II-1-14), Methiocarb (II-1-15), Methomyl (II-1-16), Metolcarb (II-1-17), Oxamyl (II-1-18), Pirimicarb (II-1-19), Propoxur (II-1-20), Thiodicarb (II-1-21), Thiofanox (II-1-22), Triazamate (II-1-23), Trimethacarb (II-1-24), XMC (II-1-25) und Xylylcarb (II-1-26); oder
   Organophosphate, z.B. Acephate (II-1-27), Azamethiphos (II-1-28), Azinphos-ethyl (II-1-29), Azinphosmethyl (II-1-30), Cadusafos (II-1-31), Chlorethoxyfos (II-1-32), Chlorfenvinphos (II-1-33), Chlormephos (II-1-34), Chlorpyrifos (II-1-35), Chlorpyrifos-methyl (II-1-36), Coumaphos (II-1-37), Cyanophos (11-1-38), Demeton-S-methyl (II-1-39), Diazinon (II-1-40), Dichlorvos/DDVP (II-1-41), Dicrotophos (II-1-42), Dimethoate (II-1-43), Dimethylvinphos (II-1-44), Disulfoton (II-1-45), EPN (II-1-46), Ethion (II-1-47), Ethoprophos (II-1-48), Famphur (II-1-49), Fenamiphos (II-1-50), Fenitrothion (II-1-51), Fenthion (11-1-52), Fosthiazate (II-1-53), Heptenophos (II-1-54), Imicyafos (II-1-55), Isofenphos (II-1-56), Isopropyl O-(methoxyaminothio-phosphoryl) salicylat (II-1-57), Isoxathion (II-1-58), Malathion (II-1-59), Mecarbam (II-1-60), Methamidophos (II-1-61), Methidathion (II-1-62), Mevinphos (II-1-63), Monocrotophos (11-1-64), Naled (II-1-65), Omethoate (II-1-66), Oxydemeton-methyl (II-1-67), Parathion (II-1-68), Parathion-methyl (II-1-69), Phenthoate (II-1-70), Phorate (II-1-71), Phosalone (II-1-72), Phosmet (II-1-73), Phosphamidon (II-1-74), Phoxim (II-1-75), Pirimiphos-methyl (II-1-76), Profenofos (II-1-77), Propetamphos (II-1-78), Prothiofos (II-1-79), Pyraclofos (II-1-80), Pyridaphenthion (II-1-81), Quinalphos (II-1-82), Sulfotep (II-1-83), Tebupirimfos (II-1-84), Temephos (II-1-85), Terbufos (II-1-86), Tetrachlorvinphos (II-1-87), Thiometon (II-1-88), Triazophos (II-1-89), Triclorfon (II-1-90) und Vamidothion (II-1-91).
(2) GABA-gesteuerte Chlorid-Kanal-Antagonisten, wie beispielsweise
   Cyclodien-organochlorine, z.B. Chlordane (II-2-1) und Endosulfan (II-2-2); oder
   Phenylpyrazole (Fiprole), z.B. Ethiprole (II-2-3) und Fipronil (II-2-4).
(3) Natrium-Kanal-Modulatoren / Spannungsabhängige Natrium-Kanal-Blocker, wie beispielsweise
   Pyrethroide, z.B. Acrinathrin (II-3-1), Allethrin (II-3-2), d-cis-trans Allethrin (II-3-3), d-trans Allethrin (II-3-4), Bifenthrin (II-3-5), Bioallethrin (II-3-6), Bioallethrin S-cyclopentenyl Isomer (II-3-7), Bioresmethrin (II-3-8), Cycloprothrin (II-3-9), Cyfluthrin (II-3-10), beta-Cyfluthrin (II-3-11), Cyhalothrin (II-3-12), lambda-Cyhalothrin (II-3-13), gamma-Cyhalothrin (II-3-14), Cypermethrin (II-3-15), alpha-Cypermethrin (II-3-16), beta-Cypermethrin (II-3-17), theta-Cypermethrin (II-3-18), zeta-Cypermethrin (II-3-19), Cyphenothrin [(1R)-trans-Isomere] (II-3-20), Deltamethrin (II-3-21), Empenthrin [(EZ)-(1R)-Isomere) (II-3-22), Esfenvalerate (II-3-23), Etofenprox (II-3-24), Fenpropathrin (II-3-25), Fenvalerate (II-3-26), Flucythrinate (II-3-27), Flumethrin (II-3-28), tau-Fluvalinate (II-3-29), Halfenprox (II-3-30), Imiprothrin (II-3-31), Kadethrin (II-3-32), Permethrin (II-3-33), Phenothrin [(1R)-trans-Isomer) (II-3-34), Prallethrin (II-3-35), Pyrethrine (pyrethrum) (II-3-36), Resmethrin (II-3-37), Silafluofen (II-3-38), Tefluthrin (II-3-39), Tetramethrin (II-3-40), Tetramethrin [(1R)- Isomere)] (II-3-41), Tralomethrin (II-3-42) und Transfluthrin (II-3-43); oder
   DDT (II-3-44); oder Methoxychlor (II-3-45).
(4) Nikotinerge Acetylcholin-Rezeptor (nAChR) Agonisten, wie beispielsweise
   Neonikotinoide, z.B. Acetamiprid (II-4-1), Clothianidin (II-4-2), Dinotefuran (II-4-3), Imidacloprid (11-4-4), Nitenpyram (II-4-5), Thiacloprid (II-4-6) und Thiamethoxam (II-4-7); oder
   Nikotin (II-4-8).
(5) Nikotinerge Acetylcholin-Rezeptor (nAChR) allosterische Aktivatoren, wie beispielsweise
   Spinosine, z.B. Spinetoram (II-5-1) und Spinosad (II-5-2).
(6) Chlorid-Kanal-Aktivatoren, wie beispielsweise
   Avermectine/Milbemycine, z.B. Abamectin (II-6-1), Emamectin-benzoat (II-6-2), Lepimectin (II-6-3) und Milbemectin (II-6-4).
(7) Juvenilhormon-Imitatoren, wie beispielsweise
   Juvenilhormon-Analoge, z.B. Hydroprene (II-7-1), Kinoprene (II-7-2) und Methoprene (II-7-3); oder Fenoxycarb (II-7-4); oder Pyriproxyfen (II-7-5).
(8) Wirkstoffe mit unbekannten oder nicht spezifischen Wirkmechanismen, wie beispielsweise
   Alkylhalide, z.B. Methylbromid (II-8-1) und andere Alkylhalide; oder
   Chloropicrin (II-8-2); oder Sulfurylfluorid (II-8-3); oder Borax (II-8-4); oder Brechweinstein (II-8-5).
(9) Selektive Fraßhemmer, z.B. Pymetrozine (II-9-1); oder Flonicamid (II-9-2).
(10) Milbenwachstumsinhibitoren, z.B. Clofentezine (II-10-1), Hexythiazox (II-10-2) und Diflovidazin (II-10-3); oder
   Etoxazole (II-10-4).
(11) Mikrobielle Disruptoren der Insektendarmmembran, z.B. *Bacillus thuringiensis* Subspezies israelensis (II-11-1), *Bacillus sphaericus* (II-11-2), *Bacillus thuringiensis* Subspezies aizawai (II-11-3), *Bacillus thuringiensis* Subspezies kurstaki (II-11-4), *Bacillus thuringiensis* Subspezies tenebrionis (II-11-5) und BT Pflanzenproteine: Cry1Ab, Cry1Ac, Cry1Fa, Cry2Ab, mCry3A, Cry3Ab, Cry3Bb, Cry34/35Ab1 (II-11-6).
(12) Inhibitoren der oxidativen Phosphorylierung, ATP-Disruptoren, wie beispielsweise Diafenthiuron (II-12-1); oder
   Organozinnverbindungen, z.B. Azocyclotin (II-12-2), Cyhexatin (II-12-3) und Fenbutatin-oxid (II-12-4); oder
   Propargite (II-12-5); oder Tetradifon (II-12-6).
(13) Entkoppler der oxidativen Phoshorylierung durch Unterbrechung des H-Protongradienten, wie beispielsweise Chlorfenapyr (II-13-1), DNOC (II-13-2) und Sulfluramid (II-13-3).
(14) Nikotinerge Acetylcholin-Rezeptor-Antagonisten, wie beispielsweise Bensultap (II-14-1), Cartaphydrochlorid (II-14-2), Thiocyclam (II-14-3) und Thiosultap-Natrium (II-14-4).
(15) Inhibitoren der Chitinbiosynthese, Typ 0, wie beispielsweise Bistrifluron (II-15-1), Chlorfluazuron (II-15-2), Diflubenzuron (II-15-3), Flucycloxuron (II-15-4), Flufenoxuron (II-15-5), Hexaflumuron (11-15-6), Lufenuron (II-15-7), Novaluron (II-15-8), Noviflumuron (II-15-9), Teflubenzuron (11-15-10) und Triflumuron (II-15-11).
(16) Inhibitoren der Chitinbiosynthese, Typ 1, wie beispielsweise Buprofezin (II-16-1).
(17) Häutungsstörende Wirkstoffe, Dipteran, wie beispielsweise Cyromazine (II-17-1).
(18) Ecdyson-Rezeptor Agonisten, wie beispielsweise Chromafenozide (II-18-1), Halofenozide (II-18-2), Methoxyfenozide (II-18-3) und Tebufenozide (II-18-4).
(19) Oktopaminerge Agonisten, wie beispielsweise Amitraz (II-19-1).
(20) Komplex-III-Elektronentransportinhibitoren, wie beispielsweise Hydramethylnon (II-20-1); oder Acequinocyl (II-20-2); oder Fluacrypyrim (II-20-3).
(21) Komplex-1-Elektronentransportinhibitoren, beispielsweise
   METI-Akarizide, z.B. Fenazaquin (II-21-1), Fenpyroximate (II-21-2), Pyrimidifen (II-21-3), Pyridaben (II-21-4), Tebufenpyrad (11-21-5) und Tolfenpyrad (11-21-6); oder
   Rotenone (Derris) (11-21-7).
(22) Spannungsabhängige Natriumkanal-Blocker, z.B. Indoxacarb (11-22-1); oder Metaflumizone (II-22-2).
(23) Inhibitoren der Acetyl-CoA-Carboxylase, wie beispielsweise
   Tetron- und Tetramsäurederivate, z.B. Spirodiclofen (II-23-1), Spiromesifen (II-23-2) und Spirotetramat (II-23-3).
(24) Komplex-IV-Elektronentransportinhibitoren, wie beispielsweise
   Phosphine, z.B. Aluminiumphosphid (11-24-1), Calciumphosphid (II-24-2), Phosphin (II-24-3) und Zinkphosphid (II-24-4); oder
   Cyanid (II-24-5).
(25) Komplex-II-Elektronentransportinhibitoren, wie beispielsweise Cyenopyrafen (II-25-1).
(28) Ryanodinrezeptor-Effektoren, wie beispielsweise
   Diamide, z.B. Chlorantraniliprole (II-28-1) und Flubendiamide (II-28-2).

Weitere Wirkstoffe mit unbekanntem Wirkmechanismus, wie beispielsweise Amidoflumet (11-29-1), Azadirachtin (II-29-2), Benclothiaz (II-29-3), Benzoximate (II-29-4), Bifenazate (II-29-5), Bromopropylate (II-29-6), Chinomethionat (II-29-7), Cryolite (II-29-8), Cyantraniliprole (Cyazypyr) (II-29-9), Cyflumetofen (11-29-10), Dicofol (II-29-11), Diflovidazin (11-29-12), Fluensulfone (11-29-13), Flufenerim (11-29-14), Flufiprole (11-29-15), Fluopyram (11-29-16), Fufenozide (11-29-17), Imidaclothiz (II-29-18), Iprodione (11-29-19), Pyridalyl (11-29-20), Pyrifluquinazon (11-29-21) und Iodmethan (11-29-22); desweiteren Präparate auf Basis von Bacillus firmus (1-1582, BioNeem, Votivo) (11-29-23) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (11-29-24) (bekannt aus WO2005/077934), 4-{[(6-Brompyrid-3-yl)methyl](2-fluorethyl)amino}furan-2(5H)-on (11-29-25) (bekannt aus WO2007/115644), 4-{[(6-Fluorpyrid-3-yl)methyl](2,2-difluorethyl)amino}furan-2(5H)-on (11-29-26) (bekannt aus WO2007/115644), 4-{[(2-Chlor-1,3-thiazol-5-yl)methyl](2-fluorethyl)amino}furan-2(5H)-on (II-29-27) (bekannt aus WO2007/115644), 4-{[(6-Chlorpyrid-3-yl)methyl](2-fluorethyl)amino}furan-2(5H)-on (11-29-28) (bekannt aus WO2007/115644), 4-{[(6-Chlorpyrid-3-yl)methyl](2,2-difluorethyl)amino}furan-2(5H)-on (11-29-29) (bekannt aus WO2007/115644), 4-{[(6-Chlor-5-fluorpyrid-3-yl)methyl](methyl)amino}furan-2(5H)-on (11-29-30) (bekannt aus WO2007/115643), 4-{[(5,6-Dichlorpyrid-3-yl)methyl](2-fluorethyl)amino}furan-2(5H)-on (11-29-31) (bekannt aus WO2007/115646), 4-{[(6-Chlor-5-fluorpyrid-3-yl)methyl](cyclopropyl)amino}furan-2(5H)-on (11-29-32) (bekannt aus WO2007/115643), 4-{[(6-Chlorpyrid-3-yl)methyl](cyclopropyl)amino}furan-2(5H)-on (11-29-33) (bekannt aus EP-A-0 539 588), 4-{[(6-Chlorpyrid-3-yl)methyl](methyl)amino}furan-2(5H)-on (11-29-34) (bekannt aus EP-A-0 539 588), {[1-(6-Chlorpyridin-3-yl)ethyl](methyl)oxido-λ⁴-sulfanyliden}cyanamid (II-29-35) (bekannt aus WO2007/149134) und seine Diastereomere {[(1R)-1-(6-Chlorpyridin-3-yl)ethyl](methyl)olido-λ⁴-sulfanyliden}cyanamid (A) (11-29-36) und {[(1S)-1-(6-Chlorpyridin-3-yl)ethyl](methyl)oxido-λ⁴-sulfanyliden}cyanamid (B) (11-29-37) (ebenfalls bekannt aus WO2007/149134) sowie Sulfoxaflor (11-29-38) (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) (11-29-39) und [(S)-Methyl(oxido){(1S)-1-[6-(trifluormethyl)pyridin-3-yl]ethyl}-λ⁴-sulfanyliden]cyanamid (A2) (11-29-40), bezeichnet als Diastereomerengruppe A (bekannt aus WO 2010/074747, WO 2010/074751), [(R)-Methyl(olido){(1S)-1-[6-(trifluormethyl)pyridin-3-yl]ethyl}-λ⁴-sulfanyliden]cyanamid (B1) (11-29-41) und [(S)-Methyl(oxido){(1R)-1-[6-(trifluormethyl)pyridin-3-yl]ethyl}-λ⁴-sulfanyliden]cyanamid (B2) (II-29-42), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (11-29-43) (bekannt aus WO2006/089633), 3-(4'-Fluor-2,4-dimethylbiphenyl-3-yl)-4-hydroxy-8-oxa-1-azaspiro[4.5]dec-3-en-2-on (11-29-44) (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (II-29-45) (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (11-29-46) (bekannt aus WO2008/066153), 2-Cyan-3-(difluormethoxy)-N,N-dimethylbenzolsulfonamid (11-29-47) (bekannt ausWO2006/056433), 2-Cyan-3-(difluormethoxy)-N-methylbenzolsulfonamid (11-29-48) (bekannt aus WO2006/100288), 2-Cyan-3-(difluormethoxy)-N-ethylbenzolsulfonamid (11-29-49) (bekannt aus WO2005/035486), 4-(Difluormethoxy)-N-ethyl-N-methyl-1,2-benzothiazol-3-amin-1,1-dioxid (11-29-50) (bekannt aus WO2007/057407), N-[1-(2,3-Dimethylphenyl)-2-(3,5-dimethylphenyl)ethyl]-4,5-dihydro-1,3-thiazol-2-amin (II-29-51) (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (11-29-52) (bekannt aus WO2003/106457), 3-(2,5-Dimethylphenyl)-4-hydroxy-8-methoxy-1,8-diazaspiro[4.5]dec-3-en-2-on (II-29-53) (bekannt aus WO2009/049851), 3-(2,5-Dimethylphenyl)-8-methoxy-2-oxo-1,8-diazaspiro[4.5]dec-3-en-4-yl-ethylcarbonat (11-29-54) (bekannt aus WO2009/049851), 4-(But-2-in-1-yloxy)-6-(3,5-dimethylpiperidin-1-yl)-5-fluorpyrimidin (11-29-55) (bekannt aus WO2004/099160), (2,2,3,3,4,4,5,5-Octafluorpentyl)(3,3,3-trifluorpropyl)malononitril (11-29-56) (bekannt aus WO2005/063094), (2,2,3,3,4,4,5,5-Octafluorpentyl)(3,3,4,4,4-pentafluorbutyl)malononitril (II-29-57) (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (11-29-58) (bekannt aus WO2007/040280), 2-Ethyl-7-methoxy-3-methyl-6-[(2,2,3,3-tetrafluor-2,3-dihydro-1,4-benzodiolin-6-yl)oxy]chinolin-4-yl-methylcarbonat (II-29-59) (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (11-29-60) (bekannt aus JP2008/110953), PF1364 (CAS-Reg.Nr. 1204776-60-2) (11-29-61) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (11-29-62) (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzo-nitril (11-29-63) (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (11-29-64) (bekannt aus WO2005/085216), 4-{[(6-Chlorpyridin-3-yl)methyl](cyclopropyl)amino}-1,3-oxazol-2(5H)-on (11-29-65), 4-{[(6-Chlorpyridin-3-yl)methyl](2,2-difluorethyl)amino}-1,3-oxazol-2(5H)-on (II-29-66), 4-{[(6-Chlorpyridin-3-yl)methyl](ethyl)amino}-1,3-oxazol-2(5H)-on (II-29-67), 4-{[(6-Chlorpyridin-3-yl)methyl](methyl)amino}-1,3-oxazol-2(5H)-on (11-29-68) (alle bekannt aus WO2010/005692), NNI-0711 (11-29-69) (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (11-29-70) (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (11-29-71) (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (11-29-72) (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazin-carboxylat (11-29-73) (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (II-29-74) (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (11-29-75) (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxy-imidazo[1,2-a]pyridin (11-29-76) (bekannt aus WO2007/101369), 2-{6-[2-(5-Fluorpyridin-3-yl)-1,3-thiazol-5-yl]pyridin-2-yl}pyrimidin (11-29-77) (bekannt aus WO2010/006713), 2-{6-[2-(Pyridin-3-yl)-1,3-thiazol-5-yl]pyridin-2-yl}pyrimidin (11-29-78) (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (11-29-79) (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (11-29-80) (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (II-29-81) (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (11-29-82) (bekannt aus WO2010/069502) und (1E)-N-[(6-Chlorpyridin-3-yl)methyl]-N'-cyan-N-(2,2-difluorethyl)ethanimidamid (11-29-83) (bekannt aus WO2008/009360).

Antimikrobiell wirkende Verbindungen:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methyl-ethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethan-amid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).

Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Schließlich wurde gefunden, dass die neuen Verbindungen der Formel (I) sich bei guter Pflanzenverträglichkeit, günstiger Warmblütertoxizität und guter Umweltverträglichkeit vor allem zur Bekämpfung von tierischen Schädlingen, insbesondere von Arthropoden, Insekten, Spinnentieren, Helminthen, Nematoden und Mollusken, die in der Landwirtschaft, in den Forsten, im Vorrats- und Materialschutz sowie auf dem Hygienesektor oder im Bereich der Tiergesundheit vorkommen, eignen. Gleichfalls können die erfindungsgemäßen Verbindungen im Bereich der Tiergesundheit verwendet werden, beispielsweise zur Bekämpfung von Endo- und/oder Ectoparasiten.

Die erfindungsgemäßen Verbindungen können als Mittel zur Bekämpfung tierischer Schädlinge, vorzugsweise als Pflanzenschutzmittel eingesetzt werden. Sie sind gegen normal sensible und resistente Arten sowie gegen alle oder einzelne Entwicklungsstadien wirksam.

Die erfindungsgemäßen Verbindungen können in allgemein bekannte Formulierungen überführt werden. Solche Formulierungen enthalten im Allgemeinen von 0,01 bis 98 Gew.-% Wirkstoff, vorzugsweise von 0,5 bis 90 Gew.-%.

Die erfindungsgemäßen Verbindungen können in ihren handelsüblichen Formulierungen sowie in den aus diesen Formulierungen bereiteten Anwendungsformen in Mischung mit anderen Wirkstoffen oder Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise von 0,00001 bis 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Coccidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Außerdem wurde gefunden, daß die erfindungsgemäßen Verbindungen eine hohe insektizide Wirkung gegen Insekten zeigen, die technische Materialien zerstören. Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene-und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam.

Unter Pflanzen werden alle Pflanzenarten, Pflanzensorten und Pflanzenpopulationen, wie erwünschte und unerwünschte Wildpflanzen oder Kulturpflanzen verstanden. Erfindungsgemäß zu behandelnde Kulturpflanzen sind Pflanzen, die natürlich vorkommen, oder solche, die durch konventionelle Züchtungs- und Optimierungsmethoden oder durch biotechnologische und gentechnologische Methoden oder durch Kombinationen der vorgenannten Methoden erhalten wurden. Der Begriff Kulturpflanze umfasst selbstverständlich auch transgene Pflanzen.

Unter Pflanzensorten versteht man Pflanzen mit neuen Eigenschaften, sogenannten Traits, die sowohl durch konventionelle Züchtung, durch Mutagenese oder durch rekombinante DNA-Techniken oder einer Kombination hieraus gezüchtet worden sind. Dies können Sorten, Rassen, Bio- und Genotypen sein.

Unter Pflanzenteilen werden alle oberirdischen und unterirdischen Teile und Organe der Pflanzen, wie Spross, Blatt, Blüte und Wurzel verstanden, insbesondere Blätter, Nadeln, Stängel, Stämme, Blüten, Fruchtkörper, Früchte, Samen, Wurzeln, Knollen und Rhizome. Der Begriff Pflanzenteilen umfasst weiterhin Erntegut sowie vegetatives und generatives Vermehrungsmaterial, wie z.B. Stecklinge, Knollen, Rhizome, Ableger und Samen bzw. Saatgut.

In einer erfindungsgemäßen Ausführungsform werden natürlich vorkommende oder durch konventionelle Züchtungs- und Optimierungsmethoden (z.B. Kreuzung oder Protoplastenfusion) erhaltene Pflanzenarten und Pflanzensorten sowie deren Pflanzenteile behandelt.

In einer weiteren erfindungsgemäßen Ausführungsform werden transgene Pflanzen, die durch gentechnologische Methoden gegebenenfalls in Kombination mit konventionellen Methoden erhalten wurden und deren Teile behandelt.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insecticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD^{®} (zum Beispiel Mais, Baumwolle, Sojabohnen), KnockOut^{®} (zum Beispiel Mais), BiteGard^{®} (zum Beispiel Mais), BT-Xtra^{®} (zum Beispiel Mais), StarLink^{®} (zum Beispiel Mais), Bollgard^{®} (Baumwolle), Nucotn^{®} (Baumwolle), Nucotn 33B^{®} (Baumwolle), NatureGard^{®} (zum Beispiel Mais), Protecta^{®} und NewLeaf^{®} (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready^{®} (Glyphosatetoleranz, zum Beispiel Mais, Baumwolle, Sojabohne), Liberty Link^{®} (Phosphinotricintoleranz, zum Beispiel Raps), IMI^{®} (Imidazolinontoleranz) und SCS^{®} (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield^{®} angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

### Zu diesen Parasiten gehören:

Aus der Ordnung der Anoplurida z.B. Haematopinus spp., Linognathus spp., Pediculus spp., Phthirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;

Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;

Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;

Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp. (z.B. Suppella longipalpa);

Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen können nach üblichen, dem Fachmann bekannten Methoden hergestellt werden.

Im Reaktionsschema 1 ist das allgemeine Darstellungsverfahren A für die erfindungsgemäßen Verbindungen (I) abgebildet.

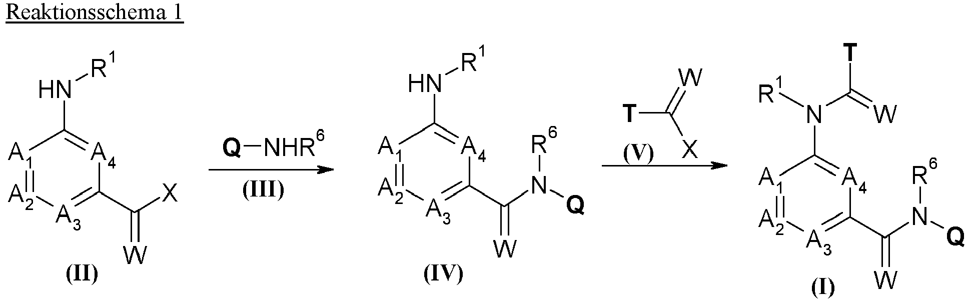

Die Reste A₁-A₄,, Q, W, R¹ und R⁶ haben die oben beschriebenen Bedeutungen. T steht für die Gruppierung, 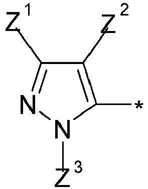 wobei die Reste Z¹, Z² und Z³ die oben genannten Bedeutung haben und der Stern die Anknüpfung an die Gruppierung C=W darstellt. X steht für eine beliebige Abgangsgruppe.

Erfindungsgemäße Verbindungen des Typs (I) können durch die Umsetzung von Aminen der allgemeinen Struktur **(IV)** mit aktivierten Carbonsäurederivaten der allgemeinen Struktur **(V)** dargestellt werden. Die Reaktion kann mit oder ohne Lösungsmittel durchgeführt werden. In diesem Schritt kann ebenfalls eine geeignete Base zum Einsatz kommen.

Im Allgemeinen ist es vorteilhaft, den ersten Reaktionsschritt des erfindungsgemäßen Darstellungsverfahrens A gegebenenfalls in Gegenwart eines geeigneten Verdünnungsmittels und gegebenenfalls in Gegenwart eines geeigneten basischen Reaktionshilfsmittels durchzuführen.

Verdünnungsmittel werden vorteilhaft in einer solchen Menge eingesetzt, dass das Reaktionsgemisch während des ganzen Verfahrens gut rührbar bleibt.

Als Lösungsmittel kann jedes Lösungsmittel verwendet werden, das die Reaktion nicht beeinträchtigt, wie zum Beispiel Wasser. In Frage kommen aromatische Kohlenwasserstoffe wie Benzol oder Toluol; halogenierte Kohlenwasserstoffe wie Dichlormethan, Chloroform oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid; Nitrile wie Acetonitril; und andere inerte Lösungsmittel wie 1,3-Dimethyl-2-imidazolidinon; die Lösungsmittel können allein oder in Kombination von zwei oder mehr eingesetzt werden.

Als Base kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat; Alkalimetallhydride wie Natriumhydrid; Alkalimetallalkoholate wie Natriummethanolat und Natriumethanolat. Diese Basen können in Verhältnissen von 0.01 bis 5.0 Moläquivalenten in Bezug auf **(IV)** und **(V)** eingesetzt werden. Des Weiteren kann auch Silber(1)-cyanid als Base und Aktivator eingesetzt werden [Journal of Organic Chemistry. 1992, 57, 4394-4400; Journal of Medicinal Chemistry 1992, 35, 3905-3918; Journal of Organic Chemistry 2003, 68, 1843-1851]

Die geeignete Reaktionstemperatur liegt im Bereich von -20°C bis zum Siedepunkt des jeweiligen Lösungsmittels und die Reaktionsdauer liegt je nach Wahl der Reaktanden, Lösungsmittel und Reaktionstemperatur zwischen wenigen Minuten bis 96 Stunden.

Zyklische Carbonsäurehalogenide, wie sie durch die allgemeine Struktur **(V)** repräsentiert werden, können einfach durch die Umsetzung einer heterozyklischen Carbonsäure mit Halogenierungsreagenzien wie Thionylchlorid, Thionylbromid, Phosphorylchlorid, Oxalylchlorid, Phosphortrichlorid, etc. hergestellt werden [Houben-Weyl, 1952, Bd. VIII, S.463 ff.].

Die Darstellung von Carboxamiden repräsentiert durch die Formel **(I)** kann aber auch unter der Verwendung von Kupplungsreagenzien wie Dicyclohexylcarbodiimid und Additiven wie 1-Hydroxybenzotriazol durchgeführt werden [Chem. Ber. 1970, 788]. Verwendbar sind ferner Kupplungsreagenzien wie 1-Ethyl-3-(3-dimethylaminopropyl)carbodiimid, 1,1'-Carbonyl-1H-imidazol und ähnliche Verbindungen.

Als Kupplungsreagenzien zur Durchführung des Dartellungsverfahrens finden alle, die zur Herstellung einer Ester- oder Amidbindung geeignet sind (vgl. z. B. Bodansky et al., Peptide Synthesis, 2nd ed., Wiley & Sons, New York, 1976; Gross, Meienhofer, The Peptide: Analysis, Synthesis, Biology (Academic Press, New York, 1979), Verwendung.

Des Weiteren können auch gemischte Anhydride zur Darstellung von **(I)** verwendet werden [J. Am. Chem. Soc 1967, 5012]. Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden.

Verbindungen der allgemeinen Struktur **(IV)** können durch die Umsetzung eines Amins der allgemeinen Struktur **(III)** mit aktivierten Carbonsäurederivaten der allgemeinen Struktur **(II)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von **(I).**

Das Reaktionsschema 2 zeigt das allgemeine Darstellungsverfahren B für die Synthese der erfindungsgemäßen Verbindungen **(I).**

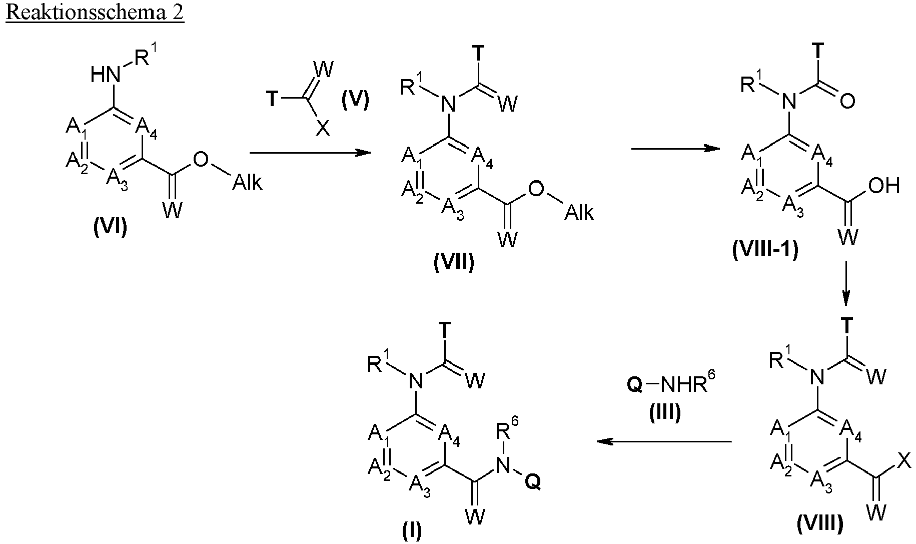

Die Reste A₁-A₄, Q, R¹, R⁶ und W haben die oben beschriebenen Bedeutungen. X steht für eine beliebige Abgangsgruppe und Alk für einen Alkylrest, wie z.B. Methyl oder Ethyl. T steht für die Gruppierung, 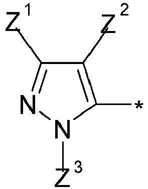 wobei die Reste Z¹, Z² und Z³ die oben genannten Bedeutung haben und der Stern die Anknüpfung an die Gruppierung C=W darstellt.

Erfindungsgemäße Verbindungen des Typs **(I)** können durch die Umsetzung eines Amins der allgemeinen Struktur **(III)** mit aktivierten Carbonsäurederivaten der allgemeinen Struktur (VIII) dargestellt werden. Dabei gelten dieselben Bedingungen für die Wahl des Lösungsmittels, der Reaktionsbedingungen, der Reaktionsdauer und der Reagenzien wie bei der im Darstellungsverfahren A beschriebenen Umsetzung von **(IV)** und **(V)** zu **(I).**

Die Darstellung von aktivierten Carbonsäurederivaten der allgemeinen Struktur **(VIII)** kann durch eine zweistufige Synthese aus den entsprechenden Carbonsäureestern der allgemeinen Struktur **(VII)** erfolgen. Im ersten Schritt wird die in Form eines Esters geschützte Carbonsäurefunktion (O-Alk) der Verbindung **(VII)** in Abhängigkeit des verwendeten Alkylesters mit einem passenden Reagenz entschützt [Greene's protective groups in organic synthesis, 4. Edition, P. G. M. Wuts, T. W. Greene, John Wiley & Sons, Inc., Hoboken, New Jersey] und die daraus resultierende freie Hydroxygruppe der Säurefunktion von **(VIII-1)** in eine Abgangsgruppe X überführt. Dabei können dieselben Verfahren eingesetzt werden, die bereits in der Darstellung von **(V)** beschrieben wurden. Verbindungen der allgemeinen Struktur **(VII)** können durch die Umsetzung von Aminen der allgemeinen Struktur **(VI)** mit aktivierten Carbonsäurederivaten der allgemeinen Struktur **(V)** dargestellt werden. Dabei gelten dieselben Bedingungen für die Wahl des Lösungsmittels, der Reaktionsbedingungen, der Reaktionsdauer, der Reagenzien wie bei der im Darstellungsverfahren A beschriebenen Synthese von **(I).**

Das Reaktionsschema 3 zeigt das allgemeine Darstellungsverfahren C für die Synthese der erfindungsgemäßen Verbindungen **(I).**

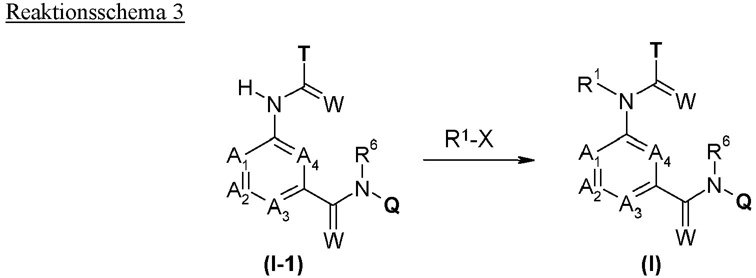

Die Reste A₁-A₄, Q, R⁶ und W haben die oben beschriebenen Bedeutungen. X steht für eine beliebige Abgangsgruppe wie z.B. Chlor, Brom oder Iod. T steht für die Gruppierung, 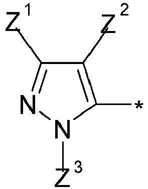 wobei die Reste Z¹, Z² und Z³ die oben genannten Bedeutung haben und der Stern die Anknüpfung an die Gruppierung C=W darstellt. R¹ steht für die oben beschriebenen Reste mit Ausnahme von Wasserstoff.

Die Verbindungen der allgemeinen Struktur (I) mit R1 ≠ H können ausgehend von Verbindungen der allgemeinen Struktur (1-1) dargestellt werden. Dabei kann auf literaturbekannte Verfahren zurück gegriffen werden [R1 = gegebenenfalls subst. Alkyl & (Het)aryl-alkyl: WO2008/061688; Journal of Heterocyclic Chemistry 1995, 32(3), 835-839; WO2011/029808; WO2010/020432; US2010/0152192; WO2010/101949; WO2010/043377, Medicinal Chemistry Letters 2011, 2(8), 632-637; Journal of Heterocyclic Chemistry 1977, 14(7), 1263-1265; WO2011/020193; WO2008/121602; WO2006/074924; WO2006/065794 | R1 = gegebenenfalls subst. Alkylcarbony & (Het)aryl(alkyl)carbonyl: WO2010/015545; Journal of the Chemical Society, Perkin Transactions 1 2002, (2), 257-274; US 7951828 | R1 = gegebenenfalls subst. Alkoxycarbonyl, & (Het)aryl(alkyl)oxycarbonyl: WO2011/112731; WO2009/027393; Journal of Organic Chemistry 2011, 76(8), 2502-2520 | R1 = gegebenenfalls subst. Alkinyl: Synthesis 2007, (18), 2920-2923; Tetrahedron 2006, 62(16), 3856-3871; Journal ofthe American Chemical Society 2006, 128(14), 4586-4587; Chemical Communications (Cambridge, United Kingdom) 2010, 46(8), 1269-1271; WO2009/027393 | R1 = gegebenenfalls subst. Alkenyl: Organic Chemistry: An Indian Journal 2010, 6(1), 52-55; European Journal of Organic Chemistry 2009, (1), 72-84; WO2006/067444; WO2005/049585].

Das Reaktionsschema 4 zeigt das allgemeine Darstellungsverfahren D für die Synthese der erfindungsgemäßen Verbindungen **(I).**

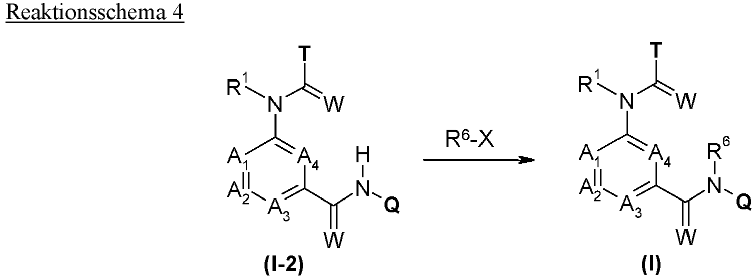

Die Reste A₁-A₄, Q, R¹ und W haben die oben beschriebenen Bedeutungen. X steht für eine beliebige Abgangsgruppe wie z.B. Chlor, Brom oder Iod. T steht für die Gruppierung, 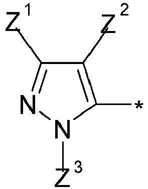 wobei die Reste Z¹, Z² und Z³ die oben genannten Bedeutung haben und der Stern die Anknüpfung an die Gruppierung C=W darstellt. R⁶ steht für die oben beschriebenen Reste.

Die Verbindungen der allgemeinen Struktur (I) ausgehend von Verbindungen der allgemeinen Struktur (I-1) dargestellt werden. Dabei kann auf die im Darstellungsverfahren C genannnten Verfahren zurück gegriffen werden.

Als Vorläufer für die Substanzen der allgemeinen Formel (II) können Verbindungen der allgemeinen Formel (II-1), (II-1-1) und (II-2) verwendet werden. Substanzen der allgemeinen Formel **(II-1-1)** sind allgemein bekannte Verbindungen der organischen Chemie, die nach etablierten Syntheseverfahren erhalten werden können. Mögliche Synthesewege der zyklischen Aminocarbonsäuren der allgemeinen Formel **(II-1-1)** sind im Reaktionsschema 5 abgebildet.

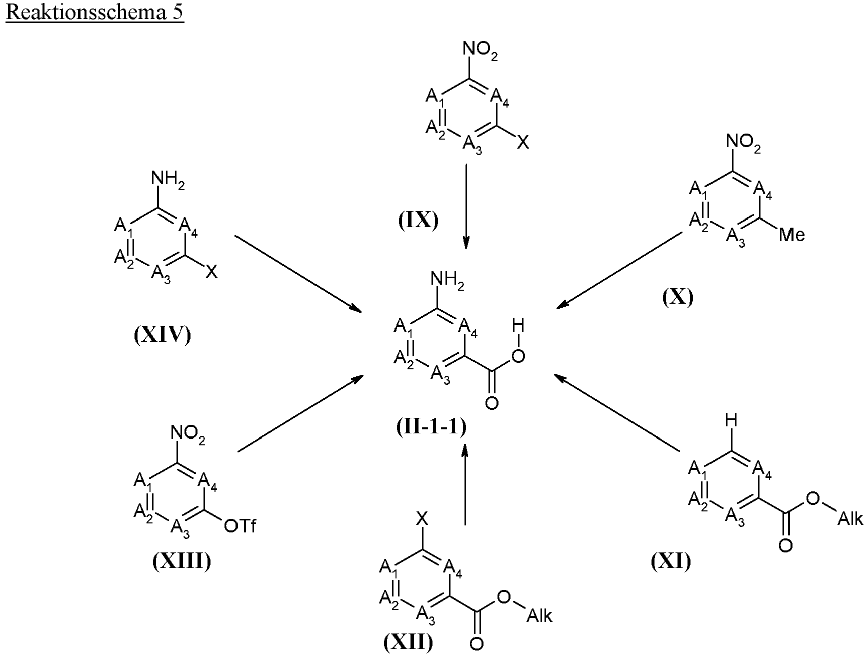

Als Edukte zur Darstellung von Aminocarbonsäuren der allgemeinen Struktur **(II-1-1)** können z.B. halogenierte (hetero)aromatische Nitro- bzw. Aminoverbindungen dienen, wie sie durch die Formeln **(IX)** und **(XIV)** repräsentiert werden. Dabei wird die Abgangsgruppe X durch eine Cyanogruppe ersetzt und diese anschließend sauer oder basisch hydrolysiert. Der Halogen-Cyano-Austausch kann z.B. durch eine nucleophile Substitution am Aromaten mit einer Cyanidspezies wie z.B. Natriumcyanid [US 4766219] oder auch durch eine Kupfer-vermittelte Reaktion [Journal of Antibiotics 1994, 47(12), 1456-65] erfolgen.

Im Fall der Nitroverbindungen **(IX, X, und XIII)** kann anschließend noch eine Reduktion der Nitrofunktion in eine Aminofunktion erfolgen. Geeignete Verfahren für solche Reduktionen sind Hydrierungen und Metall-vermittelte Reaktionen wie z.B. Zinn(II)-chlorid, Eisenpulver, Zinkpulver und diesen ähnliche Verbindungen.

Hydrierungen können in einem geeigneten Lösungsmittel in Anwesenheit eines Katalysators unter Wasserstoffatmosphäre (Normaldruck oder Hochdruck). Als Katalysatoren können Palladiumkatalysatoren wie z.B. Palladium auf Kohle, Nickelkatalysatoren wie Raney-Nickel, Kobaltkatalysatoren, Rutheniumkatalysatoren, Rhodiumkatalysatoren, Platinkatalysatoren und diesen ähnliche Verbindungen verwendet werden. Geeignete Lösungsmittel sind Wasser, Alkohole wie Methanol und Ethanol, aromatische Kohlenwasserstoffe wie Benzol und Toluol, offenkettige oder zyklische Ether wie Diethylether, Dioxan und Tetrahydrofuran sowie Ester wie Essigsäureethylester. Die Reduktionen können in einem Druckbereich von 1 bar bis 100 bar durchgeführt werden, wobei die Temperatur zwischen -20°C und dem Siedepunkt des eingesetzten Lösungsmittels variieren kann. Je nach Reaktionsbedingungen liegen die Reaktionszeiten zwischen wenigen Minuten und 96 Stunden.

Die Metall-vermittelten Reduktionen wie z.B. mit Zinn(II)-chlorid können nach einem in Organic Syntheses Coll. Vol. (III), 453 beschriebenen Verfahren durchgeführt werden.

Des Weiteren können (hetero)aromatische Aminocarbonsäuren der allgemeinen Struktur **(II-1-1)** auch aus den entsprechenden Methylvorläufern des Typs (X) durch Oxidation dargestellt werden. Für solche Oxidationen geeignete Oxidationsmittel sind z.B. Kaliumpermanganat, Natriumdichromat, Chromtrioxid und diesen ähnliche Verbindungen[Tetrahedron Letters 1995, 36(25), 4369-72; Bioorganic & Medicinal Chemistry Letters 2007, 17(4), 1043-1046]. Ebenso können für solche Oxidationen auch enzymatische Verfahren verwendet werden [PCT Int. Appl., 9502061]. Die anschließend notwendige Reduktion der Nitrofunktion kann analog zu den oben beschriebenen Verfahren durchgeführt werden.

Eine weitere Methode zur Darstellung von (hetero)aromatischen Aminocarbonsäuren der allgemeinen Struktur **(II-1-1)** ist die Nitrierung von Carbonsäurevorläufern repräsentiert durch die Formel **(XI)** bzw. **(XII)** und die anschließende Reduktion der Nitrofunktion. Die Nitrierungen können nach literaturbekannten Verfahren durchgeführt werden [Justus Liebigs Annalen der Chemie 1958, 611, 194-205; Organikum, Wiley-VCH, 22. Auflage, 358ff]. Die anschließend notwendige Reduktion der Nitrofunktion kann analog zu den oben beschriebenen Verfahren durchgeführt werden.

Des Weiteren können (hetero)aromatische Aminocarbonsäuren der allgemeinen Struktur **(II-1-1)** aus den entsprechenden (Hetero)aryl-Triflaten des Typs **(XIII)** mit Hilfe eines Palladium-katalysierten Verfahrens hergestellt werden [Synthesis 2006, (4), 594-596].

Verbindungen der allgemeinen Formel (II-1) können ausgehend von Verbindungen der allgemeinen Formel (II-1-1) nach etablierten Syntheseverfahren hergestellt werden. Ein möglicher Syntheseweg der zyklischen Aminocarbonsäuren der allgemeinen Formel **(II-1)** ist im Reaktionsschema 6 abgebildet.

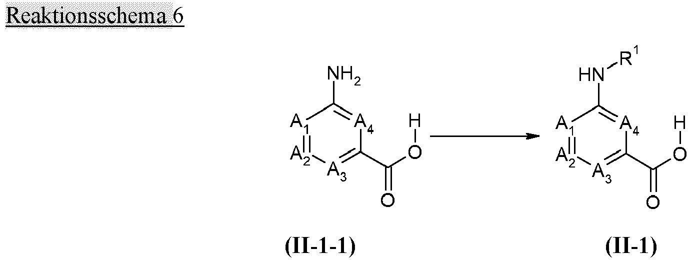

Die Reste A₁-A₄ haben die oben beschriebenen Bedeutungen. Der Rest R¹ steht für die oben beschriebenen Reste mit Ausnahme von Wasserstoff.

Die literaturbekannte Umsetzung von (II-1-1) in (II-1) kann unter anderem via reduktiver Aminierung [Bioorganic & Medicinal Chemistry Letters 2005, 15(21), 4752-4756; WO2010-142402; US2010-0324056] oder direkter Alkylierung [Tetrahedron Letters 1977, (9), 771-774; Journal of the American Chemical Society 1997, 119(9), 2315-2316; Journal of Combinatorial Chemistry 2006, 8(6), 834-840] erfolgen.

Verbindungen der allgemeinen Formel **(II-2)** können ausgehend von Verbindungen der allgemeinen Formel (II-1) nach etablierten Syntheseverfahren hergestellt werden. Ein möglicher Syntheseweg der zyklischen Aminocarbonsäuren der allgemeinen Formel **(II-2)** ist im Reaktionsschema 7 abgebildet.

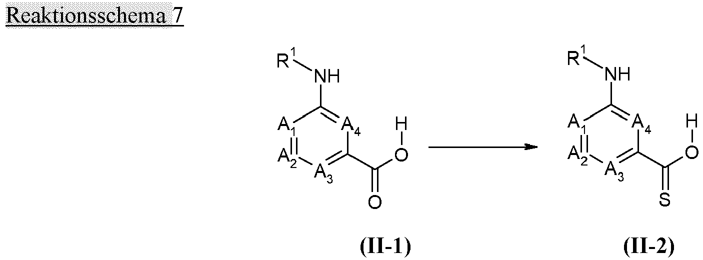

Die Reste A₁-A₄ und R¹ haben die oben beschriebenen Bedeutungen.

Die Umsetzung von Verbindungen der allgemeinen Formel (II-1) in Verbindungen der alllgemeinen Formel (II-2) kann analog literaturbekannter Umsetzungen erfolgen [US2009-0023798; WO2009-044200; WO2010-085352].

Mögliche Synthesen der heterozyklischen Carbonsäurederivate der allgemeinen Formel (V) sind im Reaktionsschema 8 abgebildet.

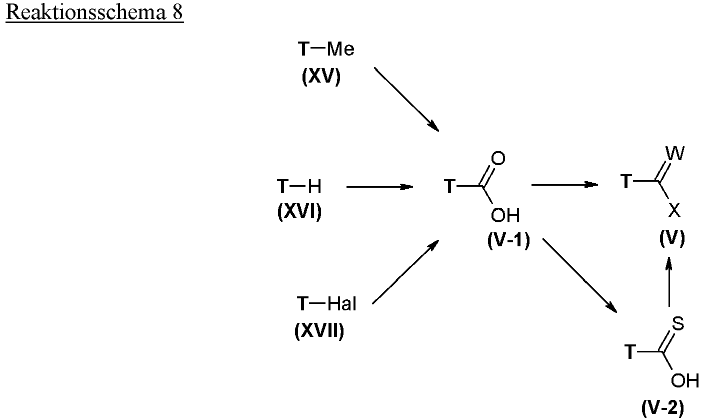

Der Rest W hat die oben beschriebenen Bedeutungen. Hal steht für ein geeignetes Halogen, z.B. Brom oder Iod. X steht für eine geeignete Abgangsgruppe wie z.B. Chlor. T steht für die Gruppierung, 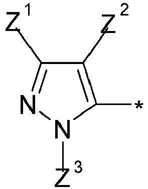 wobei die Reste Z¹, Z² und Z³ die oben genannten Bedeutung haben und der Stern die Anknüpfung an die Gruppierungen Me, H, Hal, COOH, C(=S)OH bzw. C(=W)X darstellt.

Heterozyklischen Carbonsäuren der allgemeinen Struktur **(V-1)** können unter anderem aus Methylderivaten der allgemeinen Formel **(XV)** durch Oxidation der Methylfunktion dargestellt werden. Dabei können die bereits bei der Oxidation von Methylgruppen der Verbindungen der allgemeinen Struktur **(X)** genannten Verfahren angewendet werden.

Heterozyklischen Carbonsäuren der allgemeinen Struktur **(V-1)** können aus Vorläufern der allgemeinen Struktur **(XVI)** durch Deprotonierung mit einer geeigneten Base und durch Abfangen des entsprechenden Carbanions mit Kohlendioxid dargestellt werden [Journal of Medicinal Chemistry 2008, 51(4), 937-947; Bioorganic & Medicinal Chemistry Letters 2007, 17(22), 6274-6279]. Als Base eignen sich z.B. Lithiumdiisopropylamid, n-Butyllithium, s-Butyllithium und diesen ähnliche Verbindungen.

Für das oben beschriebene Verfahren zur Darstellung von heterozyklischen Carbonsäuren der allgemeinen Struktur **(V-1)** eignen sich ebenfalls die entsprechenden halogenierten Heterozyklen **(XVII).** Dabei wird das Carbanion allerdings nicht durch Deprotonierung generiert, sondern durch eine Metallierungsreaktion [Angewandte Chemie, International Edition 2008, 47(2), 311-315]. Für diese Metallierungsreaktionen eignen sich vorzugsweise n-Butyllithium, t-Butyllithium und iso-Propylmagnesiumchlorid.

Heterozyklischen Carbonsäuren der allgemeinen Struktur **(V-1)** können ebenfalls aus halogenierten Vorläufern der allgemeinen Struktur **(XVII)** mit Hilfe von literaturbekannten Palladium-katalysierten Reaktionen in die entsprechenden heterozyklischen Carbonsäureester überführt [Russian Journal of Applied Chemistry 2007, 80(4), 571-575].

Heterozyklischen Carbonsäuren der allgemeinen Struktur **(V-1)** können des Weiteren aus halogenierten Verbindungen der allgemeinen Struktur **(XVII)** durch eine Substitutionsreaktion der Halogene mit Cyaniden und anschließender Hydrolyse der Nitrilfunktion mit starken Säure oder Basen dargestellt werden [WO 2005079801].

Heterozyklischen Thiocarbonsäuren der allgemeinen Struktur **(V-2)** können ausgehend von (V-1) analog zu denen bei der Herstellung von Verbindungen der allgemeinen Formel (II-2) beschriebenen literaturbekannten Methoden hergestellt werden.

Heterocyclische aktivierte Carbonsäurederivate, wie z.B. Carbonsäurehalogenide, wie sie durch die allgemeine Struktur **(V)** repräsentiert werden, können durch Umsetzung einer zyklischen (Thio)Carbonsäure repräsentiert durch die Formeln **(V-1) und (V-2)** mit Halogenierungsreagenzien wie Thionylchlorid, Thionylbromid, Phosphorylchlorid, Oxalylchlorid, Phosphortrichlorid, etc. hergestellt werden [Organikum, Wiley-VCH, 22. Auflage, 496ff].

Aktivierte Carbonsäurederivate der allgemeinen Struktur **(II)** können nach allgemein bekannten Literaturverfahren aus Carbonsäuren der Formel **(II-1)** dargestellt werden [Organikum, Wiley-VCH, 22. Auflage, 496ff; Chem. Ber. 1970, 788; J. Am. Chem. Soc 1967, 5012]. Die Verbindungen der Formel **(II-1)** sind kommerziell erhältlich oder können nach bekannten Literaturverfahren hergestellt werden [Synthesis 2006, (4), 594-596; Tetrahedron Letters 1995, 36(25), 4369-72; Bioorganic & Medicinal Chemistry Letters 2007, 17(4), 1043-1046; PCT Int. Appl., 9502061, Journal of Organic Chemistry 1954, 19, 357-64; WO 2001083459].

Verbindungen der allgemeinen Strukturen (III) sind käuflich und/oder können nach folgenden literaturbekannten, bzw. analogen Verfahren hergestellt werden [Journal of Organic Chemistry 1990, 55(14), 4276-81; WO 2005028429; WO 2005021485; Organic Letters 2010, 12(9), 1944-1947; Tetrahedron 1999, 55(24), 7625-7644].

Verbindungen der allgemeinen Struktur **(V)** sind im Allgemeinen käuflich und/oder können nach bekannten Literaturverfahren dargestellt werden [Journal of Medicinal Chemistry 2008, 51(4), 937-947; Bioorganic & Medicinal Chemistry Letters 2007, 17(22), 6274-6279; Russian Journal of Applied Chemistry 2007, 80(4), 571-575; WO 2005079801; Journal of Organic Chemistry 2008, 73(9), 3523-3529; Bioorganic & Medicinal Chemistry Letters 2005, 15(22), 4898-4906; US2006069270]

Die Verbindungen der allgemeinen Struktur **(VI)** können nach literaturbekannten Verfahren aus den Verbindungen der alllgemeinen Struktur **(II)** dargestellt werden [Journal of the American Chemical Society 2001, 123(34), 8177-8188; Inorganica Chimica Acta 2006, 359(6), 1912-1922].

Verbindungen der allgemeinen Strukturen **(IX)** bis **(XVII)** sind käuflich und/oder aus der einschlägigen Fachliteratur bekannt.

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agens in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag GmbH & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der t-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-t-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N,N-*Dimethyl-formamid, *N*,*N*-Dipropyl-formamid, *N,N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N,N*'-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N,N-*Dimethylanilin, *N,N*-Dimethyl-toluidin, *N,N*-Dimethyl-p-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N*,*N*,N',N'-Tetramethylendiamin, *N*,*N*,N',N'-Tetraethylendiamin, Chinoxalin, N-Propyl-diisopropylamin, N-Ethyl-diisopropylamin, *N,N*'-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

### Weiterhin eignen sich auch Schutzgruppen

vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), t-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));

vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolyl-ethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), t-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));

vom Typ eines sustituierten Benzylethers (z.B. para-Methoxy-benzylether (MPM-OR), 3,4-Dimethoxy-benzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));

vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), t-Butyldimethylsilylether (TBDMS-OR), t-Butyldiphenylsilylether(TBDPS-OR), Tribenzylsilylether, Tri-para-xylylsilylether, Triphenylsilylether (TPS-OR), Diphenylmethylsilylether (DPMS-OR), Di-t-butylmethylsilylether (DTBMS-OR), Tris(trimethylsilyl)silylether (Sisylether), Di-t-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), t-Butylmethoxyphenyl-silylether (TBMPS-OR), t-Butoxydiphenylsilylether (DPTBOS-OR));

vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxo-pentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),

vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMS-OR), 2-(Phenylsulfonyl)-ethylcarbonat (Ps-OR), 2-(Triphenylphosphonio)-ethylcarbonat (Peoc-OR), t-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und

vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , NickelKatalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer N-Oxide und t-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. t-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Experimenteller Teil

### Darstellungsverfahren A

### Beispiel (1) 4-Brom-N-{4-chlor-3-[(1-cyancyclopropyl)carbamoyl]phenyl}-3-(1-fluorcyclopropyl)-1-methyl-1H-pyrazol-5-carboxamid

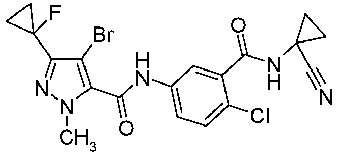

120 mg (0,45 mmol) 4-Brom-3-(1-fluorcyclopropyl)-1-methyl-1*H*-pyrazol-5-carbonsäure werden in 20 mL Dichlormethan p.a. suspendiert und mit 0,02 mL *N,N*-Dimethylformamid p.a. versetzt. Zu dieser Mischung werden 0,119 mL (1.36 mmol) Oxalsäuredichlorid getropft. Danach wird 30 Minuten bei Raumtemperatur und anschließend 30 Minuten unter Rückfluß gerührt. Das Reaktionsgemisch wird nach dem Abkühlen unter vermindertem Druck am Rotationsverdampfer eingeengt. Das so erhaltene Rohprodukt wird ohne weitere Aufreinigung weiter umgesetzt.

108 mg (0,45 mmol) 5-Amino-2-chlor-*N*-(1-cyancyclopropyl)benzamid und 92 mg (0,68 mmol) Silber(I)cyanid werden in 10 mL Dichlormethan p.a. vorgelegt. Zu dieser Suspension wird eine Lösung von 129 mg (0,45 mmol) 4-Brom-3-(1-fluorcyclopropyl)-1-methyl-1*H*-pyrazol-5-carbonsäurechlorid in 10 mL Dichlormethan p.a. getropft. Das Reaktionsgemisch wird 16h bei Raumtemperatur gerührt und anschließend über Silicagel filtriert und Ethylacetat nachgewaschen. Die Lösungsmittel werden unter vermindertem Druck am Rotationsverdampfer entfernt.

Man erhält 170 mg (78%) 4-Brom-*N*-{4-chlor-3-[(1-cyancyclopropyl)carbamoyl]phenyl}-3-(1-fluorcyclopropyl)-1-methyl-1*H*-pyrazol-5-carboxamid als farblosen Feststoff.
¹H-NMR (400 MHz, d₆-DMSO): δ = 10,96 (s, 1H), 9,49 (s, 1H), 7,85 (d, 1H), 7,74 (dd, 1H), 7,55 (d, 1H), 3,93 (s, 3H), 1,57-1,61 (m, 2H), 1,40-1,45 (m, 2H), 1,23-1,28 (m, 2H), 1,08-1,11 (m, 2H) ppm.
HPLC-MS^{a)}: logP = 2,50, Masse (m/z) = 482 [M+H]⁺.

### Darstellungsverfahren B

### Beispiel (2) N-{4-Chlor-3-[(1-cyancyclopropyl)carbamoyl]phenyl}-N,1-dimethyl-3,4-bis(trifluormethyl)-1H-pyrazol-5-carboxamid

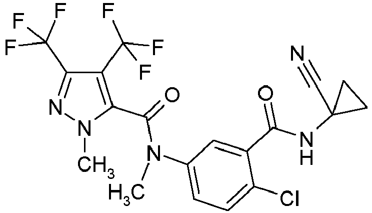

150,0 mg (0,29 mmol) 2-Chlor-5-(methyl{[1-methyl-3,4-bis(trifluormethyl)-1*H*-pyrazol-5-yl]carbonyl}amino)benzoesäure werden in 5,0 mL Dichlormethan p.a. suspendiert. Die Suspension wird anschließend nacheinander mit 0,02 mL *N,N-*Dimethylformamid p.a. und 0,075 mL (0,86 mmol) Oxalylchlorid versetzt. Das Reaktionsgemisch wird 0,5 h bei Raumtemperatur and anschließend 40 Minuten unter Rückfluß erhitzt. Das Lösungsmittel wird unter vermindertem Druck am Rotationsverdampfer entfernt. Das enstandene 2-Chlor-5-(methyl{[1-methyl-3,4-bis(trifluormethyl)-1H-pyrazol-5-yl]carbonyl}amino)benzoylchlorid wird ohne weitere Aufarbeitung für den nachfolgenden Syntheseschritt eingesetzt.

68,3 mg (0,58 mmol) 1-Aminocyclopropancarbonitrilhydrochlorid werden in 5,0 mL Dichlormethan p.a. vorgelegt und anschließend nacheinander mit 0,148 mL (0,86 mmol) *N*-Ethyl-diisopropylamin und mit 156 mg 2-Chlor-5-(methyl{[1-methyl-3,4-bis(trifluormethyl)-1H-pyrazol-5-yl]carbonyl}amino)benzoylchlorid (0,29 mmol) gelöst in 5,0 mL Dichlormethan p.a. versetzt. Die Reaktion wird 16 h bei Raumtemperatur gerührt. Die Reaktionslösung wird mit 30 mL Essigsäureethylester verdünnt. Die organische Phase wird zweimal mit 1 N Salzsäure, einmal mit 1 N Natronlauge und einmal mit gesättigter Natriumchloridlösung gewaschen. Die organische Phase wird über Magnesiumsulfat getrocknet, filtriert und Lösungsmittel am Rotationsverdampfer unter vermindertem Druck entfernt.

Das Rohprodukt wird mittels präparativer HPLC gereinigt. Man erhält 64 mg (45%) *N*-{4-Chlor-3-[(1-cyancyclopropyl)carbamoyl]phenyl}-*N*,1-dimethyl-3,4-bis(trifluormethyl)-1*H*-pyrazol-5-carboxamid als farblosen Feststoff.
¹H-NMR (400 MHz, d₃-Acetonitril, Mischung aus *cis* und *trans* konfigurierten Amiden): δ = 7,42-7,66 (m, 2H), 7,40 (d, 1H), 7,29 (d, 2H), 7,18 (dd, 1H), 3,83 & 3,99 (2 s, zusammen 3H), 3,46 & 3,23 (2 s, zusammen 3H), 1,54-1,60 (m, 2H), 1,25-1,37 (m, 2H) ppm.
HPLC-MS^{a)}: logP = 2,81, Masse (m/z) = 494 [M+H]⁺.

### Darstellungsverfahren C

### Beispiel (50) N-{4-Chlor-3-[(1-cyancyclopropyl)carbamoyl]phenyl}-N-ethyl-3-(pentafluorethyl)-1-propyl-4-(trifluormethyl)-1H-pyrazol-5-carboxamid

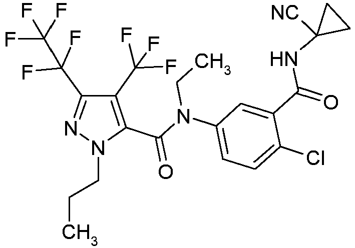

70 mg (0,13 mmol) *N*-{4-Chlor-3-[(1-cyancyclopropyl)carbamoyl]phenyl}-3-(pentafluorethyl)-1-propyl-4-(trifluormethyl)-1*H*-pyrazol-5-carboxamid und 35 mg (0.14 mmol) Kaliumcarbonat werden in 1,4 mL *N,N-*Dimethylformamid p.a. suspendiert. Zu der Mischung werden über einen Zeitraum von 16h nach und nach insgesamt 29 mg (0,19 mmol) Iodethan gegeben. Nach dem Ende der Zugabe wird die Reaktionslösung 20h bei Raumtemperatur gerührt. Das Reaktionsgemisch wird mit Wasser verdünnt und die wässrige Phase dreimal mit Ethylacetat extrahiert. Die vereinigten organischen Phasen werden zweimal mit gesättigter Natriumchloridlösung gewaschen, über Natriumsulfat getrocknet und filtriert. Die Lösungsmittel werden am Rotationsverdampfer unter vermindertem Druck entfernt.

Das Rohprodukt wird säulenchromatographisch an Kiesegel gereinigt. Man erhält 30 mg (41%) *N*-{4-Chlor-3-[(1-cyancyclopropyl)carbamoyl]phenyl}-*N*-ethyl-3-(pentafluorethyl)-1-propyl-4-(trifluormethyl)-1*H*-pyrazol-5-carboxamid als farblosen Feststoff.
¹H-NMR (400 MHz, d₆-DMSO, Mischung aus *cis* und *trans* konfigurierten Amiden): δ = 9,56 & 9,42 (2s, zusammen 1H), 7,69 & 7,56 (2d, zusammen 1H), 7,63 & 7,42 (2d, zusammen 1H), 7,48 & 7,28 (2dd, zusammen 1H), 3,52-4,32 (m, 4H), 0,79-1,87 (m, 12H) ppm.
HPLC-MS^{a)}: logP = 4,06, Masse (m/z) = 586 [M+H]⁺.

### Darstellungsverfahren D

### Beispiel (40) N-{3-[Acetyl(1-cyancyclopropyl)carbamoyl]-4-chlorphenyl}-1-methyl-3-(pentafluorethyl)-4-(trifluormethyl)-1H-pyrazol-5-carboxamid

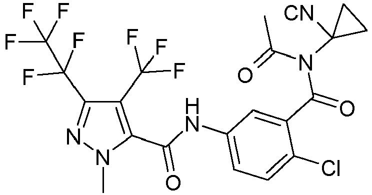

300 mg (0,57 mmol) *N*-{4-Chlor-3-[(1-cyancyclopropyl)carbamoyl]phenyl}-1-methyl-3-(pentafluorethyl)-4-(trifluormethyl)-1*H*-pyrazol-5-carboxamid werden in 6,0 mL Dichlormethan p.a. gelöst und mit einem Eisbad gekühlt. Zu der Lösung werden nacheinander 0,17 mL (0,99 mmol) *N*-Ethyl-diisopropylamin und 49 mg (0,62 mmol) Acetylchlorid gegeben. Die Reaktion wird anschließend auf Raumtemperatur erwärmt und 16h gerührt. Die Reaktionslösung wird mit Dichlromethan verdünnt und anschließend mit Wasser gewaschen. Die organische Phase wird über Natriumsulfat getrocknet, filtriert und das Lösungsmittel am Rotationsverdampfer unter vermindertem Druck entfernt.

Das Rohprodukt wird mittels präparativer HPLC gereinigt. Man erhält 130 mg (40%) *N*-{3-[Acetyl(1-cyancyclopropyl)carbamoyl]-4-chlorphenyl}-1-methyl-3-(pentafluorethyl)-4-(trifluormethyl)-1*H-*pyrazol-5-carboxamid als farblosen Feststoff.
¹H-NMR (400 MHz, d₆-DMSO): δ = 11,49 (s, 1H), 7,90 (d, 1H), 7,70 (dd, 1H), 7,57 (d, 1H), 4,03 (s, 3H), 2,49 (s, 3H), 1,85-1,91 (m, 2H), 1,57-1,67 (m, 2H) ppm.
HPLC-MS^{a)}: logP = 3,81, Masse (m/z) = 572 [M+H]⁺.
a) Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.
b) Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 microm; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters.

Mit Hilfe der oben beschriebenen Darstellungsverfahren A bis D wurden die in den Tabellen 1 & 2 aufgeführten Verbindungen dargestellt.

**Tabelle 1**

| | | | | | | | | | | | | | |
|---|---|---|---|---|---|---|---|---|---|---|---|---|---|
| 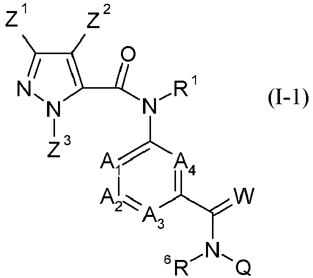 | | | | | | | | | | | | | |

| **Bsp.-Nr.** | **Z¹** | **Z²** | **Z³** | **R¹** | **A¹** | **A²** | **A³** | **A⁴** | **W** | **R⁶** | **Q** | **logP** | **Masse [m/z] 1** |
|---|---|---|---|---|---|---|---|---|---|---|---|---|---|
| 1 | 1-Fluorcyclopropyl | Br | Me | H | CH | CH | CCl | CH | O | H | 1-Cyan-cyclopropyl | 2,50 ^{a)} | 482 ^{a)} |
| 2 | CF₃ | CF₃ | Me | Me | CH | CH | CCl | CH | O | H | 1-Cyan-cyclopropyl | 2,81 ^{a)} | 494 ^{a)} |
| 3 | 1-Chlorcyclopropyl | Cl | Me | Me | CH | CH | CCl | CH | O | H | 1-Cyan-cyclopropyl | 2,46 ^{a)} | 466 ^{a)} |
| 4 | Pentafluorethyl | CF₃ | Me | Me | CH | CH | CCl | CH | O | Et | 1-Cyan-cyclopropyl | 3,77 ^{a)} | 572 ^{a)} |
| 5 | Pentafluorethyl | CF₃ | Me | Me | CH | CH | CCl | CH | O | Me | 1-Cyan-cyclopropyl | 3,59 ^{a)} | 558 ^{a)} |
| 6 | Pentafluorethyl | CF₃ | Me | Et | CH | CH | CCl | CH | O | H | 1-Cyan-cyclopropyl | 3,36 ^{a)} | 558 ^{a)} |
| 7 | Pentafluorethyl | CF₃ | Me | H | CH | CH | CCl | CH | O | H | 1-Cyan-cyclobutyl | 3,55 ^{a)} | 544 ^{a)} |
| 8 | Pentafluorethyl | CF₃ | Me | Me | CH | CH | CCl | CH | O | H | 1-Cyan-cyclopropyl | 3,21 ^{a)} | 544 ^{a)} |
| 9 | CF₃ | CF₃ | Me | H | CH | CH | CCl | CH | O | H | 1-Cyan-cyclopropyl | 2,85 ^{a)} | 480 ^{a)} |
| 10 | Pentafluorethyl | CF₃ | Me | H | CH | CH | CCl | CH | O | H | 1-Cyan-cyclopropyl | 3,27 ^{a)} | 530 ^{a)} |
| 11 | CF₃ | CF₃ | Me | H | CH | CBr | CBr | CH | O | H | 1-Cyan-cyclopropyl | 3,30 | 604 |
| 12 | CF₃ | CF₃ | Me | H | CH | CCl | CBr | CH | O | H | 1-Cyan-cyclopropyl | 3,27 | 560 |
| 13 | Pentafluorethyl | CF₃ | Me | H | CH | CBr | CCl | CH | O | H | 1-Cyan-cyclopropyl | 3,67 | 610 |
| 14 | Pentafluorethyl | CF₃ | Me | H | CH | CH | CBr | CH | O | H | 1-Cyan-cyclopropyl | 3,31 | 576 |
| 15 | Pentafluorethyl | CF₃ | Me | H | CH | CH | CCl | CH | O | Propionyl | 1-Cyan-cyclopropyl | 4,17 | 584² |
| 16 | Pentafluorethyl | CF₃ | Me | H | CH | CCl | CBr | CH | O | H | 1-Cyan-cyclopropyl | 3,73 | 610 |
| 17 | 1-Fluorcyclo-propyl | Cl | Me | H | CH | CH | CCl | CH | O | H | 1-Cyan-cyclopropyl | 2,50 | 436 |
| 18 | Pentafluorethyl | CF₃ | Me | Acetyl | CH | CH | CCl | CH | O | Acetyl | 1-Cyan-cyclopropyl | 4,04 | |
| 19 | Pentafluorethyl | CF₃ | Me | Methoxy-carbonyl | CH | CH | CCl | CH | O | H | 1-Cyan-cyclopropyl | 3,69 | 588 |
| 20 | Pentafluorethyl | CF₃ | Me | Ethoxy-carbonyl | CH | CH | CCl | CH | O | H | 1-Cyan-cyclopropyl | 3,95 | 602 |
| 21 | Pentafluorethyl | CF₃ | Me | Methoxy-carbonyl | CH | CH | CCl | CH | O | Methoxy-carbonyl | 1-Cyan-cyclopropyl | 4,32 | 646 |
| 22 | Pentafluorethyl | CF₃ | Me | Ethoxy-carbonyl | CH | CH | CCl | CH | O | Ethoxy-carbonyl | 1-Cyan-cyclopropyl | 4,80 | 674 |
| 23 | Pentafluorethyl | CF₃ | Me | 2,2-Dimethyl-propanoyl | CH | CH | CCl | CH | O | H | 1-Cyan-cyclopropyl | 4,40 | 614 |
| 24 | Pentafluorethyl | CF₃ | Me | H | CH | CMe | CCl | CH | O | H | 1-Cyan-cyclopropyl | 3,51 | 544 |
| 25 | Pentafluorethyl | CF₃ | Me | H | CH | CH | CI | CH | O | H | 1-Cyan-cyclopropyl | 3,43 | 620² |
| 26 | Pentafluorethyl | CF₃ | Me | Prop-2-in-1-yl | CH | CH | CCl | CH | O | H | 1-Cyan-cyclopropyl | 3,41 | 568 |
| 27 | Pentafluorethyl | CF₃ | Me | H | CH | CH | CF | CH | O | H | 1-Cyan-cyclopropyl | 3,21 | 514 |
| 28 | Pentafluorethyl | CF₃ | Me | 4-Chlorbenzyl | CH | CH | CCl | CH | O | H | 1-Cyan-cyclopropyl | 4,30 | 654 |
| 29 | Pentafluorethyl | CF₃ | Me | Isobutyryl | CH | CH | CCl | CH | O | H | 1-Cyan-cyclopropyl | 4,38 | 600 |
| 30 | Pentafluorethyl | CF₃ | Me | But-2-in-1-yl | CH | CH | CCl | CH | O | H | 1-Cyan-cyclopropyl | 3,60 | 582 |
| 31 | Pentafluorethyl | CF₃ | Me | Benzyl | CH | CH | CCl | CH | O | H | 1-Cyan-cyclopropyl | 3,97 | 620 |
| 32 | Pentafluorethyl | CF₃ | Me | Pyridin-2-ylmethyl | CH | CH | CCl | CH | O | H | 1-Cyan-cyclopropyl | 3,53 | 621 |
| 33 | Pentafluorethyl | CF₃ | Me | Propionyl | CH | CH | CCl | CH | O | H | 1-Cyan-cyclopropyl | 3,91 | 586 |
| 34 | Pentafluorethyl | CF₃ | Et | H | CH | CH | CCl | CH | O | H | 1-Cyan-cyclopropyl | 3,53 | 544 |
| 35 | Pentafluorethyl | CF₃ | Pr | H | CH | CH | CCl | CH | O | H | 1-Cyan-cyclopropyl | 3,87 | 558 |
| 36 | Pentafluorethyl | CF₃ | Pr | H | CH | CH | CF | CH | O | H | 1-Cyan-cyclopropyl | 3,74 | 542 |
| 37 | Pentafluorethyl | CF₃ | Et | H | CH | CH | CF | CH | O | H | 1-Cyan-cyclopropyl | 3,44 | 528 |
| 38 | Pentafluorethyl | CF₃ | Et | Me | CH | CH | CCl | CH | O | H | 1-Cyan-cyclopropyl | 3,57 | 558 |
| 39 | Pentafluorethyl | CF₃ | Me | Et | CH | CBr | CCl | CH | O | H | 1-Cyan-cyclopropyl | 3,92 | 638 |
| 40 | Pentafluorethyl | CF₃ | Me | H | CH | CH | CCl | CH | O | Acetyl | 1-Cyan-cyclopropyl | 3,81 | 572 |
| 41 | Pentafluorethyl | CF₃ | Et | Me | CH | CH | CCl | CH | O | Me | 1-Cyan-cyclopropyl | 3,85 | 572 |
| 42 | Pentafluorethyl | Methylsulfanyl | Me | H | CH | CH | CCl | CH | O | H | 1-Cyan-cyclopropyl | 3,43 | 508 |
| 43 | Pentafluorethyl | CF₃ | Et | Et | CH | CH | CCl | CH | O | H | 1-Cyan-cyclopropyl | 3,68 | 572 |
| 44 | Pentafluorethyl | CF₃ | Me | Cyanmethyl | CH | CH | CCl | CH | O | H | 1-Cyan-cyclopropyl | 3,25 | 569 |
| 45 | Pentafluorethyl | Methyl-sulfinyl | Me | H | CH | CH | CCl | CH | O | H | 1-Cyan-cyclopropyl | 2,91 | 524 |
| 46 | Pentafluorethyl | CF₃ | Pr | Me | CH | CH | CCl | CH | O | Me | 1-Cyan-cyclopropyl | 4,26 | 586 |
| 47 | Pentafluorethyl | CF₃ | Pr | Me | CH | CH | CF | CH | O | Me | 1-Cyan-cyclopropyl | 4,05 | 570 |
| 48 | Pentafluorethyl | CF₃ | Et | Me | CH | CH | CF | CH | O | Me | 1-Cyan-cyclopropyl | 3,76 | 556 |
| 49 | Pentafluorethyl | Methyl-sulfonyl | Me | H | CH | CH | CCl | CH | O | H | 1-Cyan-cyclopropyl | 2,47 | 540 |
| 50 | Pentafluorethyl | CF₃ | Pr | Et | CH | CH | CCl | CH | O | H | 1-Cyan-cyclopropyl | 4,06 | 586 |
| 51 | Pentafluorethyl | CF₃ | Pr | Et | CH | CH | CCl | CH | O | Et | 1-Cyan-cyclopropyl | 4,68 | 614 |
| 52 | Pentafluorethyl | CF₃ | Et | Et | CH | CH | CCl | CH | O | Et | 1-Cyan-cyclopropyl | 4,26 | 600 |
| 53 | Pentafluorethyl | CF₃ | Me | Acetyl | CH | CH | CCl | CH | O | H | 1-Cyan-cyclopropyl | 3,63 | 572 |
| 54 | Pentafluorethyl | CF₃ | Me | Pyridin-3-ylmethyl | CH | CH | CCl | CH | O | H | 1-Cyan-cyclopropyl | 2,85 | 621 |
| 55 | Pentafluorethyl | CF₃ | Me | 2-Methyl-prop-2-en-1-yl | CH | CH | CCl | CH | O | H | 1-Cyan-cyclopropyl | 3,78 | 584 |
| 56 | Pentafluorethyl | CF₃ | Me | Pyridin-4-ylmethyl | CH | CH | CCl | CH | O | H | 1-Cyan-cyclopropyl | 2,52 | 621 |
| 57 | Pentafluorethyl | CF₃ | Prop-2-in-1-yl | H | CH | CH | CCl | CH | O | H | 1-Cyan-cyclopropyl | 3,58 | 554 |
| 58 | Pentafluorethyl | CF₃ | Prop-2-en-1-yl | H | CH | CH | CCl | CH | O | H | 1-Cyan-cyclopropyl | 3,65 | 556 |
| 59 | Pentafluorethyl | CF₃ | Me | H | CH | CF | CBr | CH | O | H | 1-Cyan-cyclopropyl | 3,58 | 592 |
| 60 | Pentafluorethyl | CF₃ | Me | Propionyl | CH | CH | CCl | CH | O | Propionyl | 1-Cyan-cyclopropyl | 4,68 | |
| 61 | Pentafluorethyl | CF₃ | Me | H | CH | CH | CCl | CH | O | Et | 1-Cyan-cyclopropyl | 3,86 | 558 |
| 62 | Pentafluorethyl | CF₃ | Me | H | CH | CH | CCl | CH | O | Me | 1-Cyan-cyclopropyl | 3,66 | 544 |
| 63 | Pentafluorethyl | CF₃ | Me | Isopropoxy-carbonyl | CH | CH | CCl | CH | O | H | 1-Cyan-cyclopropyl | 4,19 | 616 |
| 64 | Pentafluorethyl | CF₃ | 2,2,2-Trifluorethyl | H | CH | CH | CCl | CH | O | H | 1-Cyan-cyclopropyl | 3,60 | 598 |
| 65 | CF₃ | CF₃ | Me | H | CH | CH | CCl | CH | S | H | 1-Cyan-cyclopropyl | | |
| 66 | Pentafluorethyl | CF₃ | Me | Me | CH | CH | CCl | CH | S | H | 1-Cyan-cyclopropyl | | |
| 67 | Pentafluorethyl | CF₃ | Me | H | CH | CH | CCl | CH | S | H | 1-Cyan-cyclobutyl | | |
| 68 | Pentafluorethyl | CF₃ | Me | Et | CH | CH | CCl | CH | S | H | 1-Cyan-cyclopropyl | | |
| 69 | Pentafluorethyl | CF₃ | Me | Me | CH | CH | CCl | CH | S | Me | 1-Cyan-cyclopropyl | | |
| 70 | Pentafluorethyl | CF₃ | Me | Me | CH | CH | CCl | CH | S | Et | 1-Cyan-cyclopropyl | | |
| 71 | 1-Chlorcyclopropyl | Cl | Me | Me | CH | CH | CCl | CH | S | H | 1-Cyan-cyclopropyl | | |
| 72 | CF₃ | CF₃ | Me | Me | CH | CH | CCl | CH | S | H | 1-Cyan-cyclopropyl | | |
| 73 | Pentafluorethyl | CF₃ | Me | H | CH | CH | CCl | CH | S | H | 1-Cyan-cyclopropyl | | |
| 74 | 1-Fluorcyclopropyl | Br | Me | H | CH | CH | CCl | CH | S | H | 1-Cyan-cyclopropyl | | |
| 75 | CF₃ | CF₃ | Me | H | CH | CBr | CBr | CH | S | H | 1-Cyan-cyclopropyl | | |
| 76 | CF₃ | CF₃ | Me | H | CH | CCl | CBr | CH | S | H | 1-Cyan-cyclopropyl | | |
| 77 | Pentafluorethyl | CF₃ | Me | H | CH | CBr | CCl | CH | S | H | 1-Cyan-cyclopropyl | | |
| 78 | Pentafluorethyl | CF₃ | Me | H | CH | CH | CBr | CH | S | H | 1-Cyan-cyclopropyl | | |
| 79 | Pentafluorethyl | CF₃ | Me | H | CH | CH | CCl | CH | S | Propionyl | 1-Cyan-cyclopropyl | | |
| 80 | Pentafluorethyl | CF₃ | Me | H | CH | CCl | CBr | CH | S | H | 1-Cyan-cyclopropyl | | |
| 81 | 1-Fluorcyclopropyl | Cl | Me | H | CH | CH | CCl | CH | S | H | 1-Cyan-cyclopropyl | | |
| 82 | Pentafluorethyl | CF₃ | Me | Acetyl | CH | CH | CCl | CH | S | Acetyl | 1-Cyan-cyclopropyl | | |
| 83 | Pentafluorethyl | CF₃ | Me | Methoxy-carbonyl | CH | CH | CCl | CH | S | H | 1-Cyan-cyclopropyl | | |
| 84 | Pentafluorethyl | CF₃ | Me | Ethoxy-carbonyl | CH | CH | CCl | CH | S | H | 1-Cyan-cyclopropyl | | |
| 85 | Pentafluorethyl | CF₃ | Me | Methoxy-carbonyl | CH | CH | CCl | CH | S | Methoxy-carbonyl | 1-Cyan-cyclopropyl | | |
| 86 | Pentafluorethyl | CF₃ | Me | Ethoxy- carbonyl | CH | CH | CCl | CH | S | Ethoxy- carbonyl | 1-Cyan-cyclopropyl | | |
| 87 | Pentafluorethyl | CF₃ | Me | 2,2-Dimethyl-propanoyl | CH | CH | CCl | CH | S | H | 1-Cyan-cyclopropyl | | |
| 88 | Pentafluorethyl | CF₃ | Me | H | CH | CMe | CCl | CH | S | H | 1-Cyan-cyclopropyl | | |
| 89 | Pentafluorethyl | CF₃ | Me | H | CH | CH | CI | CH | S | H | 1-Cyan-cyclopropyl | | |
| 90 | Pentafluorethyl | CF₃ | Me | Prop-2-in-1-yl | CH | CH | CCl | CH | S | H | 1-Cyan-cyclopropyl | | |
| 91 | Pentafluorethyl | CF₃ | Me | H | CH | CH | CF | CH | S | H | 1-Cyan-cyclopropyl | | |
| 92 | Pentafluorethyl | CF₃ | Me | 4-Chlorbenzyl | CH | CH | CCl | CH | S | H | 1-Cyan-cyclopropyl | | |
| 93 | Pentafluorethyl | CF₃ | Me | Isobutyryl | CH | CH | CCl | CH | S | H | 1-Cyan-cyclopropyl | | |
| 94 | Pentafluorethyl | CF₃ | Me | But-2-in-1-yl | CH | CH | CCl | CH | S | H | 1-Cyan-cyclopropyl | | |
| 95 | Pentafluorethyl | CF₃ | Me | Benzyl | CH | CH | CCl | CH | S | H | 1-Cyan-cyclopropyl | | |
| 96 | Pentafluorethyl | CF₃ | Me | Pyridin-2-ylmethyl | CH | CH | CCl | CH | S | H | 1-Cyan-cyclopropyl | | |
| 97 | Pentafluorethyl | CF₃ | Me | Propionyl | CH | CH | CCl | CH | S | H | 1-Cyan-cyclopropyl | | |
| 98 | Pentafluorethyl | CF₃ | Et | H | CH | CH | CCl | CH | S | H | 1-Cyan-cyclopropyl | | |
| 99 | Pentafluorethyl | CF₃ | Pr | H | CH | CH | CCl | CH | S | H | 1-Cyan-cyclopropyl | | |
| 100 | Pentafluorethyl | CF₃ | Pr | H | CH | CH | CF | CH | S | H | 1-Cyan-cyclopropyl | | |
| 101 | Pentafluorethyl | CF₃ | Et | H | CH | CH | CF | CH | S | H | 1-Cyan-cyclopropyl | | |
| 102 | Pentafluorethyl | CF₃ | Et | Me | CH | CH | CCl | CH | S | H | 1-Cyan-cyclopropyl | | |
| 103 | Pentafluorethyl | CF₃ | Me | Et | CH | CBr | CCl | CH | S | H | 1-Cyan-cyclopropyl | | |
| 104 | Pentafluorethyl | CF₃ | Me | H | CH | CH | CCl | CH | S | Acetyl | 1-Cyan-cyclopropyl | | |
| 105 | Pentafluorethyl | CF₃ | Et | Me | CH | CH | CCl | CH | S | Me | 1-Cyan-cyclopropyl | | |
| 106 | Pentafluorethyl | Methylsulfanyl | Me | H | CH | CH | CCl | CH | S | H | 1-Cyan-cyclopropyl | | |
| 107 | Pentafluorethyl | CF₃ | Et | Et | CH | CH | CCl | CH | S | H | 1-Cyan-cyclopropyl | | |
| 108 | Pentafluorethyl | CF₃ | Me | Cyanmethyl | CH | CH | CCl | CH | S | H | 1-Cyan-cyclopropyl | | |
| 109 | Pentafluorethyl | Methyl-sulfinyl | Me | H | CH | CH | CCl | CH | S | H | 1-Cyan-cyclopropyl | | |
| 110 | Pentafluorethyl | CF₃ | Pr | Me | CH | CH | CCl | CH | S | Me | 1-Cyan-cyclopropyl | | |
| 111 | Pentafluorethyl | CF₃ | Pr | Me | CH | CH | CF | CH | S | Me | 1-Cyan-cyclopropyl | | |
| 112 | Pentafluorethyl | CF₃ | Et | Me | CH | CH | CF | CH | S | H | 1-Cyan-cyclopropyl | | |
| 113 | Pentafluorethyl | Methylsulfonyl | Me | H | CH | CH | CCl | CH | S | H | 1-Cyan-cyclopropyl | | |
| 114 | Pentafluorethyl | CF₃ | Pr | Et | CH | CH | CCl | CH | S | H | 1-Cyan-cyclopropyl | | |
| 115 | Pentafluorethyl | CF₃ | Pr | Et | CH | CH | CCl | CH | S | Et | 1-Cyan-cyclopropyl | | |
| 116 | Pentafluorethyl | CF₃ | Et | Et | CH | CH | CCl | CH | S | Et | 1-Cyan-cyclopropyl | | |
| 117 | Pentafluorethyl | CF₃ | Me | Acetyl | CH | CH | CCl | CH | S | H | 1-Cyan-cyclopropyl | | |
| 118 | Pentafluorethyl | CF₃ | Me | Pyridin-3-ylmethyl | CH | CH | CCl | CH | S | H | 1-Cyan-cyclopropyl | | |
| 119 | Pentafluorethyl | CF₃ | Me | 2-Methylprop-2-en-1-yl | CH | CH | CCl | CH | S | H | 1-Cyan-cyclopropyl | | |
| 120 | Pentafluorethyl | CF₃ | Me | Pyridin-4-ylmethyl | CH | CH | CCl | CH | S | H | 1-Cyan-cyclopropyl | | |
| 121 | Pentafluorethyl | CF₃ | Prop-2-in-1-yl | H | CH | CH | CCl | CH | S | H | 1-Cyan-cyclopropyl | | |
| 122 | Pentafluorethyl | CF₃ | Prop-2-en-1-yl | H | CH | CH | CCl | CH | S | H | 1-Cyan-cyclopropyl | | |
| 123 | Pentafluorethyl | CF₃ | Me | H | CH | CF | CBr | CH | S | H | 1-Cyan-cyclopropyl | | |
| 124 | Pentafluorethyl | CF₃ | Me | Propionyl | CH | CH | CCl | CH | S | Propionyl | 1-Cyan-cyclopropyl | | |
| 125 | Pentafluorethyl | CF₃ | Me | H | CH | CH | CCl | CH | S | Et | 1-Cyan-cyclopropyl | | |
| 126 | Pentafluorethyl | CF₃ | Me | H | CH | CH | CCl | CH | S | Me | 1-Cyan-cyclopropyl | | |
| 127 | Pentafluorethyl | CF₃ | Me | Isopropoxy-carbonyl | CH | CH | CCl | CH | S | H | 1-Cyan-cyclopropyl | | |
| 128 | Pentafluorethyl | CF₃ | 2,2,2-Trifluorethyl | H | CH | CH | CCl | CH | S | H | 1-Cyan-cyclopropyl | | |

**Tabelle 2**

| 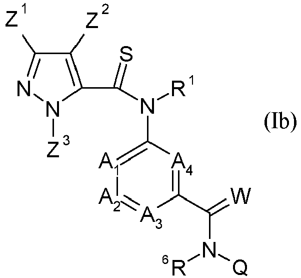 | | | | | | | | | | | | | |
|---|---|---|---|---|---|---|---|---|---|---|---|---|---|
| **Bsp.-Nr.** | **Z¹** | **Z²** | **Z³** | **R¹** | **A¹** | **A²** | **A³** | **A⁴** | **W** | **R⁶** | **Q** | **logP** | **Masse [m/z] 1** |
| 129 | 1-Fluorcyclopropyl | Br | Me | H | CH | CH | CCl | CH | O | H | 1-Cyan-cyclopropyl | | |
| 130 | CF₃ | CF₃ | Me | Me | CH | CH | CCl | CH | O | H | 1-Cyan-cyclopropyl | | |
| 131 | 1-Chlorcyclopropyl | Cl | Me | Me | CH | CH | CCl | CH | O | H | 1-Cyan-cyclopropyl | | |
| 132 | Pentafluorethyl | CF₃ | Me | Me | CH | CH | CCl | CH | O | Et | 1-Cyan-cyclopropyl | | |
| 133 | Pentafluorethyl | CF₃ | Me | Me | CH | CH | CCl | CH | O | Me | 1-Cyan-cyclopropyl | | |
| 134 | Pentafluorethyl | CF₃ | Me | Et | CH | CH | CCl | CH | O | H | 1-Cyan-cyclopropyl | | |
| 135 | Pentafluorethyl | CF₃ | Me | H | CH | CH | CCl | CH | O | H | 1-Cyan-cyclobutyl | | |
| 136 | Pentafluorethyl | CF₃ | Me | Me | CH | CH | CCl | CH | O | H | 1-Cyan-cyclopropyl | | |
| 137 | CF₃ | CF₃ | Me | H | CH | CH | CCl | CH | O | H | 1-Cyan-cyclopropyl | | |
| 138 | Pentafluorethyl | CF₃ | Me | H | CH | CH | CCl | CH | O | H | 1-Cyan-cyclopropyl | | |
| 139 | CF₃ | CF₃ | Me | H | CH | CBr | CBr | CH | O | H | 1-Cyan-cyclopropyl | | |

| **Bsp.-Nr.** | **Z¹** | **Z²** | **Z³** | **R¹** | **A¹** | **A²** | **A³** | **A⁴** | **W** | **R⁶** | **Q** | **logP** | **Masse [m/z] 1** |
|---|---|---|---|---|---|---|---|---|---|---|---|---|---|
| 140 | CF₃ | CF₃ | Me | H | CH | CCl | CBr | CH | O | H | 1-Cyan-cyclopropyl | | |
| 141 | Pentafluorethyl | CF₃ | Me | H | CH | CBr | CCl | CH | O | H | 1-Cyan-cyclopropyl | | |
| 142 | Pentafluorethyl | CF₃ | Me | H | CH | CH | CBr | CH | O | H | 1-Cyan-cyclopropyl | | |
| 143 | Pentafluorethyl | CF₃ | Me | H | CH | CH | CCl | CH | O | Propionyl | 1-Cyan-cyclopropyl | | |
| 144 | Pentafluorethyl | CF₃ | Me | H | CH | CCl | CBr | CH | O | H | 1-Cyan-cyclopropyl | | |
| 145 | 1-Fluorcyclo-propyl | Cl | Me | H | CH | CH | CCl | CH | O | H | 1-Cyan-cyclopropyl | | |
| 146 | Pentafluorethyl | CF₃ | Me | Acetyl | CH | CH | CCl | CH | O | Acetyl | 1-Cyan-cyclopropyl | | |
| 147 | Pentafluorethyl | CF₃ | Me | Methoxy-carbonyl | CH | CH | CCl | CH | O | H | 1-Cyan-cyclopropyl | | |
| 148 | Pentafluorethyl | CF₃ | Me | Ethoxy-carbonyl | CH | CH | CCl | CH | O | H | 1-Cyan-cyclopropyl | | |
| 149 | Pentafluorethyl | CF₃ | Me | Methoxy-carbonyl | CH | CH | CCl | CH | O | Methoxy-carbonyl | 1-Cyan-cyclopropyl | | |
| 150 | Pentafluorethyl | CF₃ | Me | Ethoxy-carbonyl | CH | CH | CCl | CH | O | Ethoxy-carbonyl | 1-Cyan-cyclopropyl | | |
| 151 | Pentafluorethyl | CF₃ | Me | 2,2-Dimethyl-propanoyl | CH | CH | CCl | CH | O | H | 1-Cyan-cyclopropyl | | |
| 152 | Pentafluorethyl | CF₃ | Me | H | CH | CMe | CCl | CH | O | H | 1-Cyan-cyclopropyl | | |
| 153 | Pentafluorethyl | CF₃ | Me | H | CH | CH | CI | CH | O | H | 1-Cyan-cyclopropyl | | |
| 154 | Pentafluorethyl | CF₃ | Me | Prop-2-in-1-yl | CH | CH | CCl | CH | O | H | 1-Cyan-cyclopropyl | | |
| 155 | Pentafluorethyl | CF₃ | Me | H | CH | CH | CF | CH | O | H | 1-Cyan-cyclopropyl | | |
| 156 | Pentafluorethyl | CF₃ | Me | 4-Chlorbenzyl | CH | CH | CCl | CH | O | H | 1-Cyan-cyclopropyl | | |
| 157 | Pentafluorethyl | CF₃ | Me | Isobutyryl | CH | CH | CCl | CH | O | H | 1-Cyan-cyclopropyl | | |
| 158 | Pentafluorethyl | CF₃ | Me | But-2-in-1-yl | CH | CH | CCl | CH | O | H | 1-Cyan-cyclopropyl | | |
| 159 | Pentafluorethyl | CF₃ | Me | Benzyl | CH | CH | CCl | CH | O | H | 1-Cyan-cyclopropyl | | |
| 160 | Pentafluorethyl | CF₃ | Me | Pyridin-2-ylmethyl | CH | CH | CCl | CH | O | H | 1-Cyan-cyclopropyl | | |
| 161 | Pentafluorethyl | CF₃ | Me | Propionyl | CH | CH | CCl | CH | O | H | 1-Cyan-cyclopropyl | | |
| 162 | Pentafluorethyl | CF₃ | Et | H | CH | CH | CCl | CH | O | H | 1-Cyan-cyclopropyl | | |
| 163 | Pentafluorethyl | CF₃ | Pr | H | CH | CH | CCl | CH | O | H | 1-Cyan-cyclopropyl | | |
| 164 | Pentafluorethyl | CF₃ | Pr | H | CH | CH | CF | CH | O | H | 1-Cyan-cyclopropyl | | |
| 165 | Pentafluorethyl | CF₃ | Et | H | CH | CH | CF | CH | O | H | 1-Cyan-cyclopropyl | | |
| 166 | Pentafluorethyl | CF₃ | Et | Me | CH | CH | CCl | CH | O | H | 1-Cyan-cyclopropyl | | |
| 167 | Pentafluorethyl | CF₃ | Me | Et | CH | CBr | CCl | CH | O | H | 1-Cyan-cyclopropyl | | |
| 168 | Pentafluorethyl | CF₃ | Me | H | CH | CH | CCl | CH | O | Acetyl | 1-Cyan-cyclopropyl | | |
| 169 | Pentafluorethyl | CF₃ | Et | Me | CH | CH | CCl | CH | O | Me | 1-Cyan-cyclopropyl | | |
| 170 | Pentafluorethyl | Methylsulfanyl | Me | H | CH | CH | CCl | CH | O | H | 1-Cyan-cyclopropyl | | |
| 171 | Pentafluorethyl | CF₃ | Et | Et | CH | CH | CCl | CH | O | H | 1-Cyan-cyclopropyl | | |
| 172 | Pentafluorethyl | CF₃ | Me | Cyanmethyl | CH | CH | CCl | CH | O | H | 1-Cyan-cyclopropyl | | |
| 173 | Pentafluorethyl | Methyl-sulfinyl | Me | H | CH | CH | CCl | CH | O | H | 1-Cyan-cyclopropyl | | |
| 174 | Pentafluorethyl | CF₃ | Pr | Me | CH | CH | CCl | CH | O | Me | 1-Cyan-cyclopropyl | | |
| 175 | Pentafluorethyl | CF₃ | Pr | Me | CH | CH | CF | CH | O | Me | 1-Cyan-cyclopropyl | | |
| 176 | Pentafluorethyl | CF₃ | Et | Me | CH | CH | CF | CH | O | H | 1-Cyan-cyclopropyl | | |
| 177 | Pentafluorethyl | Methyl-sulfonyl | Me | H | CH | CH | CCl | CH | O | H | 1-Cyan-cyclopropyl | | |
| 178 | Pentafluorethyl | CF₃ | Pr | Et | CH | CH | CCl | CH | O | H | 1-Cyan-cyclopropyl | | |
| 179 | Pentafluorethyl | CF₃ | Pr | Et | CH | CH | CCl | CH | O | Et | 1-Cyan-cyclopropyl | | |
| 180 | Pentafluorethyl | CF₃ | Et | Et | CH | CH | CCl | CH | O | Et | 1-Cyan-cyclopropyl | | |
| 181 | Pentafluorethyl | CF₃ | Me | Acetyl | CH | CH | CCl | CH | O | H | 1-Cyan-cyclopropyl | | |
| 182 | Pentafluorethyl | CF₃ | Me | Pyridin-3-ylmethyl | CH | CH | CCl | CH | O | H | 1-Cyan-cyclopropyl | | |
| 183 | Pentafluorethyl | CF₃ | Me | 2-Methyl-prop-2-en-1-yl | CH | CH | CCl | CH | O | H | 1-Cyan-cyclopropyl | | |
| 184 | Pentafluorethyl | CF₃ | Me | Pyridin-4-ylmethyl | CH | CH | CCl | CH | O | H | 1-Cyan-cyclopropyl | | |
| 185 | Pentafluorethyl | CF₃ | Prop-2-in-1-yl | H | CH | CH | CCl | CH | O | H | 1-Cyan-cyclopropyl | | |
| 186 | Pentafluorethyl | CF₃ | Prop-2-en-1-yl | H | CH | CH | CCl | CH | O | H | 1-Cyan-cyclopropyl | | |
| 187 | Pentafluorethyl | CF₃ | Me | H | CH | CF | CBr | CH | O | H | 1-Cyan-cyclopropyl | | |
| 188 | Pentafluorethyl | CF₃ | Me | Propionyl | CH | CH | CCl | CH | O | Propionyl | 1-Cyan-cyclopropyl | | |
| 189 | Pentafluorethyl | CF₃ | Me | H | CH | CH | CCl | CH | O | Et | 1-Cyan-cyclopropyl | | |
| 190 | Pentafluorethyl | CF₃ | Me | H | CH | CH | CCl | CH | O | Me | 1-Cyan-cyclopropyl | | |
| 191 | Pentafluorethyl | CF₃ | Me | Isopropoxy-carbonyl | CH | CH | CCl | CH | O | H | 1-Cyan-cyclopropyl | | |
| 192 | Pentafluorethyl | CF₃ | 2,2,2-Trifluorethyl | H | CH | CH | CCl | CH | O | H | 1-Cyan-cyclopropyl | | |
| 193 | CF₃ | CF₃ | Me | H | CH | CH | CCl | CH | S | H | 1-Cyan-cyclopropyl | | |
| 194 | Pentafluorethyl | CF₃ | Me | Me | CH | CH | CCl | CH | S | H | 1-Cyan-cyclopropyl | | |
| 195 | Pentafluorethyl | CF₃ | Me | H | CH | CH | CCl | CH | S | H | 1-Cyan-cyclobutyl | | |
| 196 | Pentafluorethyl | CF₃ | Me | Et | CH | CH | CCl | CH | S | H | 1-Cyan-cyclopropyl | | |
| 197 | Pentafluorethyl | CF₃ | Me | Me | CH | CH | CCl | CH | S | Me | 1-Cyan-cyclopropyl | | |
| 198 | Pentafluorethyl | CF₃ | Me | Me | CH | CH | CCl | CH | S | Et | 1-Cyan-cyclopropyl | | |
| 199 | 1-Chlorcyclo-propyl | Cl | Me | Me | CH | CH | CCl | CH | S | H | 1-Cyan-cyclopropyl | | |
| 200 | CF₃ | CF₃ | Me | Me | CH | CH | CCl | CH | S | H | 1-Cyan-cyclopropyl | | |
| 201 | Pentafluorethyl | CF₃ | Me | H | CH | CH | CCl | CH | S | H | 1-Cyan-cyclopropyl | | |
| 202 | 1-Fluorcyclopropyl | Br | Me | H | CH | CH | CCl | CH | S | H | 1-Cyan-cyclopropyl | | |
| 203 | CF₃ | CF₃ | Me | H | CH | CBr | CBr | CH | S | H | 1-Cyan-cyclopropyl | | |
| 204 | CF₃ | CF₃ | Me | H | CH | CCl | CBr | CH | S | H | 1-Cyan-cyclopropyl | | |
| 205 | Pentafluorethyl | CF₃ | Me | H | CH | CBr | CCl | CH | S | H | 1-Cyan-cyclopropyl | | |
| 206 | Pentafluorethyl | CF₃ | Me | H | CH | CH | CBr | CH | S | H | 1-Cyan-cyclopropyl | | |
| 207 | Pentafluorethyl | CF₃ | Me | H | CH | CH | CCl | CH | S | Propionyl | 1-Cyan-cyclopropyl | | |
| 208 | Pentafluorethyl | CF₃ | Me | H | CH | CCl | CBr | CH | S | H | 1-Cyan-cyclopropyl | | |
| 209 | 1-Fluorcyclopropyl | Cl | Me | H | CH | CH | CCl | CH | S | H | 1-Cyan-cyclopropyl | | |
| 210 | Pentafluorethyl | CF₃ | Me | Acetyl | CH | CH | CCl | CH | S | Acetyl | 1-Cyan-cyclopropyl | | |
| 211 | Pentafluorethyl | CF₃ | Me | Methoxy-carbonyl | CH | CH | CCl | CH | S | H | 1-Cyan-cyclopropyl | | |
| 212 | Pentafluorethyl | CF₃ | Me | Ethoxy-carbonyl | CH | CH | CCl | CH | S | H | 1-Cyan-cyclopropyl | | |
| 213 | Pentafluorethyl | CF₃ | Me | Methoxy-carbonyl | CH | CH | CCl | CH | S | Methoxy-carbonyl | 1-Cyan-cyclopropyl | | |
| 214 | Pentafluorethyl | CF₃ | Me | Ethoxy-carbonyl | CH | CH | CCl | CH | S | Ethoxy-carbonyl | 1-Cyan-cyclopropyl | | |
| 215 | Pentafluorethyl | CF₃ | Me | 2,2-Dimethyl-propanoyl | CH | CH | CCl | CH | S | H | 1-Cyan-cyclopropyl | | |
| 216 | Pentafluorethyl | CF₃ | Me | H | CH | CMe | CCl | CH | S | H | 1-Cyan-cyclopropyl | | |
| 217 | Pentafluorethyl | CF₃ | Me | H | CH | CH | CI | CH | S | H | 1-Cyan-cyclopropyl | | |
| 218 | Pentafluorethyl | CF₃ | Me | Prop-2-in-1-yl | CH | CH | CCl | CH | S | H | 1-Cyan-cyclopropyl | | |
| 219 | Pentafluorethyl | CF₃ | Me | H | CH | CH | CF | CH | S | H | 1-Cyan-cyclopropyl | | |
| 220 | Pentafluorethyl | CF₃ | Me | 4-Chlorbenzyl | CH | CH | CCl | CH | S | H | 1-Cyan-cyclopropyl | | |
| 221 | Pentafluorethyl | CF₃ | Me | Isobutyryl | CH | CH | CCl | CH | S | H | 1-Cyan-cyclopropyl | | |
| 222 | Pentafluorethyl | CF₃ | Me | But-2-in-1-yl | CH | CH | CCl | CH | S | H | 1-Cyan-cyclopropyl | | |
| 223 | Pentafluorethyl | CF₃ | Me | Benzyl | CH | CH | CCl | CH | S | H | 1-Cyan-cyclopropyl | | |
| 224 | Pentafluorethyl | CF₃ | Me | Pyridin-2-ylmethyl | CH | CH | CCl | CH | S | H | 1-Cyan-cyclopropyl | | |
| 225 | Pentafluorethyl | CF₃ | Me | Propionyl | CH | CH | CCl | CH | S | H | 1-Cyan-cyclopropyl | | |
| 226 | Pentafluorethyl | CF₃ | Et | H | CH | CH | CCl | CH | S | H | 1-Cyan-cyclopropyl | | |
| 227 | Pentafluorethyl | CF₃ | Pr | H | CH | CH | CCl | CH | S | H | 1-Cyan-cyclopropyl | | |
| 228 | Pentafluorethyl | CF₃ | Pr | H | CH | CH | CF | CH | S | H | 1-Cyan-cyclopropyl | | |
| 229 | Pentafluorethyl | CF₃ | Et | H | CH | CH | CF | CH | S | H | 1-Cyan-cyclopropyl | | |
| 230 | Pentafluorethyl | CF₃ | Et | Me | CH | CH | CCl | CH | S | H | 1-Cyan-cyclopropyl | | |
| 231 | Pentafluorethyl | CF₃ | Me | Et | CH | CBr | CCl | CH | S | H | 1-Cyan-cyclopropyl | | |
| 232 | Pentafluorethyl | CF₃ | Me | H | CH | CH | CCl | CH | S | Acetyl | 1-Cyan-cyclopropyl | | |
| 233 | Pentafluorethyl | CF₃ | Et | Me | CH | CH | CCl | CH | S | Me | 1-Cyan-cyclopropyl | | |
| 234 | Pentafluorethyl | Methyl-sulfanyl | Me | H | CH | CH | CCl | CH | S | H | 1-Cyan-cyclopropyl | | |
| 235 | Pentafluorethyl | CF₃ | Et | Et | CH | CH | CCl | CH | S | H | 1-Cyan-cyclopropyl | | |
| 236 | Pentafluorethyl | CF₃ | Me | Cyanmethyl | CH | CH | CCl | CH | S | H | 1-Cyan-cyclopropyl | | |
| 237 | Pentafluorethyl | Methyl-sulfinyl | Me | H | CH | CH | CCl | CH | S | H | 1-Cyan-cyclopropyl | | |
| 238 | Pentafluorethyl | CF₃ | Pr | Me | CH | CH | CCl | CH | S | Me | 1-Cyan-cyclopropyl | | |
| 239 | Pentafluorethyl | CF₃ | Pr | Me | CH | CH | CF | CH | S | Me | 1-Cyan-cyclopropyl | | |
| 240 | Pentafluorethyl | CF₃ | Et | Me | CH | CH | CF | CH | S | H | 1-Cyan-cyclopropyl | | |
| 241 | Pentafluorethyl | Methyl-sulfonyl | Me | H | CH | CH | CCl | CH | S | H | 1-Cyan-cyclopropyl | | |
| 242 | Pentafluorethyl | CF₃ | Pr | Et | CH | CH | CCl | CH | S | H | 1-Cyan-cyclopropyl | | |
| 243 | Pentafluorethyl | CF₃ | Pr | Et | CH | CH | CCl | CH | S | Et | 1-Cyan-cyclopropyl | | |
| 244 | Pentafluorethyl | CF₃ | Et | Et | CH | CH | CCl | CH | S | Et | 1-Cyan-cyclopropyl | | |
| 245 | Pentafluorethyl | CF₃ | Me | Acetyl | CH | CH | CCl | CH | S | H | 1-Cyan-cyclopropyl | | |
| 246 | Pentafluorethyl | CF₃ | Me | Pyridin-3-ylmethyl | CH | CH | CCl | CH | S | H | 1-Cyan-cyclopropyl | | |
| 247 | Pentafluorethyl | CF₃ | Me | 2-Methylprop-2-en-1-yl | CH | CH | CCl | CH | S | H | 1-Cyan-cyclopropyl | | |
| 248 | Pentafluorethyl | CF₃ | Me | Pyridin-4-ylmethyl | CH | CH | CCl | CH | S | H | 1-Cyan-cyclopropyl | | |
| 249 | Pentafluorethyl | CF₃ | Prop-2-in-1-yl | H | CH | CH | CCl | CH | S | H | 1-Cyan-cyclopropyl | | |
| 250 | Pentafluorethyl | CF₃ | Prop-2-en-1-yl | H | CH | CH | CCl | CH | S | H | 1-Cyan-cyclopropyl | | |
| 251 | Pentafluorethyl | CF₃ | Me | H | CH | CF | CBr | CH | S | H | 1-Cyan-cyclopropyl | | |
| 252 | Pentafluorethyl | CF₃ | Me | Propionyl | CH | CH | CCl | CH | S | Propionyl | 1-Cyan-cyclopropyl | | |
| 253 | Pentafluorethyl | CF₃ | Me | H | CH | CH | CCl | CH | S | Et | 1-Cyan-cyclopropyl | | |
| 254 | Pentafluorethyl | CF₃ | Me | H | CH | CH | CCl | CH | S | Me | 1-Cyan-cyclopropyl | | |
| 255 | Pentafluorethyl | CF₃ | Me | Isopropoxycarbonyl | CH | CH | CCl | CH | S | H | 1-Cyan-cyclopropyl | | |
| 256 | Pentafluorethyl | CF₃ | 2,2,2-Trifluorethyl | H | CH | CH | CCl | CH | S | H | 1-Cyan-cyclopropyl | | |

Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit ² gekennzeichnet.
² Bei der angegebenen Masse handelt es sich um den Peak des Isotopenmusters des [M-H]⁻ Ions mit der höchsten Intensität.
   a) Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.
   b) Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 microm; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters.

### NMR-Daten ausgewählter Beispiele

Die ¹H-NMR-Daten ausgewählter Beispiele sind in Form von ¹H-NMR-Peaklisten notiert. Zu jedem Signalpeak sind der δ-Wert in ppm und die Signalintensität in Klammern aufgeführt.

| |
|---|
| Ex.. 3, Solvent: [DMSO], Spektrometer: 600,13MHz |
| 11,2357 (5,81); 9,4015 (2,32); 7,9529 (0,33); 7,9112 (0,33); 7,6345 (0,34); 7,4603 (2,39); 7,3952 (0,42); 7,3764 (0,35); 7,3658 (0,38); 7,3009 (0,7); 7,2887 (0,72); 4,4308 (0,59); 4,4236 (0,6); 4,0461 (1,28); 4,0342 (3,7); 4,0223 (3,71); 4,0105 (1,26); 3,853 (0,6); 3,8474 (0,42); 3,8307 (0,44); 3,7989 (0,86); 3,788 (1,25); 3,7603 (10,68); 3,7331 (1,16); 3,726 (1,01); 3,7196 (0,75); 3,7116 (0,53); 3,7053 (0,33); 3,459 (0,59); 3,4058 (13,82); 3,3755 (1,92); 3,3496 (164,63); 3,3259 (1,89); 3,2237 (0,45); 2,8908 (2,26); 2,7309 (1,92); 2,6212 (0,39); 2,6184 (0,58); 2,6154 (0,72); 2,6125 (0,61); 2,5427 (1,49); 2,5244 (3,1); 2,5214 (3,56); 2,5182 (3,84); 2,5093 (28,51); 2,5064 (58,78); 2,5034 (80,56); 2,5004 (60,43); 2,4975 (29,82); 2,3903 (0,38); 2,3873 (0,5); 2,3843 (0,39); 2,2647 (0,57); 2,2538 (0,97); 2,2442 (0,58); 2,2423 (0,63); 2,0871 (0,62); 2,0233 (0,41); 2,0152 (0,44); 2,0134 (0,46); 1,9905 (16); 1,9096 (1,12); 1,851 (0,52); 1,8378 (0,99); 1,7685 (0,38); 1,7588 (0,55); 1,7475 (0,49); 1,7373 (0,4); 1,7288 (0,34); 1,7133 (0,34); 1,7077 (0,35); 1,6908 (0,37); 1,6852 (0,36); 1,6645 (0,89); 1,6546 (1,09); 1,6502 (1,34); 1,6452 (1,8); 1,6419 (1,74); 1,637 (1,45); 1,6253 (1,25); 1,6174 (0,98); 1,6101 (0,88); 1,5994 (5,38); 1,5901 (11,8); 1,5856 (12,84); 1,5767 (5,21); 1,55 (0,56); 1,4866 (0,63); 1,473 (0,68); 1,467 (0,7); 1,4621 (0,62); 1,4595 (0,61); 1,4523 (0,5); 1,4479 (0,41); 1,4361 (0,35); 1,3967 (1,99); 1,3471 (5,81); 1,2984 (1,34); 1,2782 (1,83); 1,2518 (6,83); 1,2423 (12,83); 1,2384 (14,34); 1,2293 (8,19); 1,2043 (2,47); 1,1864 (5,57); 1,1746 (9,7); 1,1627 (5,14); 1,1493 (0,89); 1,1396 (0,87); 1,1361 (0,62); 1,1312 (0,65); 1,1158 (0,53); 1,111 (0,55); 1,108 (0,57); 1,1022 (0,42); 1,0935 (0,51); 1,0875 (0,48); 1,0847 (0,45); 1,0749 (0,38); 1,0668 (0,41); 1,0552 (0,36); 0,8965 (0,36); 0,8891 (1,06); 0,8851 (0,68); 0,8808 (0,62); 0,8743 (0,76); 0,8695 (0,73); 0,8627 (0,7); 0,8536 (0,94); 0,8421 (0,6); 0,8361 (0,4); 0,8234 (0,34); -0,0001 (1,54) |
| Ex.. 4, Solvent: [DMSO], Spektrometer: 399,95MHz |
| 19,9995 (0,35); 11,1852 (0,43); 7,6987 (0,97); 7,6759 (1,26); 7,5988 (1,74); 7,5752 (1,65); 7,4867 (2,1); 7,3938 (0,43); 7,3652 (0,84); 7,2019 (0,66); 5,7464 (4,97); 4,0662 (6,84); 4,0391 (1,88); 4,0214 (2,04); 3,9933 (16); 3,595 (0,77); 3,5647 (0,67); 3,4575 (11,78); 3,3059 (1902,13); 3,2823 (28,24); 3,251 (7,34); 3,1867 (1,32); 3,0111 (0,86); 2,9377 (0,79); 2,9232 (0,87); 2,8698 (1,22); 2,7378 (0,55); 2,6938 (0,68); 2,6741 (2,27); 2,6694 (2,78); 2,6647 (2,28); 2,5392 (5,5); 2,5089 (156,27); 2,5047 (278,63); 2,5003 (354,49); 2,496 (248,83); 2,3948 (0,32); 2,3317 (1,82); 2,327 (2,38); 2,0693 (1,78); 2,0088 (0,37); 1,9867 (5,95); 1,9077 (1,11); 1,7114 (5,17); 1,6397 (0,5); 1,5825 (0,5); 1,4849 (1,82); 1,3986 (7,92); 1,3522 (3,78); 1,2986 (3,52); 1,259 (3,75); 1,2366 (4,08); 1,2171 (1,92); 1,1981 (2,64); 1,1928 (3,24); 1,1751 (4,15); 1,1572 (2,51); 1,1005 (2,54); 0,8906 (0,33); 0,8673 (0,4); 0,8547 (0,48); - 0,0002 (5,05) |
| Ex.. 5, Solvent: [DMSO], Spektrometer: 601,6MHz |
| 7,7016 (0,47); 7,6872 (0,61); 7,646 (0,57); 7,6419 (0,64); 7,6063 (0,37); 7,6024 (0,36); 7,5921 (0,54); 7,5882 (0,56); 7,579 (0,6); 7,3776 (1,3); 7,3475 (0,34); 5,7617 (16); 4,0701 (2,56); 4,0607 (0,79); 4,0345 (0,58); 4,0227 (0,63); 4,0108 (0,48); 3,9904 (2,73); 3,4829 (0,66); 3,4608 (9,78); 3,4259 (0,46); 3,3469 (320,39); 3,3232 (1,5); 3,2777 (0,75); 3,2493 (2,86); 3,2349 (0,66); 3,1106 (0,9); 3,076 (0,68); 2,8649 (2,78); 2,6213 (0,57); 2,6183 (0,8); 2,6153 (0,97); 2,6122 (0,82); 2,6093 (0,59); 2,543 (0,42); 2,5246 (1,24); 2,5215 (1,51); 2,5184 (1,42); 2,5096 (29,65); 2,5066 (65,23); 2,5035 (89,35); 2,5005 (63,63); 2,4975 (28,79); 2,3908 (0,39); 2,3877 (0,55); 2,3847 (0,39); 2,0771 (1); 1,9902 (1,99); 1,6767 (1,95); 1,6734 (2,2); 1,424 (0,34); 1,2981 (0,62); 1,2582 (0,83); 1,2356 (0,77); 1,1865 (0,59); 1,1747 (1,16); 1,1629 (0,58); -0,0002 (5,52) |
| Ex.. 6, Solvent: [DMSO], Spektrometer: 399,95MHz |
| 9,5105 (1,11); 9,37 (2,71); 7,6861 (0,89); 7,6648 (1,1); 7,6188 (1,04); 7,6126 (1,08); 7,5402 (2,64); 7,5188 (3,08); 7,5037 (0,69); 7,4974 (0,6); 7,4823 (0,53); 7,4761 (0,48); 7,4235 (2,42); 7,4171 (2,51); 7,2689 (1,57); 7,2623 (1,43); 7,2476 (1,35); 7,2409 (1,22); 4,1197 (0,76); 4,1021 (1,01); 4,0852 (1,27); 4,0686 (4,88); 4,0575 (1,57); 4,0496 (0,54); 4,0397 (3,72); 4,0219 (3,71); 4,0041 (1,27); 3,8782 (10,99); 3,835 (0,33); 3,817 (0,89); 3,7993 (1,08); 3,7824 (0,96); 3,7647 (0,74); 3,6837 (0,33); 3,6654 (0,43); 3,6475 (0,38); 3,5854 (0,39); 3,5673 (0,46); 3,5489 (0,34); 3,3007 (145,63); 3,2772 (2,79); 2,6695 (0,34); 2,5395 (0,82); 2,5225 (1,85); 2,5092 (19,84); 2,5049 (35,06); 2,5004 (44,17); 2,4961 (30,18); 2,4917 (14,34); 1,987 (16); 1,6127 (0,63); 1,6036 (1,61); 1,5987 (1,68); 1,5905 (3,88); 1,5832 (3,24); 1,57 (1,36); 1,3041 (0,63); 1,2906 (1,18); 1,2841 (1,18); 1,2696 (0,62); 1,2594 (0,5); 1,2375 (1,04); 1,2307 (1,64); 1,2171 (3,03); 1,2106 (3,03); 1,1931 (4,97); 1,1753 (9,03); 1,1575 (7,71); 1,1395 (7,05); 1,1216 (3,13); 0,962 (1,16); 0,9444 (2,37); 0,9267 (1,1); -0,0002 (4,85) |
| Ex.. 7, Solvent: [DMSO], Spektrometer: 399,95MHz |
| 11,4754 (1,96); 9,5774 (3,66); 7,8103 (3,62); 7,804 (4,04); 7,7228 (1,61); 7,7165 (1,38); 7,701 (2,26); 7,6946 (2,08); 7,606 (3,39); 7,5841 (2,38); 4,0559 (1,18); 4,0345 (16); 4,0204 (3,51); 4,0025 (1,13); 3,3203 (18,4); 2,7184 (0,84); 2,7027 (1,21); 2,6977 (1,45); 2,6851 (1,56); 2,6827 (1,57); 2,6699 (2,11); 2,6503 (1,2); 2,5102 (14,26); 2,5059 (28,52); 2,5014 (37,33); 2,4969 (26,79); 2,4927 (12,96); 2,4835 (1,57); 2,4608 (2,15); 2,4525 (1,25); 2,4404 (1,55); 2,4316 (1,69); 2,4084 (0,97); 2,1036 (0,56); 2,0953 (0,6); 2,085 (1,52); 2,0732 (1,03); 2,0651 (2,45); 2,058 (1,57); 2,0451 (1,65); 2,0352 (0,77); 2,0288 (0,39); 2,0252 (0,41); 2,0196 (0,4); 1,9889 (13,81); 1,1925 (3,78); 1,1747 (7,53); 1,1569 (3,66); -0,0002 (0,53) |
| Ex.. 8, Solvent: [CD3CN], Spektrometer: 399,95MHz |
| 7,6396 (0,36); 7,5867 (0,51); 7,5653 (0,89); 7,544 (0,65); 7,5381 (0,86); 7,5157 (0,67); 7,5093 (0,52); 7,4941 (0,53); 7,4875 (0,63); 7,4768 (0,79); 7,3968 (2,11); 7,3753 (2,53); 7,3065 (1,94); 7,2998 (2,09); 7,1716 (1,4); 7,1649 (1,32); 7,1502 (1,17); 7,1434 (1,07); 3,9903 (3,54); 3,8495 (10,24); 3,4568 (16); 3,2228 (4,6); 2,1412 (168,83); 2,1318 (2,24); 2,1187 (0,37); 2,1125 (0,33); 2,1063 (0,38); 1,9749 (1,26); 1,9632 (92,07); 1,9513 (18,04); 1,9451 (33,99); 1,9389 (49,06); 1,9328 (33,45); 1,9266 (16,89); 1,7909 (0,5); 1,5939 (0,45); 1,5781 (1,51); 1,5737 (1,53); 1,5637 (2,7); 1,5577 (3,02); 1,5476 (0,89); 1,5443 (0,86); 1,3605 (0,47); 1,3468 (0,97); 1,3398 (1,12); 1,3254 (0,39); 1,2938 (1,35); 1,2881 (1,36); 1,2837 (1,68); 1,2798 (2,1); 1,2733 (1,74); 1,2636 (1,03); -0,0002 (5,39) |
| Ex.. 9, Solvent: [CD3CN], Spektrometer: 601,6MHz |
| 9,3097 (0,56); 7,77 (3,79); 7,7657 (4,14); 7,6801 (2,48); 7,6757 (2,25); 7,6656 (2,92); 7,6612 (2,72); 7,6146 (1,14); 7,4969 (4,24); 7,4825 (3,68); 3,981 (16); 2,1615 (181,62); 2,0874 (0,35); 2,0771 (1,5); 2,0604 (0,46); 2,0563 (0,85); 2,0522 (1,16); 2,048 (0,87); 2,044 (0,44); 1,9735 (2,01); 1,9659 (259,46); 1,9576 (3,22); 1,9536 (3,65); 1,9499 (74,77); 1,9457 (141,93); 1,9416 (218,7); 1,9375 (150,41); 1,9334 (73,06); 1,9287 (2,12); 1,9246 (0,98); 1,8507 (1,44); 1,8351 (0,43); 1,831 (0,84); 1,8269 (1,17); 1,8227 (0,83); 1,8186 (0,43); 1,5805 (1,94); 1,5707 (4,1); 1,5665 (4,32); 1,5572 (2,31); 1,351 (2,23); 1,3416 (4); 1,3375 (4,07); 1,3276 (1,81); 0,9112 (1,45); 0,0053 (0,53); -0,0002 (20,71); -0,0057 (0,61) |
| Ex.. 10, Solvent: [DMSO], Spektrometer: 600,13MHz |
| 11,4592 (3,78); 9,4859 (4,45); 7,7721 (3,8); 7,7678 (4,24); 7,7112 (2,06); 7,7068 (1,78); 7,6966 (2,55); 7,6922 (2,36); 7,5881 (4,49); 7,5736 (3,69); 4,0356 (0,65); 4,0285 (16); 3,3215 (12,57); 2,5227 (0,37); 2,5196 (0,46); 2,5165 (0,45); 2,5077 (11,12); 2,5047 (24,25); 2,5017 (33,57); 2,4986 (24,06); 2,4956 (10,94); 1,989 (1,5); 1,6018 (1,64); 1,5924 (3,8); 1,5879 (4,14); 1,5789 (1,69); 1,2696 (1,81); 1,2604 (3,74); 1,256 (4,13); 1,2464 (1,5); 1,1871 (0,41); 1,1753 (0,83); 1,1634 (0,41); 0,0053 (0,46); -0,0001 (14,7); -0,0057 (0,44) |
| Ex.. 11, Solvent: [CD3CN], Spektrometer: 601,6MHz |
| 9,4276 (0,94); 8,1604 (0,81); 8,1562 (0,84); 8,0909 (3,21); 8,0868 (3,24); 7,8499 (0,65); 7,8457 (0,65); 7,6042 (1,17); 7,5896 (3,34); 7,5855 (3,31); 3,99 (0,48); 3,9775 (16); 2,2102 (11,21); 2,0881 (0,39); 2,0779 (0,4); 2,0656 (0,66); 2,0611 (0,41); 2,057 (0,54); 2,0529 (0,65); 2,0486 (0,62); 2,048 (0,62); 2,0447 (0,36); 1,9732 (0,39); 1,9666 (16,06); 1,9584 (0,96); 1,9542 (1,36); 1,9506 (29,13); 1,9464 (53,99); 1,9423 (82,18); 1,9382 (57,22); 1,9341 (28,42); 1,9294 (1,09); 1,9253 (0,59); 1,8317 (0,39); 1,8276 (0,51); 1,8234 (0,37); 1,5826 (1,54); 1,5728 (3,25); 1,5686 (3,38); 1,5593 (1,89); 1,3599 (1,74); 1,3506 (3,08); 1,3464 (3,24); 1,3366 (1,49); 0,911 (0,48); -0,0002 (0,46) |
| Ex.. 12, Solvent: [CD3CN], Spektrometer: 601,6MHz |
| 9,4577 (0,94); 7,9506 (3,67); 7,9465 (3,74); 7,6252 (1,29); 7,6178 (0,41); 7,5485 (3,85); 7,5444 (3,76); 5,4498 (1,24); 4,1225 (0,39); 3,9907 (0,48); 3,9784 (16); 2,2016 (26,81); 2,0656 (0,9); 2,0571 (0,36); 2,053 (0,47); 2,0479 (0,79); 1,9732 (1,48); 1,9668 (2,89); 1,9586 (0,78); 1,9572 (0,41); 1,9544 (1,17); 1,9507 (24,16); 1,9466 (45,51); 1,9424 (67,8); 1,9383 (46,53); 1,9342 (23,27); 1,9296 (0,68); 1,8277 (0,39); 1,5858 (1,78); 1,576 (3,73); 1,5718 (3,9); 1,5625 (2,15); 1,3635 (2,04); 1,3541 (3,59); 1,3499 (3,77); 1,3401 (1,77); 1,269 (0,34); 1,2159 (0,42); 1,2041 (0,83); 1,1922 (0,4); 0,911 (0,44) |
| Ex.. 13, Solvent: [CD3CN], Spektrometer: 399,95MHz |
| 9,425 (1,09); 8,1057 (3,59); 8,0995 (3,63); 8,089 (0,38); 8,0828 (0,33); 7,9667 (0,34); 7,6477 (3,95); 7,6415 (4,02); 7,626 (1,54); 7,6058 (0,49); 7,5997 (0,42); 7,5882 (0,4); 7,582 (0,48); 7,4416 (0,5); 4,1118 (0,33); 4,0862 (1,68); 4,0683 (4,78); 4,0505 (4,77); 4,0327 (1,64); 3,9761 (16); 3,8931 (3,57); 2,1489 (347,53); 2,1318 (7,91); 2,1127 (2,21); 2,1065 (2,07); 2,1003 (1,56); 2,0941 (1,09); 2,0486 (0,56); 2,0434 (0,54); 1,9712 (32,63); 1,9634 (416,05); 1,9514 (67,15); 1,9453 (119,86); 1,9391 (167,42); 1,9329 (114,6); 1,9267 (58,47); 1,7911 (2,41); 1,7799 (0,49); 1,7737 (0,78); 1,7675 (1,01); 1,7614 (0,72); 1,7553 (0,42); 1,6282 (0,54); 1,6129 (1,02); 1,6069 (1,1); 1,604 (0,72); 1,5923 (0,84); 1,5869 (1,9); 1,5726 (4,35); 1,5656 (4,47); 1,5518 (2,49); 1,5116 (0,39); 1,3551 (2,45); 1,3413 (4,31); 1,3343 (4,21); 1,3295 (1,9); 1,3202 (2,1); 1,3078 (0,68); 1,2852 (0,55); 1,2708 (1,26); 1,2216 (6,04); 1,2038 (11,75); 1,1859 (5,85); 0,9117 (0,79); 0,008 (0,82); -0,0002 (18,6); -0,0085 (0,72) |
| Ex.. 14, Solvent: [DMSO], Spektrometer: 601,6MHz |
| 11,475 (2,8); 9,482 (4,01); 7,7377 (4,18); 7,7332 (5,35); 7,7173 (3,05); 7,6669 (0,49); 7,6647 (0,45); 7,6299 (2,01); 7,6257 (1,86); 7,6155 (1,61); 7,6111 (1,54); 4,046 (0,85); 4,0342 (3,8); 4,0263 (16); 4,0225 (3,74); 4,0105 (0,89); 3,3858 (0,71); 3,3809 (1,58); 3,3784 (1,63); 3,3578 (1678,47); 3,3341 (3,39); 3,0116 (0,41); 2,8065 (0,44); 2,6184 (0,64); 2,6153 (0,91); 2,6123 (0,64); 2,543 (0,47); 2,5247 (1,22); 2,5216 (1,5); 2,5185 (1,46); 2,5097 (46,46); 2,5066 (102,74); 2,5036 (142,42); 2,5006 (100,14); 2,4975 (44,69); 2,3908 (0,62); 2,3877 (0,87); 2,3847 (0,62); 2,0764 (1,87); 1,99 (10,94); 1,6062 (1,8); 1,5968 (4,05); 1,5923 (4,48); 1,5834 (1,81); 1,3972 (8,53); 1,272 (1,92); 1,2628 (3,88); 1,2584 (4,36); 1,2489 (1,8); 1,1863 (2,99); 1,1745 (5,92); 1,1626 (2,92); 0,0053 (0,68); -0,0002 (22,51); -0,0058 (0,62) |
| Ex.. 15, Solvent: [CD3CN], Spektrometer: 601,6MHz |
| 7,6736 (3,25); 7,6594 (3,63); 7,6529 (0,62); 7,6383 (0,67); 7,5026 (1,73); 7,476 (1,42); 7,4719 (1,04); 7,4618 (1,32); 7,4578 (1,01); 7,4501 (0,41); 7,4349 (0,37); 5,3097 (0,35); 5,2976 (0,39); 4,0762 (0,87); 4,0644 (2,65); 4,0525 (2,74); 4,0407 (0,93); 3,9757 (0,91); 3,9408 (5,26); 3,9313 (16); 2,8568 (0,62); 2,8452 (1,86); 2,8333 (1,86); 2,8212 (0,7); 2,5123 (0,35); 2,4998 (1,1); 2,4874 (1,07); 2,4749 (0,42); 2,2936 (1,21); 2,2824 (1,31); 2,2699 (0,62); 2,2265 (0,62); 2,2149 (0,61); 2,1802 (0,36); 2,1739 (0,55); 2,1673 (0,84); 2,1535 (644,37); 2,1294 (0,59); 2,1215 (0,48); 2,0601 (0,87); 2,0561 (1,51); 2,0519 (2,22); 2,0478 (1,52); 2,0437 (0,77); 1,9727 (12,69); 1,9656 (21,18); 1,9575 (11,83); 1,9534 (14,58); 1,9496 (147,5); 1,9454 (266,85); 1,9413 (402,32); 1,9372 (276,01); 1,9331 (137,69); 1,9244 (1,96); 1,8503 (0,47); 1,8347 (1,84); 1,8307 (2,73); 1,8266 (3,2); 1,8225 (2,22); 1,8184 (1,32); 1,7246 (0,62); 1,7188 (0,99); 1,7132 (0,82); 1,6902 (1,73); 1,6658 (1); 1,6361 (0,55); 1,6214 (0,44); 1,6159 (0,49); 1,5864 (0,66); 1,5822 (0,66); 1,5728 (0,47); 1,5666 (0,46); 1,5571 (0,39); 1,5334 (1,65); 1,5063 (1,02); 1,5029 (0,92); 1,4777 (0,65); 1,4659 (0,65); 1,3852 (0,49); 1,3515 (0,42); 1,3403 (1,5); 1,3284 (0,54); 1,2851 (2,14); 1,2701 (1,88); 1,2384 (0,36); 1,2227 (0,53); 1,2158 (4,13); 1,2039 (7,13); 1,1963 (1,02); 1,1921 (4,09); 1,1765 (0,52); 1,1568 (0,64); 1,1488 (1,95); 1,1363 (4,5); 1,1315 (0,55); 1,1238 (2,55); 1,1152 (0,75); 1,1112 (1,54); 1,1021 (0,47); 1,0987 (0,78); 1,0915 (6,22); 1,0797 (12,97); 1,0677 (6,14); 1,0509 (0,37); 1,0462 (0,5); 1,0418 (0,48); 1,0022 (0,57); 0,9849 (0,79); 0,9637 (0,69); 0,949 (4,67); 0,9431 (1,35); 0,9371 (10,26); 0,9313 (2,24); 0,9244 (5,69); 0,9194 (1,11); 0,9121 (1,74); 0,8817 (0,48); 0,0964 (0,45); 0,0053 (3,79); -0,0002 (131,79); -0,0058 (3,52); -0,1 (0,49) |
| Ex.. 16, Solvent: [DMSO], Spektrometer: 601,6MHz |
| 11,6171 (2,98); 9,5642 (4,11); 8,0041 (4,64); 8,0001 (4,81); 7,5795 (3,62); 7,5754 (3,58); 4,377 (0,35); 4,3686 (0,7); 4,3601 (0,34); 4,0417 (16); 4,0342 (2,77); 4,0223 (2,5); 4,0105 (0,81); 3,9261 (1,01); 3,4494 (0,63); 3,4409 (0,64); 3,4378 (0,67); 3,4293 (0,66); 3,3467 (410,03); 3,3231 (3,18); 2,6178 (0,42); 2,6148 (0,6); 2,6118 (0,44); 2,5425 (0,44); 2,524 (1,29); 2,521 (1,64); 2,5178 (1,9); 2,509 (30,94); 2,5061 (65,8); 2,5031 (89,43); 2,5 (63,28); 2,497 (28,23); 2,3902 (0,4); 2,3872 (0,55); 2,3842 (0,39); 2,0769 (0,6); 1,99 (10,77); 1,9093 (0,47); 1,6161 (1,9); 1,6067 (4,15); 1,6022 (4,51); 1,5933 (1,81); 1,3972 (13,57); 1,3139 (0,32); 1,2846 (2,01); 1,2754 (3,98); 1,271 (4,39); 1,2614 (1,64); 1,1863 (2,93); 1,1745 (6,06); 1,1627 (2,93); 1,0665 (1,46); 1,0549 (2,75); 1,0433 (1,33); 0,0052 (0,71); -0,0002 (20,09); -0,0057 (0,51) |
| Ex.. 17, Solvent: [DMSO], Spektrometer: 399,95MHz |
| 10,9164 (2,69); 9,4701 (3,13); 7,8379 (2,16); 7,8316 (2,46); 7,7664 (1,22); 7,76 (1,05); 7,7445 (1,48); 7,7381 (1,32); 7,5569 (3,32); 7,5351 (2,79); 5,7527 (16); 4,0382 (0,59); 4,0204 (0,58); 3,9311 (10,48); 3,9291 (10,23); 3,4476 (0,32); 3,4238 (0,79); 3,3995 (1,41); 3,3529 (444,16); 3,2649 (0,45); 2,6723 (0,35); 2,5255 (1,04); 2,5123 (20,85); 2,5078 (42,03); 2,5032 (55,95); 2,4986 (40,59); 2,4941 (19,88); 2,3299 (0,35); 2,0728 (0,88); 1,9887 (2,51); 1,6084 (1,2); 1,5942 (2,85); 1,5874 (2,99); 1,5741 (1,42); 1,4745 (0,54); 1,4586 (1,66); 1,4548 (1,64); 1,4399 (0,7); 1,4294 (0,58); 1,4133 (1,6); 1,4094 (1,68); 1,3975 (0,83); 1,3948 (0,79); 1,2663 (1,44); 1,2525 (2,82); 1,246 (3,04); 1,2314 (1,24); 1,1927 (0,77); 1,1749 (1,45); 1,1571 (0,71); 1,129 (0,6); 1,1124 (1,86); 1,1085 (2,26); 1,0923 (2,2); 1,0884 (1,8); 1,0706 (0,5) |
| Ex.. 18, Solvent: [DMSO], Spektrometer: 601,6MHz |
| 7,8045 (2,23); 7,7904 (2,42); 7,6562 (0,38); 4,0456 (0,8); 4,0338 (2,39); 4,0304 (0,69); 4,0219 (3,08); 4,015 (16); 3,3608 (569,24); 3,3375 (3,07); 2,6187 (0,62); 2,6157 (0,85); 2,6127 (0,62); 2,5434 (0,54); 2,525 (1,66); 2,5219 (2,15); 2,5188 (2,4); 2,51 (45,96); 2,507 (97,64); 2,504 (134,23); 2,5009 (97,3); 2,4979 (44,27); 2,4056 (15,53); 2,3974 (1,27); 2,3945 (0,66); 2,3911 (0,88); 2,3882 (1,2); 2,3851 (0,77); 2,3823 (0,42); 2,2183 (1,21); 2,0872 (0,45); 2,0779 (2,65); 2,0487 (3,59); 2,0305 (0,77); 2,0256 (0,48); 1,9905 (10,01); 1,9538 (0,39); 1,9098 (4,41); 1,8867 (0,87); 1,8814 (0,87); 1,3969 (14,61); 1,2345 (0,46); 1,1862 (2,73); 1,1744 (5,37); 1,1653 (0,38); 1,1625 (2,65); -0,0002 (9,42) |
| Ex.. 19, Solvent: [DMSO], Spektrometer: 399,95MHz |
| 9,6008 (2,87); 7,7336 (2,68); 7,7122 (3,27); 7,6828 (2,15); 7,6766 (2,25); 7,518 (1,37); 7,5117 (1,26); 7,4968 (1,12); 7,4904 (1,05); 4,066 (10,48); 4,0381 (0,83); 4,0203 (1,07); 4,0026 (0,33); 3,6767 (16); 3,487 (0,45); 3,4643 (0,44); 3,4467 (0,79); 3,4391 (0,81); 3,4231 (1,05); 3,4136 (1,13); 3,3468 (882,82); 3,3224 (20,56); 3,2828 (0,63); 2,6764 (0,54); 2,6718 (0,74); 2,6674 (0,52); 2,542 (0,61); 2,5249 (2,21); 2,5118 (42,56); 2,5073 (85,34); 2,5028 (113,7); 2,4982 (82,44); 2,4937 (40); 2,3341 (0,49); 2,3295 (0,72); 2,3249 (0,52); 2,0729 (3,68); 1,9886 (3,32); 1,6558 (0,33); 1,6144 (1,37); 1,6001 (2,7); 1,5932 (2,9); 1,5801 (1,41); 1,2938 (1,3); 1,2802 (2,57); 1,2735 (2,8); 1,2588 (1,33); 1,2356 (0,78); 1,1925 (0,98); 1,1748 (2); 1,1569 (1); 0,008 (1,05); -0,0002 (28,19); -0,0085 (1,08) |
| Ex.. 20, Solvent: [DMSO], Spektrometer: 601,6MHz |
| 9,6087 (4,57); 7,7293 (4,27); 7,7152 (4,9); 7,6801 (2,65); 7,676 (2,67); 7,4972 (1,63); 7,493 (1,51); 7,4831 (1,43); 7,4788 (1,38); 4,3682 (0,58); 4,1597 (0,43); 4,1483 (1); 4,1369 (1,24); 4,1257 (1,28); 4,1143 (1); 4,1025 (0,47); 4,0779 (16); 4,0458 (0,46); 4,0339 (1,37); 4,0221 (1,98); 4,0103 (0,59); 3,449 (0,49); 3,4406 (0,49); 3,4374 (0,48); 3,429 (0,51); 3,3716 (1,66); 3,3457 (1847,32); 3,3222 (24,12); 2,6205 (0,63); 2,6176 (1,47); 2,6145 (2,05); 2,6115 (1,48); 2,6085 (0,63); 2,5422 (0,95); 2,5239 (2,46); 2,5208 (3,15); 2,5177 (3,1); 2,5088 (106,25); 2,5058 (238,45); 2,5028 (334,46); 2,4997 (242,59); 2,4967 (110,57); 2,393 (0,68); 2,39 (1,49); 2,3869 (2,09); 2,3839 (1,49); 2,3808 (0,69); 2,0766 (4,02); 1,9898 (5,37); 1,6109 (1,83); 1,6016 (3,88); 1,5971 (4,4); 1,5882 (2,19); 1,5763 (0,45); 1,2977 (0,37); 1,2859 (1,83); 1,2767 (3,71); 1,2723 (4,13); 1,2627 (1,75); 1,2579 (0,87); 1,2535 (1,01); 1,2499 (0,7); 1,2416 (1,16); 1,2342 (1,17); 1,23 (0,84); 1,1861 (1,54); 1,1742 (3,2); 1,1624 (1,62); 1,1496 (0,36); 1,0663 (1,12); 1,0546 (2); 1,043 (1,01); 0,9965 (6,51); 0,9847 (13,73); 0,973 (6,33); 0,0965 (0,41); 0,0053 (3,4); -0,0002 (125,56); -0,0058 (3,65); -0,1001 (0,45) |
| Ex.. 21, Solvent: [DMSO], Spektrometer: 399,95MHz |
| 7,7082 (2,45); 7,6866 (3,64); 7,6836 (2,1); 7,6766 (1,8); 7,663 (0,46); 7,5662 (0,33); 7,5492 (1,41); 7,5429 (1,29); 7,5279 (1,11); 7,5216 (1,06); 4,0591 (10,46); 4,038 (1,4); 4,0203 (2,19); 4,0025 (0,48); 3,7168 (0,33); 3,679 (15,83); 3,6733 (4,07); 3,6676 (16); 3,4322 (0,35); 3,3435 (321,55); 3,3363 (458,6); 3,2717 (0,52); 3,1751 (0,88); 3,162 (0,79); 2,6807 (0,32); 2,676 (0,65); 2,6714 (0,93); 2,6667 (0,65); 2,5415 (0,74); 2,5246 (2,58); 2,5114 (50,63); 2,5069 (102,7); 2,5023 (137,44); 2,4977 (99,21); 2,4931 (48,14); 2,3337 (0,63); 2,3291 (0,88); 2,3244 (0,63); 2,0731 (1,7); 1,9885 (5,54); 1,9008 (1,74); 1,8954 (2,04); 1,6574 (0,47); 1,6142 (1,05); 1,5995 (1,75); 1,5933 (1,89); 1,2728 (0,35); 1,2585 (0,57); 1,2522 (0,48); 1,2358 (0,96); 1,1925 (1,64); 1,1747 (3,39); 1,1569 (1,68); -0,0002 (6,5) |
| Ex.. 22, Solvent: [DMSO], Spektrometer: 601,6MHz |
| 7,7596 (0,35); 7,711 (3,38); 7,6968 (4,08); 7,6797 (1,48); 7,5444 (1,6); 7,5402 (1,49); 7,5303 (1,29); 7,526 (1,24); 4,3683 (0,44); 4,2593 (0,33); 4,2476 (0,33); 4,1566 (0,43); 4,1449 (0,94); 4,1332 (1,15); 4,1243 (1,15); 4,1129 (1,05); 4,1045 (0,75); 4,0946 (1,48); 4,0828 (3,99); 4,0686 (16); 4,0595 (1,27); 4,034 (0,45); 4,0221 (0,58); 4,0158 (0,43); 4,0103 (0,4); 3,449 (0,38); 3,4406 (0,38); 3,4374 (0,4); 3,429 (0,41); 3,3742 (1,02); 3,3457 (1563,22); 3,322 (28,53); 2,6206 (0,69); 2,6176 (1,46); 2,6146 (2,04); 2,6115 (1,44); 2,6085 (0,64); 2,5423 (1,22); 2,5239 (3,79); 2,5208 (5,05); 2,5176 (5,93); 2,5089 (109,54); 2,5059 (236,08); 2,5028 (323,23); 2,4997 (233,05); 2,4967 (103,3); 2,4766 (0,35); 2,3931 (0,66); 2,39 (1,43); 2,387 (2); 2,3839 (1,38); 2,3809 (0,63); 2,0767 (3,48); 1,9899 (1,42); 1,9031 (2,58); 1,6908 (0,35); 1,6696 (0,36); 1,6437 (0,33); 1,6184 (0,43); 1,5761 (1,67); 1,2579 (0,49); 1,2485 (0,35); 1,2344 (1,07); 1,2265 (0,65); 1,2146 (1,08); 1,2029 (0,52); 1,1861 (0,52); 1,1743 (1,03); 1,1705 (0,37); 1,1625 (0,52); 1,1492 (0,42); 1,0663 (0,87); 1,0547 (1,54); 1,043 (0,81); 1,0013 (6,44); 0,9895 (13,63); 0,9848 (1,07); 0,9817 (2,21); 0,9778 (6,34); 0,97 (0,97); 0,9557 (6,62); 0,9439 (14,2); 0,9321 (6,4); 0,0052 (3,03); -0,0002 (83,54); -0,0058 (2,47) |
| Ex.. 23, Solvent: [DMSO], Spektrometer: 399,95MHz |
| 9,5605 (1,88); 7,757 (1,46); 7,7355 (1,78); 7,6605 (0,8); 7,545 (0,66); 7,5386 (0,61); 7,5238 (0,55); 7,5174 (0,52); 4,058 (1,05); 4,0402 (3,29); 4,0271 (6,99); 4,0225 (4,44); 4,0046 (1,09); 3,3924 (24,23); 3,386 (32,63); 3,3799 (54,59); 3,377 (71,91); 2,5111 (7,26); 2,5069 (9,99); 2,5034 (6,54); 1,9898 (14,31); 1,6154 (0,75); 1,6011 (1,63); 1,5941 (1,77); 1,5811 (0,79); 1,3053 (0,83); 1,2917 (1,6); 1,2849 (1,74); 1,2704 (0,67); 1,2049 (0,71); 1,1943 (4); 1,1765 (8,08); 1,1587 (3,94); 1,1133 (2,92); 1,012 (16); -0,0002 (1,63) |
| Ex.. 24, Solvent: [DMSO], Spektrometer: 399,95MHz |
| 19,0481 (0,34); 12,999 (0,34); 11,6718 (0,32); 11,3817 (2,98); 11,2103 (0,33); 10,7432 (0,44); 10,1704 (0,38); 9,4484 (4,12); 9,4182 (0,34); 7,674 (2,45); 7,6678 (2,75); 7,5767 (3,19); 7,5699 (2,9); 7,471 (0,33); 4,1638 (0,77); 4,0557 (0,89); 4,0379 (3,26); 4,0189 (16); 4,0023 (1,06); 3,9243 (2,59); 3,4462 (0,32); 3,409 (0,48); 3,3939 (0,61); 3,3326 (387,86); 3,3263 (665,26); 3,3027 (8,07); 2,675 (1,43); 2,6707 (2,09); 2,6661 (1,44); 2,5406 (0,85); 2,5238 (3,39); 2,5058 (218,8); 2,5017 (290,84); 2,4581 (0,39); 2,4169 (0,78); 2,3781 (14,51); 2,3549 (0,74); 2,3328 (1,45); 2,3283 (2,04); 2,3241 (1,4); 2,3115 (0,8); 2,2563 (0,65); 2,0733 (1,71); 1,9884 (10,86); 1,6699 (0,36); 1,6549 (0,65); 1,6463 (0,63); 1,6341 (0,43); 1,6004 (1,6); 1,5859 (3,6); 1,5791 (3,91); 1,5661 (1,86); 1,3966 (0,4); 1,3312 (0,52); 1,318 (0,62); 1,3117 (0,71); 1,2978 (0,44); 1,2651 (1,73); 1,2518 (3,47); 1,2451 (3,89); 1,2311 (1,8); 1,217 (0,45); 1,1924 (3,07); 1,1745 (5,77); 1,1568 (2,92); 0,0081 (1,01); -0,0002 (34,96); -0,0085 (1,17);-3,0246 (0,33) |
| Ex.. 25, Solvent: [CD3CN], Spektrometer: 601,6MHz |
| 7,9022 (3,82); 7,8879 (4,08); 7,6652 (3,91); 7,6609 (4,01); 7,52 (1,53); 7,4355 (2,47); 7,4312 (2,34); 7,4212 (2,34); 7,4169 (2,23); 4,0758 (0,58); 4,064 (1,8); 4,0521 (1,78); 4,0403 (0,62); 3,9724 (16); 3,893 (0,72); 2,1596 (251,61); 2,0611 (0,72); 2,057 (1,25); 2,0529 (1,77); 2,0487 (1,27); 2,0447 (0,68); 1,9732 (8,88); 1,9666 (29,58); 1,9584 (12,1); 1,9543 (16,7); 1,9505 (119,09); 1,9464 (214,31); 1,9423 (320,41); 1,9382 (222,08); 1,9341 (110,06); 1,9254 (2,33); 1,917 (0,76); 1,9128 (0,53); 1,9086 (0,36); 1,8357 (0,67); 1,8317 (1,23); 1,8275 (1,73); 1,8234 (1,22); 1,8193 (0,63); 1,5855 (1,79); 1,5758 (4,1); 1,5715 (4,17); 1,5623 (2,12); 1,382 (2,17); 1,3727 (4,02); 1,3684 (4,12); 1,3586 (1,71); 1,2691 (1,08); 1,2158 (2,25); 1,204 (4,73); 1,1922 (2,22); 0,0053 (1,55); -0,0002 (41,75); -0,0058 (1,45) |
| Ex.. 26, Solvent: [DMSO], Spektrometer: 399,95MHz |
| 14,5618 (0,37); 14,1854 (0,35); 9,5578 (1,5); 9,4271 (4,93); 9,2998 (0,36); 9,182 (0,38); 7,7634 (0,46); 7,7069 (1,14); 7,6847 (2,16); 7,5756 (3,33); 7,5541 (4,12); 7,5266 (0,76); 7,4862 (3,47); 7,3446 (0,38); 7,3053 (0,47); 7,275 (2,4); 7,2683 (2,29); 7,2533 (2,08); 7,2469 (1,92); 6,2641 (0,37); 4,9734 (1,6); 4,9321 (1,8); 4,5811 (1,98); 4,537 (3,53); 4,1428 (0,59); 4,0566 (5,17); 4,0378 (2,79); 4,0204 (2,99); 4,0026 (0,88); 3,9185 (0,48); 3,8903 (16); 3,8573 (0,39); 3,427 (1,33); 3,3939 (4,28); 3,3402 (400,48); 3,3359 (525,28); 3,3323 (787,02); 3,3084 (5,46); 3,272 (0,61); 3,2561 (0,46); 3,247 (0,37); 3,1263 (0,42); 2,7317 (0,36); 2,6709 (1,52); 2,5869 (0,39); 2,5415 (0,81); 2,5019 (262,13); 2,3291 (1,73); 2,0734 (1,24); 1,9884 (10,84); 1,5957 (5,63); 1,5917 (5,18); 1,576 (1,96); 1,5443 (0,37); 1,3969 (0,85); 1,278 (1,69); 1,2718 (1,78); 1,2585 (0,99); 1,2348 (1,26); 1,2215 (2,19); 1,207 (4,49); 1,2015 (4,56); 1,1924 (3,48); 1,1863 (1,89); 1,1747 (5,89); 1,1566 (2,78); 1,1479 (0,43); 0,8742 (0,46); 0,8572 (0,47); -0,0002 (33,26); -3,0698 (0,43) |
| Ex.. 27, Solvent: [DMSO], Spektrometer: 399,95MHz |
| 11,4041 (3,19); 9,3381 (2,87); 7,9571 (1,61); 7,9504 (1,81); 7,9418 (1,7); 7,9349 (1,72); 7,8013 (0,9); 7,7942 (0,92); 7,7904 (1,04); 7,7832 (0,95); 7,7788 (1,01); 7,7717 (1,07); 7,7681 (1,06); 7,7609 (0,81); 7,4187 (1,73); 7,3949 (2,44); 7,3715 (1,58); 4,0894 (4,37); 4,0563 (1,29); 4,0384 (4,43); 4,0308 (15,34); 4,0206 (4,05); 4,0029 (1,29); 3,5817 (0,36); 3,5615 (0,4); 3,5454 (0,49); 3,3749 (56,68); 3,1998 (0,54); 3,1712 (0,38); 3,0611 (0,42); 2,6762 (0,39); 2,6716 (0,49); 2,6674 (0,4); 2,5249 (0,94); 2,5068 (58,75); 2,5027 (78,81); 2,4986 (54,63); 2,3339 (0,38); 2,3294 (0,51); 2,325 (0,35); 1,9888 (16); 1,9093 (0,63); 1,5971 (1,56); 1,5828 (3,77); 1,5759 (3,95); 1,5626 (1,86); 1,2977 (2,14); 1,2839 (3,86); 1,2772 (4,13); 1,2626 (1,77); 1,2355 (1,38); 1,1928 (4,29); 1,175 (8,54); 1,1572 (4,24); -0,0002 (11,21); -0,0085 (0,33) |
| Ex.. 28, Solvent: [DMSO], Spektrometer: 399,95MHz |
| 9,475 (0,97); 9,3642 (5,1); 9,3069 (0,43); 8,7987 (0,37); 7,6689 (0,73); 7,5838 (0,66); 7,5622 (0,78); 7,4575 (2,92); 7,4414 (4,62); 7,4312 (1,02); 7,4246 (5,68); 7,42 (6,85); 7,4037 (7,53); 7,3379 (0,95); 7,3163 (1,1); 7,2661 (6,47); 7,2452 (5,07); 7,0778 (1,07); 7,0631 (0,5); 7,0582 (0,85); 6,9726 (1,86); 6,966 (1,92); 6,9512 (1,68); 6,945 (1,6); 5,4201 (2,11); 5,3833 (2,31); 4,9665 (0,6); 4,9267 (2,61); 4,8893 (2,28); 4,0551 (0,77); 4,045 (2,59); 4,0382 (2,1); 4,02 (1,77); 4,0026 (0,63); 3,8971 (16); 3,4603 (0,41); 3,4347 (0,7); 3,4009 (1,13); 3,3491 (582,58); 3,3413 (1358,67); 3,2752 (0,66); 2,6758 (1,21); 2,6711 (1,86); 2,5416 (1,04); 2,5247 (3,14); 2,5068 (206,51); 2,5026 (285,02); 2,4985 (203,82); 2,3336 (1,4); 2,3291 (1,76); 2,073 (2,52); 1,9885 (7,71); 1,6108 (1,9); 1,5968 (4,8); 1,59 (5,1); 1,5767 (2,1); 1,2799 (0,58); 1,2589 (1,23); 1,2344 (1,37); 1,2154 (2,1); 1,2016 (4,33); 1,1931 (5,24); 1,1801 (1,87); 1,1748 (4,28); 1,1569 (2,03); 0,8595 (0,42); 0,6779 (0,36); -0,0002 (6,05) |
| Ex.. 29, Solvent: [DMSO], Spektrometer: 399,95MHz |
| 11,5012 (3,45); 7,8648 (3,09); 7,8592 (3,49); 7,7164 (1,51); 7,7101 (1,37); 7,6943 (2,21); 7,6884 (2,2); 7,6159 (3,95); 7,594 (2,49); 4,0557 (0,65); 4,0379 (1,84); 4,0213 (16); 4,0026 (0,71); 3,4274 (0,34); 3,3515 (266,05); 3,349 (285,95); 3,3448 (214,54); 3,3422 (330,15); 3,3382 (346,98); 3,3349 (418,93); 3,293 (1,7); 3,2756 (1,97); 3,2593 (1,38); 3,2431 (0,63); 2,6712 (1,08); 2,6665 (0,8); 2,5414 (0,49); 2,5021 (170,74); 2,3288 (1,03); 2,3248 (0,78); 2,0733 (0,83); 1,9886 (6,8); 1,9206 (1,12); 1,8997 (3,5); 1,8826 (1,26); 1,5485 (1,15); 1,5306 (3,67); 1,5136 (0,94); 1,2328 (0,5); 1,1925 (1,89); 1,1747 (3,72); 1,1569 (1,93); 1,121 (14,9); 1,1043 (14,88); -0,0002 (10,09) |
| Ex.. 30, Solvent: [DMSO], Spektrometer: 399,95MHz |
| 17,8767 (0,49); 16,7278 (0,5); 15,2907 (0,46); 14,6922 (0,48); 11,3194 (0,47); 9,9917 (0,45); 9,5478 (1,93); 9,4196 (4,84); 7,9522 (1,92); 7,697 (1,24); 7,6759 (1,54); 7,6545 (1,48); 7,6289 (0,45); 7,5637 (3,75); 7,5423 (4,82); 7,5163 (0,81); 7,4807 (3,23); 7,4748 (3,56); 7,2683 (2,26); 7,2621 (1,93); 7,2473 (1,94); 7,24 (1,91); 6,5709 (0,48); 6,1831 (0,45); 4,9061 (1,2); 4,8649 (1,59); 4,5294 (1,43); 4,5247 (1,64); 4,4875 (1,39); 4,4825 (1,3); 4,4256 (1,93); 4,1384 (0,72); 4,0647 (6,28); 4,0384 (0,58); 4,0199 (0,67); 3,9606 (0,54); 3,9181 (0,47); 3,8854 (16); 3,759 (0,48); 3,4178 (0,55); 3,3771 (1,43); 3,3282 (1057,25); 3,251 (0,64); 2,8906 (12,28); 2,7309 (10,63); 2,6754 (1,93); 2,6707 (2,33); 2,5407 (1,33); 2,5057 (285,9); 2,5017 (383,65); 2,4982 (282,92); 2,3283 (2,31); 2,0733 (2,62); 1,9883 (1,97); 1,8712 (0,61); 1,7761 (11,28); 1,7311 (4,12); 1,6113 (1,99); 1,5966 (6,09); 1,5904 (5,32); 1,5777 (2,22); 1,2931 (0,88); 1,279 (1,92); 1,273 (2,06); 1,2578 (0,96); 1,2365 (1,31); 1,2229 (1,94); 1,2088 (4,32); 1,2025 (4,5); 1,1879 (1,67); 1,1747 (0,96); 1,1567 (0,93); 0,0078 (1,82); -0,0002 (58,33); -0,0086 (1,83); -2,3925 (0,46); -2,4975 (0,45); -3,438 (0,46) |
| Ex.. 31, Solvent: [DMSO], Spektrometer: 399,95MHz |
| 9,6271 (0,33); 9,4864 (1,04); 9,3652 (4,55); 7,6886 (0,76); 7,6822 (0,75); 7,5821 (0,72); 7,5605 (1,05); 7,4744 (2,86); 7,4687 (2,84); 7,4508 (0,45); 7,4243 (4,62); 7,4029 (5,04); 7,3666 (0,94); 7,362 (1,49); 7,3577 (0,73); 7,345 (4,29); 7,341 (2,48); 7,3269 (4,69); 7,3153 (1,34); 7,311 (2,53); 7,3074 (1,78); 7,3007 (0,69); 7,2937 (2,58); 7,2842 (0,59); 7,2752 (0,73); 7,2699 (0,96); 7,2509 (1,5); 7,2417 (3,98); 7,2377 (4,77); 7,2211 (3,59); 7,0343 (0,74); 7,0277 (0,66); 7,0153 (0,67); 6,96 (1,73); 6,9536 (1,69); 6,9387 (1,64); 6,9318 (1,6); 5,4626 (2,05); 5,4257 (2,49); 4,9216 (1,23); 4,9128 (2,37); 4,8762 (2,17); 4,0758 (0,37); 4,0556 (0,88); 4,0379 (2,56); 4,0201 (2,74); 4,0023 (0,88); 3,9667 (3,2); 3,9535 (0,34); 3,8996 (16); 3,3929 (0,35); 3,3332 (300,56); 3,3257 (628,51); 3,3027 (7,43); 2,6752 (1,18); 2,6706 (1,5); 2,6659 (1,01); 2,5407 (0,85); 2,524 (2,63); 2,5193 (3,68); 2,5058 (169,66); 2,5016 (233,25); 2,4977 (156,25); 2,3329 (1,14); 2,3282 (1,65); 2,3237 (1,1); 2,0733 (1,21); 1,9884 (11,36); 1,6066 (1,94); 1,5924 (4,57); 1,5856 (4,63); 1,5728 (2,04); 1,2823 (0,53); 1,2698 (0,96); 1,2621 (1,07); 1,2482 (0,71); 1,235 (1,64); 1,2116 (1,99); 1,198 (3,98); 1,1921 (7,04); 1,1746 (6,85); 1,1567 (3,11); 0,1462 (0,36); 0,0081 (2); -0,0002 (76,87); -0,0085 (2,13); -0,1493 (0,33) |
| Ex.. 32, Solvent: [DMSO], Spektrometer: 399,95MHz |
| 19,4883 (0,33); 14,4743 (0,34); 9,9628 (0,33); 9,4772 (1,71); 9,366 (4,51); 8,5874 (0,8); 8,5782 (0,78); 8,5563 (2,19); 8,547 (2,12); 7,8384 (1,13); 7,8194 (2,22); 7,8147 (2,2); 7,8004 (1,32); 7,7955 (1,32); 7,7145 (0,49); 7,6895 (0,9); 7,6743 (0,51); 7,6709 (0,55); 7,5658 (1,2); 7,5441 (1,65); 7,5333 (1,55); 7,5212 (3,6); 7,5155 (3,87); 7,4745 (3,75); 7,4531 (4,46); 7,4106 (3,5); 7,3909 (2,71); 7,3429 (1,51); 7,3315 (1,79); 7,3238 (1,72); 7,3124 (1,73); 7,2885 (0,58); 7,2779 (0,44); 7,2717 (2,25); 7,265 (2,21); 7,2499 (1,86); 7,2432 (1,77); 7,1046 (0,9); 7,0848 (0,86); 5,296 (1,4); 5,256 (3,53); 5,2123 (3,53); 5,1725 (1,5); 4,9686 (2,74); 4,1368 (5,32); 4,0557 (0,94); 4,0379 (2,86); 4,0202 (2,95); 3,9976 (16); 3,3915 (0,41); 3,3305 (304,35); 3,3279 (296,34); 3,3249 (391,32); 3,2506 (0,34); 2,6708 (1,53); 2,5409 (0,84); 2,5016 (246,17); 2,4386 (0,36); 2,4326 (0,43); 2,3282 (1,61); 2,0734 (1,6); 1,9885 (11,41); 1,9079 (0,43); 1,6004 (2,1); 1,5867 (5,4); 1,5798 (5,52); 1,5671 (2,28); 1,2692 (0,65); 1,2565 (1,56); 1,2491 (1,54); 1,235 (1,41); 1,2013 (1,93); 1,192 (5,11); 1,1816 (4,31); 1,1746 (6,69); 1,1672 (1,83); 1,1569 (3,02); 0,0075 (2,36); -0,0002 (43,57); -1,8063 (0,33) |
| Ex.. 33, Solvent: [DMSO], Spektrometer: 399,95MHz |
| 9,5775 (4,49); 9,5053 (0,45); 7,7786 (3,6); 7,7682 (0,92); 7,7572 (4,87); 7,7138 (0,34); 7,692 (0,35); 7,5946 (0,68); 7,573 (0,96); 4,0552 (0,63); 4,0374 (1,94); 4,0273 (2,07); 4,0163 (16); 4,002 (0,93); 3,4947 (0,62); 3,4821 (0,65); 3,3765 (615,2); 3,372 (488,62); 3,3684 (526,05); 3,3645 (546,77); 3,3625 (654,08); 3,359 (560,12); 3,2239 (0,35); 2,6782 (1,18); 2,6736 (1,67); 2,669 (1,25); 2,5438 (0,92); 2,527 (3); 2,5222 (4,56); 2,5136 (82,6); 2,5091 (180,41); 2,5045 (246,26); 2,5 (178,36); 2,4955 (86,07); 2,3358 (1,2); 2,3312 (1,65); 2,3267 (1,27); 2,2807 (1,71); 2,2634 (1,73); 2,2212 (0,46); 2,2023 (0,41); 2,0759 (1,71); 1,9902 (8,07); 1,6323 (1,61); 1,6181 (3,95); 1,6112 (4,4); 1,5979 (2,13); 1,3975 (0,64); 1,3544 (0,4); 1,2986 (2,04); 1,2849 (4,05); 1,2782 (4,47); 1,2636 (1,91); 1,2528 (0,8); 1,2356 (1,05); 1,1924 (2,24); 1,1746 (4,33); 1,1568 (2,15); 1,0046 (0,45); 0,9858 (0,8); 0,967 (0,39); 0,9035 (4,02); 0,8858 (8,59); 0,8681 (3,9); 0,008 (0,47); -0,0002 (15,07); -0,0086 (0,55) |
| Ex.. 34, Solvent: [DMSO], Spektrometer: 601,6MHz |
| 11,5357 (2,35); 9,5094 (2,93); 7,7744 (2,41); 7,7701 (2,69); 7,7094 (1,22); 7,7051 (1,07); 7,6949 (1,54); 7,6906 (1,44); 7,5923 (2,62); 7,5777 (2,13); 4,3468 (0,77); 4,3348 (2,43); 4,3228 (2,45); 4,3107 (0,79); 4,0465 (1,21); 4,0346 (3,71); 4,0228 (3,71); 4,011 (1,24); 3,3485 (39,29); 3,063 (1,4); 2,8638 (1,42); 2,5098 (6,24); 2,5069 (13,35); 2,5039 (18,23); 2,5009 (13,5); 2,498 (6,41); 1,991 (16); 1,6057 (1,04); 1,5963 (2,46); 1,5918 (2,7); 1,5829 (1,07); 1,4232 (3,53); 1,4112 (7,51); 1,3991 (3,52); 1,3831 (0,35); 1,371 (0,73); 1,359 (0,33); 1,2704 (1,14); 1,2612 (2,43); 1,2568 (2,67); 1,2472 (1); 1,1869 (4,33); 1,175 (8,76); 1,1632 (4,27); -0,0002 (1,62) |
| Ex.. 35, Solvent: [DMSO], Spektrometer: 399,95MHz |
| 11,5005 (4,87); 9,4951 (6,27); 7,7618 (4,81); 7,7554 (5,95); 7,7144 (2,63); 7,7078 (2); 7,6925 (3,37); 7,686 (2,85); 7,5904 (5,35); 7,5686 (3,92); 4,2725 (3,11); 4,2554 (6,27); 4,2381 (3,15); 4,0382 (0,36); 4,0188 (0,33); 3,4841 (0,32); 3,4614 (0,46); 3,4443 (0,55); 3,4137 (0,86); 3,3433 (1492,06); 3,2822 (3,03); 3,25 (1,84); 3,2136 (1,16); 3,1836 (0,68); 3,1568 (0,48); 3,0604 (1,58); 2,8553 (1,58); 2,6765 (0,88); 2,6715 (1,15); 2,6668 (0,89); 2,5808 (0,37); 2,5419 (0,94); 2,5068 (140,47); 2,5027 (192,83); 2,4986 (142,05); 2,4395 (1,46); 2,4062 (0,88); 2,4024 (0,84); 2,3507 (0,48); 2,3295 (1,39); 2,3251 (1,13); 2,3016 (0,38); 2,0748 (2,08); 1,9892 (1,51); 1,8724 (0,6); 1,8543 (2,3); 1,8363 (4,51); 1,8184 (4,61); 1,8002 (2,47); 1,7815 (0,63); 1,6082 (2,21); 1,5942 (5,23); 1,5868 (5,61); 1,5738 (2,55); 1,5334 (0,32); 1,3974 (9,15); 1,2748 (2,5); 1,2617 (5,29); 1,2545 (5,68); 1,24 (2,34); 1,1923 (0,45); 1,1741 (0,77); 1,1568 (0,42); 0,8614 (7,69); 0,8431 (16); 0,8244 (7,18); -0,0002 (12,56); -0,0087 (0,62) |
| Ex.. 36, Solvent: [DMSO], Spektrometer: 601,6MHz |
| 11,4548 (1,5); 9,3583 (1,27); 7,9479 (0,56); 7,9434 (0,64); 7,9378 (0,61); 7,9332 (0,58); 7,7878 (0,37); 7,7856 (0,39); 7,7807 (0,35); 7,7778 (0,36); 7,7728 (0,41); 7,771 (0,4); 7,4149 (0,55); 7,3992 (0,89); 7,3837 (0,52); 4,2745 (0,87); 4,2629 (1,71); 4,2513 (0,87); 4,0461 (1,23); 4,0343 (3,73); 4,0224 (3,73); 4,0106 (1,24); 3,3508 (5,32); 3,0615 (0,46); 2,857 (0,46); 2,5216 (0,38); 2,5067 (18,22); 2,5038 (24,1); 2,5008 (17,69); 1,9909 (16); 1,8508 (0,62); 1,8388 (1,21); 1,8269 (1,23); 1,8149 (0,65); 1,5953 (0,63); 1,5859 (1,53); 1,5813 (1,8); 1,5724 (0,68); 1,2932 (0,72); 1,2841 (1,56); 1,2796 (1,68); 1,2702 (0,7); 1,1866 (4,25); 1,1747 (8,49); 1,1629 (4,18); 0,9297 (0,46); 0,8596 (1,94); 0,8473 (4,1); 0,835 (1,9); 0,8263 (0,36); -0,0002 (1,87) |
| Ex.. 37, Solvent: [DMSO], Spektrometer: 399,95MHz |
| 11,4582 (4,4); 9,3437 (3,47); 7,9546 (1,99); 7,9477 (2,21); 7,9391 (2,06); 7,9323 (2,22); 7,7969 (1,11); 7,7896 (1,15); 7,7858 (1,31); 7,7786 (1,09); 7,7744 (1,19); 7,7674 (1,35); 7,7633 (1,35); 7,7565 (1,05); 7,4195 (2,16); 7,3953 (2,98); 7,3726 (1,97); 4,4209 (0,48); 4,402 (0,49); 4,3576 (1,39); 4,3397 (4,42); 4,3215 (4,47); 4,3031 (1,4); 4,2264 (0,44); 4,209 (0,72); 4,1911 (0,99); 4,1728 (0,68); 4,0554 (1,12); 4,0376 (3,61); 4,0198 (3,61); 4,002 (1,17); 3,4598 (0,4); 3,3366 (26,64); 3,2408 (0,63); 3,2327 (0,6); 3,18 (0,49); 3,0617 (7,35); 2,9068 (0,33); 2,862 (7,44); 2,6758 (0,84); 2,6713 (1,17); 2,6666 (0,87); 2,5416 (0,6); 2,5245 (2,07); 2,5199 (2,85); 2,5063 (123,98); 2,5022 (170,52); 2,4985 (114,01); 2,3384 (0,38); 2,3333 (0,8); 2,3286 (1,21); 2,3245 (0,85); 2,3194 (0,49); 2,0755 (0,47); 1,9893 (16); 1,9091 (0,6); 1,5982 (1,91); 1,5841 (4,44); 1,5769 (4,72); 1,5638 (2,26); 1,4339 (6,76); 1,4158 (15,95); 1,3976 (7,06); 1,3891 (2,15); 1,3757 (2,45); 1,371 (4,02); 1,3576 (1,14); 1,3528 (1,86); 1,2979 (2,59); 1,2842 (4,49); 1,2773 (4,85); 1,2627 (2,22); 1,2354 (0,95); 1,1923 (4,5); 1,1745 (8,88); 1,1567 (4,35); 0,8883 (0,35); 0,008 (0,92); -0,0002 (35,96); -0,0085 (1,05) |
| Ex.. 38, Solvent: [DMSO], Spektrometer: 601,6MHz |
| 9,5496 (0,57); 9,4141 (0,99); 7,9531 (2,11); 7,6736 (0,9); 7,6699 (0,6); 7,6588 (0,63); 7,5771 (0,34); 7,5534 (0,98); 7,5391 (1,1); 7,433 (0,87); 7,4286 (0,92); 7,2912 (0,53); 7,2868 (0,49); 7,2769 (0,48); 7,2725 (0,45); 4,3763 (0,46); 4,3643 (0,47); 4,2607 (0,37); 4,1747 (0,32); 4,1628 (0,38); 3,4447 (5,41); 3,3584 (170,61); 3,335 (1,41); 3,2387 (2,83); 2,8911 (16); 2,7312 (13,04); 2,7307 (12,77); 2,5251 (0,36); 2,522 (0,46); 2,5189 (0,48); 2,5099 (12,87); 2,507 (27,63); 2,504 (37,72); 2,501 (27,87); 2,4981 (13,11); 1,6046 (0,84); 1,6005 (0,7); 1,5953 (0,94); 1,5911 (1,16); 1,5824 (0,42); 1,4525 (0,74); 1,4405 (1,57); 1,4285 (0,74); 1,308 (1,26); 1,296 (2,69); 1,2908 (0,39); 1,284 (1,46); 1,2771 (0,59); 1,1964 (0,45); 1,1876 (0,87); 1,1835 (0,88); 1,1744 (0,48); -0,0002 (5,92) |
| Ex.. 39, Solvent: [DMSO], Spektrometer: 399,95MHz |
| 16,4832 (0,33); 15,8688 (0,33); 13,2954 (0,33); 9,6257 (0,34); 9,6097 (2,28); 9,4715 (4,25); 9,4357 (0,37); 9,3286 (0,34); 8,0115 (1,98); 8,0058 (2,14); 7,6392 (4,36); 7,6335 (3,82); 7,6013 (0,4); 7,5949 (0,44); 7,5777 (0,47); 7,5615 (0,48); 7,5253 (0,37); 7,4896 (3,22); 7,4834 (3,02); 7,4395 (0,43); 7,4325 (0,57); 7,4255 (0,52); 7,4196 (0,37); 5,7522 (0,33); 4,1774 (0,4); 4,1601 (0,99); 4,1421 (1,31); 4,1253 (1,47); 4,1073 (1,28); 4,0847 (8,62); 4,0554 (1,29); 4,0376 (3,86); 4,0199 (3,78); 4,0022 (1,25); 3,9064 (0,38); 3,8793 (1,92); 3,8664 (14,52); 3,8554 (1,87); 3,8367 (0,71); 3,8182 (1,21); 3,8004 (1,64); 3,7833 (1,43); 3,7657 (1,14); 3,7471 (0,43); 3,7013 (0,51); 3,6822 (0,73); 3,6642 (0,9); 3,6454 (1); 3,6338 (0,83); 3,6158 (0,91); 3,5968 (0,64); 3,5781 (0,38); 3,4622 (0,34); 3,4401 (0,39); 3,4079 (0,75); 3,3466 (768,76); 3,3431 (1758,18); 3,3199 (5,3); 3,2876 (0,84); 3,2746 (0,59); 3,2684 (0,52); 3,2291 (0,35); 3,2188 (0,44); 2,6767 (1,39); 2,6717 (1,92); 2,6678 (1,4); 2,5418 (0,89); 2,5252 (3,39); 2,5111 (113,97); 2,5071 (219,12); 2,5029 (302,49); 2,4987 (211,86); 2,4946 (105,8); 2,4557 (0,43); 2,3342 (1,35); 2,3295 (1,8); 2,3249 (1,43); 2,075 (2,09); 1,9893 (16); 1,6178 (2,02); 1,6037 (6,07); 1,5977 (5,35); 1,5842 (2,19); 1,5755 (0,39); 1,3835 (0,33); 1,3449 (0,36); 1,3127 (1,03); 1,298 (2,27); 1,2915 (2,11); 1,2761 (0,89); 1,2588 (0,57); 1,2327 (3,01); 1,2191 (4,19); 1,2121 (4,3); 1,1979 (1,6); 1,1924 (4,68); 1,1746 (8,79); 1,1567 (8,3); 1,1383 (9,27); 1,1204 (4,08); 1,0992 (0,35); 0,9885 (0,33); 0,9703 (2,29); 0,9523 (4,58); 0,9345 (2,19); -0,0002 (11,63); -0,0088 (0,43); -3,0168 (0,32) |
| Ex.. 40, Solvent: [DMSO], Spektrometer: 399,95MHz |
| 11,491 (2,41); 7,8981 (2,63); 7,8918 (2,76); 7,7092 (1,29); 7,7028 (1,18); 7,6873 (1,79); 7,6809 (1,74); 7,5849 (3,2); 7,563 (2,31); 4,056 (0,73); 4,0381 (2,56); 4,0288 (11,26); 4,0205 (2,67); 4,0026 (0,76); 3,3518 (0,45); 3,3286 (152,33); 2,5244 (1,09); 2,5195 (1,69); 2,5109 (19,1); 2,5065 (38,19); 2,502 (49,79); 2,4974 (35,73); 2,493 (17,31); 2,3955 (16); 2,3286 (0,34); 2,074 (0,42); 1,9888 (9,22); 1,8766 (1,77); 1,6065 (0,84); 1,1926 (2,46); 1,1748 (4,87); 1,157 (2,4); -0,0002 (5,14) |
| Ex.. 41, Solvent: [DMSO], Spektrometer: 601,6MHz |
| 9,4121 (0,42); 7,9528 (1,18); 7,7388 (0,36); 7,7028 (0,82); 7,6882 (1,07); 7,674 (0,37); 7,6583 (0,38); 7,6479 (0,99); 7,6438 (1,12); 7,6126 (1,11); 7,5995 (1,46); 7,5769 (0,36); 7,5533 (0,48); 7,5395 (0,48); 7,433 (0,5); 7,4291 (0,53); 7,3221 (0,64); 7,2903 (0,44); 7,2761 (0,33); 7,2716 (0,34); 4,3716 (1,39); 4,3595 (1,67); 4,272 (0,39); 4,2601 (0,39); 4,2481 (0,36); 4,1277 (0,42); 4,1191 (0,42); 4,0334 (0,56); 4,0216 (0,56); 3,5061 (0,42); 3,4808 (1,12); 3,4549 (16); 3,444 (2,94); 3,4284 (0,6); 3,3956 (1,34); 3,3758 (5,24); 3,3517 (4904,54); 3,3282 (80,11); 3,2755 (1,57); 3,2474 (4,78); 3,2383 (1,31); 3,1704 (1,74); 3,1617 (1,71); 3,1108 (1,82); 3,0743 (1,14); 2,8906 (8,65); 2,8645 (4,58); 2,7306 (7,07); 2,618 (10,41); 2,615 (14,06); 2,6121 (10,4); 2,5428 (5,84); 2,5243 (20,27); 2,5213 (25,42); 2,5182 (25,36); 2,5091 (738,32); 2,5062 (1554,79); 2,5033 (2093,86); 2,5003 (1556,28); 2,4974 (750,49); 2,3904 (9,64); 2,3874 (13,16); 2,3845 (9,61); 2,283 (0,4); 2,0787 (15,29); 1,9906 (2,25); 1,6777 (3,23); 1,6044 (0,51); 1,591 (0,77); 1,582 (0,49); 1,5066 (0,63); 1,4948 (0,86); 1,4825 (0,67); 1,4514 (2,04); 1,4394 (4,05); 1,4273 (2,94); 1,415 (1,05); 1,3496 (2,36); 1,3072 (1,28); 1,2954 (1,78); 1,2833 (1,18); 1,2666 (0,69); 1,2576 (1,03); 1,2338 (6,68); 1,1951 (0,48); 1,1859 (1,2); 1,174 (1,49); 1,1622 (0,72); 1,0541 (0,42); 0,8646 (0,43); 0,8535 (1,03); 0,8416 (0,52); 0,0965 (1,89); 0,0052 (14,67); -0,0002 (473,67);-0,0057 (19,93); -0,1 (1,92) |
| Ex.. 42, Solvent: [DMSO], Spektrometer: 601,6MHz |
| 11,0933 (3,12); 10,6126 (0,51); 9,5132 (3,58); 9,5027 (0,8); 7,8641 (2,73); 7,86 (2,86); 7,8428 (1,09); 7,839 (0,51); 7,8313 (0,4); 7,7552 (1,31); 7,751 (1,2); 7,7406 (1,56); 7,7365 (1,44); 7,5785 (3,5); 7,564 (2,35); 7,5578 (0,51); 7,5418 (0,41); 4,1945 (2,61); 4,0334 (0,76); 4,0215 (0,81); 4,0064 (12,33); 3,3813 (1,49); 3,3561 (1038,24); 3,3325 (10,5); 2,6153 (1,84); 2,543 (0,72); 2,5245 (2,78); 2,5214 (3,59); 2,5181 (4,4); 2,5063 (218,81); 2,5036 (275,25); 2,3877 (1,67); 2,2932 (0,59); 2,2827 (16); 2,0785 (0,51); 1,9907 (3,1); 1,6109 (1,37); 1,6016 (3,69); 1,5971 (3,87); 1,5881 (1,44); 1,2646 (1,41); 1,2554 (3,39); 1,2511 (3,52); 1,2413 (1,86); 1,2344 (0,88); 1,1859 (0,82); 1,1741 (1,59); 1,1622 (0,81); 0,0049 (1,62); -0,0002 (30,57) |
| Ex.. 43, Solvent: [DMSO], Spektrometer: 601,6MHz |
| 9,5611 (2,61); 9,4204 (5,99); 7,6917 (2,08); 7,6775 (2,5); 7,644 (2,35); 7,6398 (2,43); 7,5687 (5,27); 7,5544 (5,96); 7,5008 (1,35); 7,4967 (1,29); 7,4867 (1,16); 7,4825 (1,15); 7,4199 (4,23); 7,4158 (4,42); 7,2921 (2,65); 7,288 (2,5); 7,2779 (2,46); 7,2736 (2,33); 4,3914 (0,77); 4,3788 (2,21); 4,367 (2,26); 4,3549 (0,78); 4,268 (0,45); 4,2567 (1,31); 4,2449 (1,84); 4,2335 (2,3); 4,2217 (1,95); 4,21 (0,64); 4,1658 (0,64); 4,1541 (1,99); 4,1423 (2,5); 4,1343 (1,96); 4,1311 (2,19); 4,1224 (2,3); 4,1193 (1,88); 4,1114 (2,32); 4,0996 (1,8); 4,0878 (0,56); 4,0334 (0,37); 4,0216 (0,36); 3,7795 (0,53); 3,7678 (1,63); 3,7559 (2,07); 3,7448 (1,99); 3,7328 (1,56); 3,7214 (0,53); 3,6918 (0,56); 3,68 (0,78); 3,6676 (0,94); 3,6558 (0,78); 3,5796 (0,83); 3,5678 (1); 3,5556 (0,85); 3,5436 (0,6); 3,4046 (0,45); 3,3945 (1,21); 3,3858 (1,16); 3,3796 (1,18); 3,3518 (1984,37); 3,3279 (16,06); 3,3028 (0,46); 3,1702 (0,33); 3,1616 (0,43); 2,6179 (4,37); 2,6151 (5,71); 2,6123 (4,23); 2,5427 (2,81); 2,5332 (0,94); 2,5242 (9,59); 2,5211 (13,27); 2,5179 (16,58); 2,5062 (657,61); 2,5033 (846,44); 2,5005 (615,2); 2,4784 (2,21); 2,3903 (3,99); 2,3875 (5,24); 2,3847 (3,83); 2,0787 (4,37); 1,9906 (1,25); 1,6177 (1,12); 1,6056 (4,24); 1,5967 (6,28); 1,5925 (6,54); 1,5838 (2,53); 1,4508 (3,21); 1,4388 (6,55); 1,4268 (3,06); 1,306 (7,37); 1,2941 (16); 1,282 (9,65); 1,2721 (1,16); 1,2576 (0,69); 1,2338 (3,09); 1,2054 (2,8); 1,1965 (5,73); 1,1922 (5,97); 1,183 (2,39); 1,1741 (0,91); 1,1621 (0,47); 1,1436 (6,79); 1,1317 (13,97); 1,1198 (6,51); 0,9438 (2,6); 0,932 (5,36); 0,9202 (2,47); 0,8535 (0,4); 0,0967 (0,49); 0,005 (5,69); -0,0002 (104,53); -0,0999 (0,47) |
| Ex.. 44, Solvent: [DMSO], Spektrometer: 601,6MHz |
| 9,4685 (3,13); 7,6186 (2,15); 7,6043 (2,37); 7,5156 (2,09); 7,5118 (2,18); 7,3065 (1,24); 7,3024 (1,24); 7,2923 (1,16); 7,2882 (1,12); 5,2573 (1,78); 5,2279 (2,2); 4,9892 (2,11); 4,9598 (1,74); 4,0648 (0,9); 4,0453 (1,36); 4,0335 (3,87); 4,0217 (3,86); 4,0098 (1,32); 3,9354 (10,54); 3,4336 (0,7); 3,3766 (1,21); 3,352 (365,17); 3,3252 (0,47); 2,6151 (0,96); 2,5429 (0,42); 2,5034 (150,98); 2,3876 (0,93); 1,9907 (16); 1,6188 (1,49); 1,6097 (3,52); 1,6054 (3,55); 1,5965 (1,28); 1,2734 (0,4); 1,2338 (0,67); 1,217 (1,28); 1,2079 (2,97); 1,2038 (3,11); 1,1942 (1,13); 1,186 (4,37); 1,1742 (8,34); 1,1623 (4,17); -0,0002 (1,27) |
| Ex.. 45, Solvent: [DMSO], Spektrometer: 399,95MHz |
| 11,5629 (3,17); 9,5197 (3,89); 7,8939 (0,68); 7,8145 (2,97); 7,8081 (3,34); 7,6813 (1,42); 7,675 (1,32); 7,6594 (2,05); 7,653 (2,01); 7,5849 (4,03); 7,563 (2,7); 7,5353 (0,32); 5,7625 (0,52); 4,1942 (1); 4,1104 (12,73); 4,0549 (0,57); 4,0371 (1,62); 4,0193 (1,67); 4,0016 (0,65); 3,3488 (381,8); 3,3413 (634,34); 3,0337 (16); 2,681 (0,92); 2,6766 (1,94); 2,672 (2,7); 2,6675 (1,97); 2,5421 (1,6); 2,5254 (4,95); 2,5207 (7,26); 2,512 (132,84); 2,5075 (285,73); 2,503 (383,95); 2,4984 (271,1); 2,4939 (125,41); 2,3389 (0,83); 2,3343 (1,83); 2,3297 (2,55); 2,3251 (1,84); 2,0772 (0,62); 1,99 (7,2); 1,612 (1,36); 1,5977 (3,37); 1,5909 (3,47); 1,5778 (1,53); 1,2731 (1,73); 1,2596 (3,49); 1,2529 (3,76); 1,2378 (2,87); 1,1921 (1,91); 1,1742 (3,78); 1,1565 (1,84); 0,8539 (0,37); 0,008 (1,19); -0,0002 (35,07); -0,0077 (1,04) |
| Ex.. 45, Solvent: [DMSO], Spektrometer: 399,95MHz |
| 11,5617 (2,61); 9,5199 (2,8); 7,8135 (2,59); 7,8072 (2,92); 7,6806 (1); 7,6741 (0,94); 7,6586 (1,53); 7,6522 (1,56); 7,5846 (2,73); 7,5627 (1,8); 4,1102 (12,24); 4,0369 (0,87); 4,0191 (0,88); 4,0016 (0,61); 3,3499 (1797,52); 3,2245 (0,46); 3,033 (16); 2,6813 (0,63); 2,6767 (1,35); 2,6721 (1,89); 2,6675 (1,39); 2,6629 (0,67); 2,5424 (1,08); 2,5255 (3,44); 2,5208 (5,1); 2,5121 (94,77); 2,5076 (206,74); 2,503 (279,76); 2,4984 (197,63); 2,4939 (91,37); 2,3389 (0,58); 2,3343 (1,29); 2,3297 (1,81); 2,3252 (1,31); 2,0765 (2,83); 1,9898 (4,26); 1,6115 (1,29); 1,5972 (2,98); 1,5904 (3,18); 1,5772 (1,42); 1,273 (1,66); 1,2592 (3,23); 1,2526 (3,51); 1,2379 (1,98); 1,192 (1,13); 1,1742 (2,25); 1,1564 (1,08); -0,0002 (8,57) |
| Ex.. 46, Solvent: [DMSO], Spektrometer: 399,95MHz |
| 9,5373 (0,47); 9,3983 (0,81); 7,7053 (0,41); 7,6838 (0,63); 7,6757 (0,42); 7,6635 (0,45); 7,6546 (0,75); 7,6313 (0,77); 7,6092 (1,26); 7,5886 (1,33); 7,5792 (0,79); 7,5486 (0,9); 7,527 (0,97); 7,4347 (0,79); 7,4282 (0,89); 7,3883 (0,47); 7,3078 (1,04); 7,2891 (0,66); 7,2824 (0,56); 7,2677 (0,47); 7,2612 (0,45); 4,2924 (1,02); 4,2751 (0,9); 4,182 (0,47); 4,1649 (0,51); 4,1476 (0,56); 4,1292 (0,35); 4,0547 (1,37); 4,0369 (3,7); 4,0191 (3,74); 4,0013 (1,31); 3,4557 (10,09); 3,4453 (5,02); 3,3427 (771,28); 3,319 (13,1); 3,2679 (1,37); 3,2419 (3,06); 3,2325 (2,72); 3,1112 (1,16); 3,072 (0,85); 2,8904 (0,43); 2,8646 (2,78); 2,7312 (0,35); 2,6764 (1,44); 2,6718 (2); 2,6673 (1,53); 2,5422 (1,53); 2,5253 (3,59); 2,5205 (5,22); 2,5117 (95,58); 2,5073 (207,41); 2,5028 (281,65); 2,4982 (204,86); 2,4938 (99,47); 2,334 (1,35); 2,3295 (1,9); 2,325 (1,43); 2,0769 (2,16); 1,9899 (16); 1,9015 (0,53); 1,8832 (1); 1,8655 (1,18); 1,847 (0,97); 1,8267 (0,75); 1,8103 (0,56); 1,7924 (0,52); 1,7738 (0,52); 1,6767 (2,49); 1,6439 (0,6); 1,6192 (0,38); 1,5969 (1,32); 1,5899 (1,04); 1,5768 (0,52); 1,4012 (0,6); 1,2967 (0,71); 1,2823 (0,75); 1,2755 (0,81); 1,258 (0,71); 1,2347 (1,66); 1,2004 (0,58); 1,192 (4,72); 1,1743 (9,08); 1,1564 (4,38); 0,8945 (1,45); 0,876 (3,76); 0,8567 (3,53); 0,8335 (2,44); 0,8148 (2,63); 0,7962 (1,13); 0,146 (0,39); 0,008 (2,78); -0,0002 (90,84); -0,0085 (3,33); -0,1499 (0,34) |
| Ex.. 47, Solvent: [DMSO], Spektrometer: 601,6MHz |
| 7,6521 (0,53); 7,4488 (0,87); 7,4092 (0,79); 7,3946 (0,85); 7,3798 (0,87); 4,3079 (0,35); 4,2962 (0,64); 4,2842 (0,81); 4,2717 (0,82); 4,2604 (0,93); 4,2489 (1,03); 4,2369 (0,57); 4,1612 (0,58); 4,1506 (0,59); 4,1386 (0,49); 4,127 (0,54); 4,1149 (0,59); 4,1037 (0,33); 4,0456 (0,57); 4,0338 (1,71); 4,022 (1,73); 4,0101 (0,58); 3,4532 (16); 3,4036 (1,29); 3,3797 (0,39); 3,3536 (287,87); 3,33 (4,06); 3,2402 (1,45); 3,1042 (0,53); 3,095 (0,47); 3,0631 (0,57); 2,9405 (0,67); 2,6832 (2,06); 2,6186 (0,56); 2,6157 (0,74); 2,6128 (0,54); 2,5249 (1,23); 2,5218 (1,83); 2,5069 (84,85); 2,504 (111,28); 2,501 (80,94); 2,391 (0,55); 2,3881 (0,73); 2,3852 (0,53); 1,9909 (7,37); 1,8792 (0,58); 1,8669 (0,68); 1,8549 (0,61); 1,8371 (0,59); 1,7449 (0,46); 1,7341 (0,59); 1,7232 (0,61); 1,7109 (0,61); 1,6979 (0,68); 1,686 (0,86); 1,6637 (1,33); 1,4754 (0,37); 1,3882 (0,95); 1,2578 (0,37); 1,2343 (1,25); 1,1862 (2,13); 1,1744 (4,02); 1,1625 (2,03); 0,8889 (1,11); 0,8766 (2,26); 0,8638 (2,34); 0,8493 (3,1); 0,6607 (0,53); 0,6484 (1,06); 0,6361 (0,51); 0,0051 (1,29); -0,0002 (25,89); -0,0056 (0,98) |
| Ex.. 48, Solvent: [DMSO], Spektrometer: 601,6MHz |
| 7,6625 (0,59); 7,6566 (0,58); 7,6531 (0,62); 7,441 (0,93); 7,4125 (0,97); 7,3978 (1,05); 4,3751 (0,97); 4,3634 (1,3); 4,3523 (1,07); 4,3412 (1,13); 4,3294 (0,96); 4,3177 (0,36); 4,2724 (0,53); 4,0335 (0,92); 4,0217 (0,91); 3,4526 (16); 3,3768 (0,53); 3,3522 (580,58); 3,3286 (7,62); 3,2446 (1,32); 3,1036 (0,51); 3,0656 (0,51); 2,9399 (0,7); 2,6805 (2,05); 2,6182 (1,09); 2,6153 (1,49); 2,6123 (1,1); 2,543 (0,62); 2,5245 (2,25); 2,5215 (3,05); 2,5183 (3,8); 2,5094 (79,16); 2,5065 (164,86); 2,5035 (221,76); 2,5005 (162,52); 2,4977 (78,12); 2,3906 (1,05); 2,3877 (1,43); 2,3847 (1,05); 2,0789 (0,5); 1,9907 (3,97); 1,9066 (0,38); 1,6608 (1,13); 1,4749 (0,38); 1,4506 (0,98); 1,4387 (1,81); 1,4266 (1,1); 1,3791 (0,88); 1,3467 (2,94); 1,2977 (0,44); 1,2577 (0,56); 1,2337 (1,42); 1,186 (1,28); 1,1742 (2,31); 1,1623 (1,19); 0,0052 (2,55); -0,0002 (61,56); -0,0057 (2,57) |
| Ex.. 49, Solvent: [DMSO], Spektrometer: 399,95MHz |
| 11,3656 (3,86); 9,5142 (4,4); 7,7755 (3,59); 7,7692 (3,97); 7,6847 (1,75); 7,6783 (1,49); 7,6628 (2,52); 7,6564 (2,33); 7,5817 (4,49); 7,5599 (3,05); 4,0562 (1,15); 4,0384 (3,4); 4,0206 (3,6); 4,0034 (16); 3,3453 (94,9); 3,3448 (94,73); 3,3197 (17,97); 2,5245 (0,51); 2,511 (7,72); 2,5068 (14,99); 2,5023 (19,26); 2,4978 (13,84); 2,4937 (6,76); 2,0729 (0,59); 1,9887 (13,71); 1,6091 (1,56); 1,5949 (3,86); 1,588 (4,03); 1,5747 (1,73); 1,277 (1,86); 1,2635 (3,82); 1,2568 (4,07); 1,2422 (1,5); 1,1928 (3,82); 1,1749 (7,49); 1,1572 (3,7) |
| Ex.. 50, Solvent: [DMSO], Spektrometer: 601,6MHz |
| 9,5618 (2,56); 9,4177 (6,18); 7,9529 (0,98); 7,6923 (2,28); 7,6781 (2,69); 7,635 (2,45); 7,6308 (2,6); 7,5617 (5,97); 7,5475 (6,73); 7,5004 (1,4); 7,4963 (1,31); 7,4863 (1,23); 7,482 (1,22); 7,4233 (4,05); 7,4191 (4,25); 7,2874 (2,63); 7,283 (2,53); 7,2731 (2,42); 7,2688 (2,31); 4,3107 (0,81); 4,3036 (0,87); 4,2993 (1,52); 4,2921 (1,53); 4,2806 (0,81); 4,1677 (0,49); 4,1596 (1,05); 4,1559 (1,81); 4,1474 (2,05); 4,144 (2,45); 4,1362 (2,11); 4,1329 (2,74); 4,1249 (2,63); 4,1211 (2,27); 4,1128 (1,37); 4,0421 (1,16); 4,0323 (1,53); 4,0297 (1,5); 4,0196 (1,98); 4,0097 (1,08); 4,0068 (1,07); 3,9968 (0,83); 3,7605 (0,48); 3,7486 (1,68); 3,7369 (2,16); 3,7256 (2,07); 3,7139 (1,61); 3,7018 (0,54); 3,6876 (0,53); 3,6756 (0,76); 3,6634 (0,93); 3,6514 (0,75); 3,5736 (0,78); 3,5616 (0,93); 3,5493 (0,81); 3,5373 (0,58); 3,4009 (0,71); 3,3887 (0,8); 3,3806 (1,37); 3,3578 (1317,4); 3,334 (5,83); 2,8908 (7,36); 2,7308 (5,98); 2,6185 (1,57); 2,6155 (2,16); 2,6126 (1,59); 2,5433 (0,92); 2,5248 (3,01); 2,5217 (4,1); 2,5186 (4,76); 2,5095 (118,16); 2,5067 (245,74); 2,5038 (330,23); 2,5009 (240,75); 2,4981 (114,58); 2,3909 (1,49); 2,3879 (2,06); 2,385 (1,48); 2,0787 (0,93); 1,8893 (1); 1,8773 (2,04); 1,8652 (2,07); 1,8532 (1,07); 1,8217 (0,59); 1,8094 (1,15); 1,7977 (1,5); 1,7863 (1,74); 1,7743 (1,53); 1,7621 (0,74); 1,6799 (0,65); 1,6676 (1,17); 1,6574 (1,68); 1,645 (1,65); 1,6348 (1,13); 1,6225 (0,67); 1,6179 (1,23); 1,6084 (4,94); 1,6039 (3,6); 1,5991 (6,24); 1,5947 (7,45); 1,5859 (2,64); 1,2954 (1,19); 1,2863 (2,43); 1,2819 (2,67); 1,2724 (1); 1,2578 (0,42); 1,2335 (1,57); 1,2037 (2,86); 1,1947 (5,93); 1,1903 (6,34); 1,181 (2,45); 1,1407 (7,11); 1,1288 (14,87); 1,1168 (6,89); 0,9415 (2,6); 0,9297 (5,46); 0,9179 (2,57); 0,8865 (3,17); 0,8743 (6,67); 0,862 (3,13); 0,8535 (0,38); 0,8172 (7,63); 0,8049 (16); 0,7926 (7,21); 0,0051 (1,34); -0,0002 (32,06); -0,0057 (1,19) |
| Ex.. 51, Solvent: [DMSO], Spektrometer: 601,6MHz |
| 11,2435 (1); 9,3497 (0,74); 7,9531 (0,39); 7,7736 (0,6); 7,7601 (0,7); 7,7304 (2,91); 7,7161 (3,31); 7,6522 (3,79); 7,6386 (3,74); 7,6244 (3,53); 7,5889 (2,65); 7,5753 (2,41); 7,5207 (1,21); 7,4121 (11,62); 7,4027 (3,64); 7,3862 (2,56); 7,3118 (2,15); 7,2988 (1,8); 7,2009 (0,39); 6,8169 (0,48); 4,3007 (3,14); 4,2901 (3,3); 4,2794 (2,58); 4,2566 (2,26); 4,2028 (1,63); 4,1284 (3,82); 4,1178 (3,61); 4,107 (3,02); 4,0674 (1,32); 4,055 (1,55); 4,0456 (2,7); 4,0338 (4,82); 4,022 (4,4); 4,0102 (1,78); 3,9587 (1,64); 3,947 (2,06); 3,9353 (1,89); 3,9165 (1,96); 3,9041 (2,3); 3,8933 (1,94); 3,8468 (1,72); 3,7093 (1,04); 3,6044 (0,96); 3,3585 (2804,89); 3,3349 (16,31); 3,2711 (0,92); 3,2466 (0,88); 3,1423 (0,68); 3,0944 (0,54); 3,061 (1,47); 3,0465 (1,27); 2,9693 (1,33); 2,8912 (2,99); 2,8563 (1,82); 2,8399 (3,68); 2,8293 (3,66); 2,7312 (2,47); 2,6188 (3,62); 2,616 (4,79); 2,6132 (3,59); 2,5436 (1,95); 2,525 (9,26); 2,522 (12,69); 2,5186 (16,24); 2,507 (555,84); 2,5043 (726,37); 2,5015 (536,53); 2,3912 (3,62); 2,3884 (4,75); 2,3856 (3,53); 2,2958 (0,37); 2,2837 (0,81); 2,2716 (0,33); 2,0789 (2,13); 1,991 (15,17); 1,8862 (1,72); 1,8744 (3,51); 1,8624 (4,2); 1,8509 (3,63); 1,8425 (3,66); 1,8307 (3,67); 1,8188 (3,58); 1,8066 (2,89); 1,794 (2,27); 1,7812 (2,03); 1,7642 (2,22); 1,7173 (14,52); 1,6892 (3,36); 1,6419 (1,13); 1,5709 (1,75); 1,5668 (1,84); 1,5023 (3,27); 1,4837 (3,07); 1,374 (3,95); 1,3622 (2,97); 1,3502 (2,21); 1,3334 (3,83); 1,3217 (5,49); 1,3103 (4,13); 1,2978 (3,26); 1,2679 (4,55); 1,2579 (5,13); 1,2343 (9,67); 1,1974 (3,99); 1,1862 (10,63); 1,1743 (14,27); 1,1624 (12,11); 1,1558 (13,88); 1,1412 (16); 1,1295 (8,23); 1,1049 (6,61); 1,0708 (6); 1,0599 (8,58); 0,9401 (3,69); 0,9284 (5,1); 0,9172 (2,68); 0,8835 (5,11); 0,8713 (10,54); 0,865 (6,44); 0,8588 (10,32); 0,8536 (8,8); 0,845 (12,86); 0,8317 (12,07); 0,78 (1,98); 0,7676 (0,91); 0,0966 (0,62); 0,005 (6,46); -0,0002 (127,14); -0,0055 (5,46); -0,1 (0,6) |
| Ex.. 52, Solvent: [DMSO], Spektrometer: 399,95MHz |
| 9,4063 (0,33); 7,7318 (0,72); 7,7103 (0,89); 7,6897 (0,37); 7,6725 (0,36); 7,6593 (1,1); 7,6531 (1,26); 7,6406 (1,06); 7,6247 (0,89); 7,5947 (0,61); 7,5698 (0,76); 7,5483 (0,56); 7,5048 (0,39); 7,4401 (0,74); 7,4106 (1,09); 7,3918 (1,37); 7,2958 (0,47); 7,2895 (0,56); 7,2747 (0,49); 7,2686 (0,49); 4,4001 (0,35); 4,3823 (1,01); 4,3643 (1,22); 4,2654 (0,77); 4,2473 (0,94); 4,2311 (0,91); 4,2129 (0,73); 4,1602 (0,44); 4,147 (0,55); 4,1419 (0,6); 4,1332 (0,55); 4,1246 (0,64); 4,1084 (0,36); 4,055 (1,34); 4,0461 (0,4); 4,0372 (3,72); 4,0282 (0,46); 4,0194 (3,81); 4,0016 (1,44); 3,9349 (0,82); 3,9199 (0,86); 3,6846 (0,33); 3,6661 (0,36); 3,6171 (0,37); 3,5995 (0,37); 3,5078 (0,35); 3,3637 (1,04); 3,3424 (804,32); 3,3186 (9,85); 3,2893 (0,5); 3,2722 (0,6); 3,2519 (0,38); 3,2175 (0,32); 3,0961 (0,41); 3,0623 (0,35); 2,9842 (0,35); 2,8907 (1,32); 2,83 (0,8); 2,8117 (0,8); 2,7313 (0,96); 2,6766 (1,05); 2,6721 (1,47); 2,6675 (1,09); 2,5424 (0,67); 2,5255 (2,36); 2,5208 (3,63); 2,512 (75,12); 2,5075 (162,37); 2,503 (218,4); 2,4984 (156,12); 2,494 (73,27); 2,3388 (0,55); 2,3342 (1,08); 2,3297 (1,5); 2,3252 (1,13); 1,99 (16); 1,7116 (3,31); 1,6105 (0,4); 1,5971 (0,55); 1,5894 (0,58); 1,576 (0,43); 1,4981 (0,96); 1,458 (1,92); 1,44 (3,22); 1,4221 (1,86); 1,4094 (0,97); 1,3406 (3,68); 1,3235 (3,33); 1,3135 (3,04); 1,2956 (3,01); 1,2777 (2,74); 1,2582 (2,19); 1,2343 (5,2); 1,2036 (1,29); 1,1922 (5,62); 1,1863 (2,32); 1,1744 (10,68); 1,1676 (3,46); 1,1566 (7,96); 1,1448 (5,05); 1,1332 (3,63); 1,1153 (1,98); 1,0491 (1,91); 0,9496 (1,15); 0,9317 (1,85); 0,914 (0,87); 0,8933 (0,63); 0,8748 (1,42); 0,8624 (1,03); 0,8539 (1,15); 0,8363 (0,42); 0,1459 (0,45); 0,008 (3,24); -0,0002 (113,96); -0,0085 (3,81); -0,1496 (0,43) |
| Ex.. 53, Solvent: [DMSO], Spektrometer: 399,95MHz |
| 11,4811 (0,46); 9,5802 (3,86); 9,504 (0,61); 7,8067 (0,33); 7,7817 (3,36); 7,7747 (1,02); 7,7681 (1,19); 7,7604 (4,49); 7,7135 (0,41); 7,6917 (0,47); 7,6851 (0,47); 7,5943 (0,9); 7,5726 (1,01); 7,3651 (0,37); 4,106 (0,36); 4,0549 (0,76); 4,037 (2,46); 4,0205 (16); 4,0015 (0,98); 3,4808 (0,47); 3,4264 (0,76); 3,4141 (1,01); 3,366 (481,46); 3,3609 (380,93); 3,3594 (381,76); 3,3541 (595,63); 3,3505 (698,07); 3,0316 (0,61); 2,6772 (1,33); 2,6727 (1,85); 2,6681 (1,36); 2,5429 (0,97); 2,5261 (3,27); 2,5213 (4,97); 2,5126 (89,86); 2,5081 (194,5); 2,5036 (263,53); 2,4991 (189,65); 2,4946 (89,56); 2,4051 (1,69); 2,335 (1,23); 2,3303 (1,72); 2,3258 (1,27); 2,0763 (1,33); 2,0394 (9,05); 1,9899 (10,76); 1,9092 (0,76); 1,6335 (1,56); 1,6194 (3,67); 1,6124 (4,01); 1,5991 (1,94); 1,5757 (0,35); 1,3543 (0,43); 1,2973 (1,85); 1,2837 (3,68); 1,277 (4,01); 1,2623 (1,85); 1,2521 (0,95); 1,2369 (1,14); 1,1922 (2,96); 1,1744 (5,8); 1,1566 (2,83); 0,008 (0,76); -0,0002 (21,52); -0,0085 (0,61) |
| Ex.. 54, Solvent: [DMSO], Spektrometer: 399,95MHz |
| 9,4882 (0,79); 9,3813 (4,43); 8,5176 (2,05); 8,5137 (2,27); 8,5056 (2,27); 8,5017 (2,28); 8,4323 (3,23); 8,4273 (3,41); 8,2561 (0,5); 8,2509 (0,53); 7,952 (0,59); 7,7143 (0,68); 7,7082 (0,72); 7,6847 (1,07); 7,6801 (1,7); 7,6753 (1,15); 7,6651 (1,28); 7,6603 (1,95); 7,6556 (1,27); 7,5766 (0,52); 7,555 (0,77); 7,5036 (2,71); 7,4973 (2,86); 7,4812 (0,43); 7,4689 (0,59); 7,4626 (0,76); 7,4508 (4,33); 7,4415 (0,51); 7,4293 (4,72); 7,4088 (1,88); 7,3969 (1,79); 7,3893 (1,66); 7,3773 (1,57); 6,9932 (1,56); 6,9868 (1,58); 6,9719 (1,46); 6,9654 (1,45); 5,4683 (2,14); 5,4307 (2,45); 5,0433 (0,53); 4,9943 (0,63); 4,9816 (2,41); 4,9544 (0,36); 4,9441 (2,09); 4,0808 (2,62); 4,0555 (0,62); 4,0377 (1,82); 4,02 (1,85); 4,0022 (0,63); 3,9068 (16); 3,3269 (326,92); 3,322 (337,09); 2,8902 (5,17); 2,7304 (4,11); 2,6791 (0,48); 2,675 (1,04); 2,6704 (1,48); 2,6658 (1,11); 2,6613 (0,56); 2,5408 (0,86); 2,5237 (2,77); 2,5102 (73,18); 2,5058 (152,67); 2,5013 (207,94); 2,4967 (155,09); 2,4923 (79,01); 2,337 (0,46); 2,3326 (1,01); 2,3281 (1,42); 2,3235 (1,05); 2,3191 (0,53); 2,0737 (0,39); 1,9884 (7,83); 1,6127 (2,1); 1,5986 (4,95); 1,5917 (5,39); 1,5785 (2,38); 1,2787 (0,41); 1,2653 (0,83); 1,2583 (1,18); 1,2433 (0,76); 1,2353 (1,12); 1,2176 (1,97); 1,204 (4,14); 1,1973 (4,51); 1,1924 (3,36); 1,183 (1,78); 1,1745 (4,42); 1,1567 (2,17); 0,008 (1,38); - 0,0002 (45,87); -0,0084 (2,11) |
| Ex.. 55, Solvent: [DMSO], Spektrometer: 399,95MHz |
| 9,4969 (1,3); 9,3847 (4,47); 7,9522 (0,67); 7,6698 (0,96); 7,664 (1,17); 7,6364 (0,52); 7,615 (1,61); 7,6008 (1,05); 7,5951 (0,94); 7,5792 (0,35); 7,5731 (0,36); 7,507 (3,9); 7,4855 (4,65); 7,4513 (3,32); 7,4447 (3,77); 7,2367 (2,05); 7,2301 (2,11); 7,2153 (1,83); 7,2086 (1,89); 4,9577 (1,05); 4,9518 (1,43); 4,9482 (1,23); 4,8765 (3,22); 4,7971 (5,72); 4,7583 (2,3); 4,7061 (0,96); 4,3904 (2,06); 4,3516 (1,9); 4,3323 (0,37); 4,2925 (0,75); 4,2475 (0,75); 4,2081 (0,32); 4,0563 (5,38); 4,0379 (2,52); 4,0201 (2,62); 4,0023 (0,85); 3,9551 (0,69); 3,8648 (16); 3,6011 (0,36); 3,5766 (15,78); 3,3572 (0,33); 3,3255 (554,45); 2,8905 (5,77); 2,731 (4,53); 2,6751 (0,77); 2,6707 (1,1); 2,666 (0,83); 2,5407 (0,36); 2,524 (1,72); 2,5105 (54,47); 2,5061 (115,62); 2,5015 (158,95); 2,497 (121,97); 2,4927 (65,07); 2,3327 (0,86); 2,3283 (1,14); 2,3238 (0,89); 2,1326 (0,37); 2,1203 (5,45); 1,9886 (10,49); 1,7799 (6,3); 1,7121 (13,9); 1,6499 (0,4); 1,6077 (2,28); 1,5936 (5,52); 1,5872 (5,41); 1,5736 (3,14); 1,5679 (4,32); 1,2928 (0,62); 1,2791 (1,24); 1,2727 (1,44); 1,2583 (0,93); 1,2352 (1,63); 1,2184 (1,97); 1,205 (4,02); 1,1984 (4,28); 1,1924 (4); 1,1846 (1,85); 1,1745 (5,9); 1,1568 (3,09); 1,1023 (0,52); 1,0152 (11,7); 0,9974 (0,34); -0,0002 (0,61) |
| Ex.. 56, Solvent: [DMSO], Spektrometer: 601,6MHz |
| 9,4759 (0,64); 9,3699 (3,96); 8,5556 (5,83); 8,5529 (3,5); 8,5482 (3,58); 8,5456 (6,22); 8,4651 (0,81); 8,4626 (0,53); 8,4577 (0,53); 8,4551 (0,83); 7,9522 (0,41); 7,7446 (0,57); 7,5812 (1,4); 7,5194 (2,55); 7,5151 (2,6); 7,4619 (4,25); 7,4476 (4,7); 7,2711 (4,79); 7,2612 (4,76); 7,1362 (0,73); 7,1264 (0,73); 7,1037 (1,56); 7,0993 (1,54); 7,0894 (1,48); 7,0849 (1,45); 5,4328 (2,05); 5,4068 (2,29); 5,0763 (0,44); 5,0253 (2,42); 4,9994 (2,01); 4,0725 (2,43); 4,0467 (1,03); 4,0348 (2,93); 4,023 (2,97); 4,0112 (0,99); 3,9285 (16); 3,3673 (0,36); 3,3234 (775,68); 3,2995 (6,23); 2,8904 (3,65); 2,7311 (2,88); 2,6191 (0,72); 2,6161 (1,53); 2,6131 (2,16); 2,61 (1,53); 2,607 (0,72); 2,5406 (0,65); 2,5377 (0,44); 2,5223 (5,37); 2,5193 (6,39); 2,5162 (5,82); 2,5074 (108,34); 2,5044 (234,17); 2,5013 (320,07); 2,4983 (230,08); 2,4953 (105,4); 2,3916 (0,64); 2,3885 (1,45); 2,3855 (2,03); 2,3824 (1,42); 2,3794 (0,63); 2,0737 (0,79); 1,9885 (12,97); 1,604 (2,16); 1,5948 (4,81); 1,5903 (5,36); 1,5814 (2,18); 1,2674 (0,33); 1,2583 (0,74); 1,2538 (0,79); 1,2441 (0,52); 1,2351 (0,55); 1,2076 (1,71); 1,1984 (3,86); 1,1941 (4,14); 1,1863 (4,31); 1,1745 (7,06); 1,1626 (3,5); 0,0965 (0,4); 0,0052 (3,02); -0,0002 (99,19); -0,0058 (2,91); - 0,1001 (0,39) |
| Ex.. 57, Solvent: [DMSO], Spektrometer: 399,95MHz |
| 11,5339 (4,33); 9,4933 (1,31); 9,4791 (5,1); 7,7783 (0,46); 7,7659 (1,88); 7,7568 (1,13); 7,7496 (4,1); 7,7332 (1,88); 7,697 (8,58); 7,6917 (3,71); 7,6793 (3,75); 7,6736 (2,13); 7,6497 (0,47); 7,6332 (0,74); 7,6173 (0,48); 7,5851 (3,7); 7,567 (1,83); 7,5617 (2,51); 7,5506 (0,43); 7,5346 (0,34); 7,5192 (0,33); 5,9815 (0,48); 5,9665 (0,79); 5,95 (0,57); 5,9152 (0,52); 5,8999 (0,51); 5,8617 (1,01); 5,8285 (6,59); 5,8124 (6,52); 5,3477 (1,24); 5,3423 (1,27); 4,0562 (1,36); 4,0382 (3,76); 4,0202 (3,91); 4,0025 (1,35); 3,9786 (1,07); 3,6883 (0,81); 3,5728 (0,36); 3,5567 (0,37); 3,5234 (0,37); 3,5028 (0,42); 3,4858 (0,46); 3,4778 (0,41); 3,4639 (0,45); 3,4477 (0,59); 3,4164 (0,76); 3,3245 (1168,23); 3,2622 (0,43); 3,2123 (0,32); 3,1803 (0,58); 3,1683 (0,47); 3,0473 (1,21); 2,9933 (4,69); 2,9063 (0,59); 2,8597 (1,25); 2,8422 (4,92); 2,6749 (3,65); 2,6707 (4,76); 2,6666 (3,51); 2,5505 (1,32); 2,5403 (2,36); 2,5237 (16,88); 2,506 (550,85); 2,5017 (693,41); 2,4974 (500,71); 2,438 (0,61); 2,4143 (0,41); 2,393 (0,38); 2,3369 (10); 2,3286 (4,78); 2,3239 (3,48); 2,2986 (0,44); 2,2881 (0,35); 2,2187 (0,35); 2,1917 (0,47); 2,074 (0,97); 2,0503 (0,97); 1,9889 (16); 1,6079 (2,47); 1,5934 (6,31); 1,5869 (6,86); 1,5737 (3,53); 1,5483 (0,66); 1,5334 (0,56); 1,3133 (0,48); 1,2989 (0,51); 1,2759 (2,83); 1,2618 (6,03); 1,2551 (6,78); 1,2402 (4,56); 1,1923 (4,5); 1,1745 (8,44); 1,1571 (4,12); 0,8537 (0,55); 0,0077 (1,92); 0,0004 (50,97); -0,0002 (51,39); -0,0076 (2,47) |
| Ex.. 58, Solvent: [DMSO], Spektrometer: 399,95MHz |
| 11,4625 (0,76); 9,4924 (0,91); 7,7342 (0,69); 7,7279 (0,87); 7,6972 (0,38); 7,6754 (0,52); 7,669 (0,44); 7,5846 (0,89); 7,5628 (0,61); 5,2886 (0,42); 5,2653 (0,38); 5,2629 (0,4); 5,2177 (0,4); 5,2149 (0,4); 5,1751 (0,34); 5,1722 (0,34); 5,0002 (0,7); 4,9854 (0,68); 4,0553 (1,25); 4,0375 (3,8); 4,0197 (3,84); 4,0019 (1,29); 3,3327 (56,56); 2,5244 (0,53); 2,5107 (9,02); 2,5065 (18,16); 2,502 (23,94); 2,4976 (17,52); 2,4933 (8,69); 1,9892 (16); 1,6078 (0,32); 1,5935 (0,8); 1,5866 (0,87); 1,5734 (0,37); 1,2731 (0,38); 1,2595 (0,79); 1,2527 (0,87); 1,2381 (0,4); 1,1924 (4,32); 1,1746 (8,51); 1,1568 (4,22) |
| Ex.. 59, Solvent: [DMSO], Spektrometer: 399,95MHz |
| 11,651 (3,43); 9,5701 (3,87); 7,8258 (0,42); 7,8066 (0,45); 7,7856 (1,77); 7,7798 (1,75); 7,7597 (1,67); 7,7539 (1,75); 7,4937 (2,27); 7,4912 (2,53); 7,4879 (2,46); 4,055 (1,17); 4,0364 (16); 4,0195 (2,96); 4,0017 (0,96); 3,3272 (189,39); 3,3038 (1,7); 2,6753 (2,02); 2,6707 (2,77); 2,6662 (2,03); 2,6617 (1); 2,5409 (1,96); 2,538 (2); 2,5241 (10,07); 2,5194 (14,6); 2,5106 (143,21); 2,5062 (290,59); 2,5017 (381,24); 2,4971 (273,16); 2,4927 (130,54); 2,3372 (0,88); 2,3329 (1,89); 2,3284 (2,64); 2,3238 (1,92); 1,9891 (12,1); 1,6247 (1,42); 1,6106 (3,42); 1,6037 (3,59); 1,5904 (1,59); 1,3354 (0,45); 1,2977 (0,37); 1,2887 (1,71); 1,2751 (3,39); 1,2684 (3,63); 1,254 (1,5); 1,2493 (0,86); 1,2349 (1,15); 1,1921 (3,43); 1,1743 (6,77); 1,1565 (3,32); 0,146 (0,95); 0,008 (8,15); -0,0002 (242,95); -0,0085 (7,93); -0,1497 (0,99) |
| Ex.. 60, Solvent: [DMSO], Spektrometer: 399,95MHz |
| 7,794 (4,24); 7,7727 (4,24); 7,6351 (0,76); 4,0377 (0,78); 4,0274 (0,64); 4,0196 (1,38); 4,0064 (16); 3,3192 (128,67); 3,2968 (0,7); 2,8209 (1,39); 2,8055 (1,44); 2,6747 (0,95); 2,6701 (1,33); 2,6657 (0,98); 2,5404 (0,79); 2,5235 (3,49); 2,51 (67,6); 2,5057 (137,84); 2,5011 (183,23); 2,4966 (131,99); 2,4922 (63,54); 2,4323 (0,41); 2,414 (0,36); 2,3324 (1,13); 2,3278 (1,54); 2,3234 (1,25); 2,3189 (0,84); 2,2912 (1,59); 2,2752 (1,59); 1,9886 (2,92); 1,8835 (2,17); 1,6149 (0,87); 1,4668 (0,4); 1,449 (0,35); 1,3351 (0,44); 1,2492 (0,66); 1,2339 (0,36); 1,1922 (0,88); 1,1744 (1,71); 1,1566 (0,85); 1,1112 (0,34); 1,0892 (0,48); 1,084 (0,4); 1,0655 (0,87); 1,0372 (4,91); 1,0194 (10,33); 1,0015 (4,75); 0,9043 (4,3); 0,8866 (9,1); 0,8688 (4,15); 0,008 (1,09); -0,0002 (32,33); -0,0085 (1,18) |
| Ex.. 61, Solvent: [DMSO], Spektrometer: 399,95MHz |
| 11,5111 (0,37); 11,475 (1,82); 7,8171 (0,36); 7,761 (1,76); 7,7563 (1,78); 7,6607 (0,63); 7,6561 (0,62); 7,6393 (1,64); 7,6344 (1,68); 7,6196 (2,16); 7,5979 (0,65); 4,0388 (7,82); 4,0199 (1,67); 3,3272 (155,76); 3,3046 (0,44); 3,2089 (0,61); 3,0604 (0,48); 2,8649 (0,47); 2,6801 (0,39); 2,6754 (0,85); 2,6708 (1,18); 2,6662 (0,84); 2,6616 (0,37); 2,541 (0,64); 2,5242 (3,65); 2,5194 (6,12); 2,5108 (63,64); 2,5063 (126,44); 2,5017 (165,51); 2,4971 (117,67); 2,4926 (54,8); 2,3375 (0,45); 2,333 (0,89); 2,3285 (1,2); 2,3239 (0,84); 2,3193 (0,4); 1,9892 (1,79); 1,717 (1,85); 1,4686 (0,61); 1,3975 (16); 1,3356 (0,56); 1,3185 (0,45); 1,3025 (0,73); 1,2491 (0,7); 1,2357 (0,51); 1,2144 (1,84); 1,1967 (3,53); 1,179 (1,74); 1,1744 (1,8); 1,1565 (0,59); 0,008 (0,73); -0,0002 (19,27); -0,0085 (0,58) |
| Ex.. 62, Solvent: [DMSO], Spektrometer: 399,95MHz |
| 11,4809 (1,28); 7,7806 (1,15); 7,7762 (1,17); 7,6487 (0,33); 7,6426 (0,35); 7,6268 (1,18); 7,6215 (1,31); 7,6147 (1,72); 7,593 (0,33); 4,0556 (0,42); 4,0348 (5,9); 4,0202 (1,98); 4,0022 (0,4); 3,3283 (21,01); 3,1067 (0,99); 2,8649 (5,51); 2,5245 (0,58); 2,511 (9,79); 2,5067 (19,73); 2,5022 (26,27); 2,4977 (19,46); 2,4934 (9,59); 1,9895 (4,55); 1,6728 (1,36); 1,4933 (1,02); 1,3975 (16); 1,1926 (1,27); 1,1748 (2,51); 1,157 (1,23); -0,0002 (2,53) |
| Ex.. 63, Solvent: [DMSO], Spektrometer: 399,95MHz |
| 9,6002 (1,23); 7,7269 (1,04); 7,7055 (1,24); 7,6766 (0,93); 7,6704 (0,97); 7,4809 (0,56); 7,4747 (0,53); 7,4597 (0,48); 7,4533 (0,47); 4,8951 (0,42); 4,8795 (0,59); 4,864 (0,43); 4,0855 (4,08); 4,0568 (1,41); 4,039 (4,12); 4,0212 (4,16); 4,0034 (1,43); 3,3262 (2,69); 2,5117 (1,75); 2,5073 (3,52); 2,5028 (4,67); 2,4983 (3,47); 2,4939 (1,71); 1,9898 (16); 1,617 (0,42); 1,6028 (1,05); 1,596 (1,11); 1,5828 (0,47); 1,2947 (0,5); 1,281 (1,05); 1,2745 (1,1); 1,2599 (0,4); 1,1934 (5,01); 1,1756 (9,46); 1,1578 (4,87); 1,0379 (1,17); 1,0225 (1,27); 0,994 (1,27); 0,9787 (1,15); -0,0002 (1,31) |
| Ex.. 64, Solvent: [DMSO], Spektrometer: 399,95MHz |
| 11,531 (5,19); 9,4981 (6,09); 8,3176 (0,59); 7,7614 (4,9); 7,7551 (5,67); 7,69 (2,44); 7,6836 (2,05); 7,6681 (3,54); 7,6617 (3,29); 7,588 (6,35); 7,5661 (4,3); 5,5721 (1,17); 5,5509 (3,42); 5,5291 (3,53); 5,5072 (1,24); 4,0553 (0,61); 4,0375 (1,86); 4,0197 (1,88); 4,0019 (0,63); 3,3757 (6,9); 2,6756 (1,2); 2,671 (1,66); 2,6665 (1,24); 2,5379 (1,15); 2,5242 (6,33); 2,5193 (9,89); 2,5108 (89,72); 2,5065 (179,68); 2,5019 (237,24); 2,4974 (175,66); 2,4931 (88,49); 2,3331 (1,22); 2,3287 (1,67); 2,3242 (1,26); 1,989 (7,98); 1,609 (2,14); 1,5947 (5,27); 1,5878 (5,67); 1,5746 (2,44); 1,3977 (16); 1,3122 (0,32); 1,2725 (2,58); 1,2588 (5,23); 1,2521 (5,65); 1,2375 (2,18); 1,1926 (2,19); 1,1748 (4,28); 1,157 (2,11); 0,8854 (0,38); 0,0079 (1,5); -0,0002 (40,02); -0,0083 (1,91) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Ein Fachmann, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

### Herstellung der Ausgangsverbindungen

### Ethyl-3-(1-chlorcyclopropyl)-1-methyl-1H-pyrazol-5-carboxylat

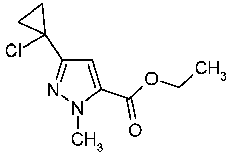

10,16 g (254,2 mmol) Natriumhydrid werden in 125 mL Tetrahydrofuran p.a. suspendiert und auf -15°C gekühlt. Zu dieser Suspension wird eine Lösung von 15,0 g (127,1 mmol) 1-(1-Chlorcyclopropyl)ethanon in 25 mL Tetrahydrofuran p.a. getropft. Die Suspension wird 2h bei -15°C gerührt und anschließend mit 37,12 g (254,2 mmol) Diethyloxalat versetzt. Nach 3h bei Raumtemperatur wird die Reaktion mit Eiswasser gequencht. Die wässrige Phase wird mehrmals mit Ethylacetat extrahiert. Die vereinigten organischen Phasen werden mit gesättigter Natriumchlorid Lösung gewaschen, über Natriumsulfat getrocknet und filtriert. Das Lösungsmittel wird am Rotationsverdampfer unter vermindertem Druck entfernt.

Der Rückstand wird in 150 mL Ethanol p.a. gelöst und unter Rückfluß gekocht. 36,09 g (254,2 mmol) Methylhydrazinsulfat werden zu der refluxierenden Mischung hinzugegeben und für weitere 4h unter Rückfluß gekocht. Nach dem Abkühlen wird die Reaktion unter vermindertem Druck am Rotationsverdampfer eingeengt und der so erhaltene Rückstand in einem Gemisch aus Wasser und Ethylacetat aufgenommen. Die wässrige Phase wird mehrfach mit Ethylacetat extrahiert. Die vereinigten organischen Phasen werden mit gesättigter Natriumchlorid Lösung gewaschen, über Natriumsulfat getrocknet und filtriert. Das Lösungsmittel wird am Rotationsverdampfer unter vermindertem Druck entfernt. Das Rohprodukt wird mittels Säulenchromatographie gereinigt. Man erhält 4,34 g (15%) Ethyl-3-(1-chlorcyclopropyl)-1-methyl-1H-pyrazol-5-carboxylat.
¹H-NMR (300 MHz, d₁-Chloroform) δ = 6,89 (s, 1H), 4,36 (q, 2H), 4,11 (s, 3H), 1,35 (t, 3H) ppm;

### Ethyl-4-chlor-3-(1-chlorcyclopropyl)-1-methyl-1H-pyrazol-5-carbolylat

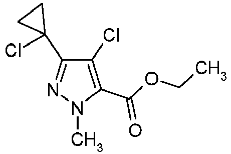

500 mg (2,19 mmol) Ethyl-3-(1-chlorcyclopropyl)-1-methyl-1*H*-pyrazol-5-carboxylat werden in 10 mL N,N-Dimethylformamid p.a. gelöst und mit 438 mg (3,28 mmol)) N-Chlorsuccinimid versetzt. Das Reaktionsgemisch wird 15 auf 80° C erhitzt. Die abgekühlte Reaktionslösung wird mit Wasser verdünnt und zweimal mit Ethylacetat extrahiert. Die vereinigten organischen Phasen werden mit gesättigter Natriumchlorid Lösung gewaschen, über Natriumsulfat getrocknet und filtriert. Das Lösungsmittel wird unter vermindertem Druck am Rotationsverdampfer entfernt. Das Rohprodukt wird über Kieselgel filtriert und mit Ethylacetat eluiert. Man erhält 517 mg (80%) Ethyl-4-chlor-3-(1-chlorcyclopropyl)-1-methyl-1*H*-pyrazol-5-carboxylat in 89%-iger Reinheit.
¹H-NMR (400 MHz, d₆-DMSO): δ = 4,35 (q, 2H), 4,04 (s, 3H), 1,42-1,46 (m, 2H), 1,31-1.38 (m, 5H) ppm.
HPLC-MS ^{a)}: logP = 3,52, Masse (m/z) = 263 [M+H]⁺.

### 4-Chlor-3-(1-chlorcyclopropyl)-1-methyl-1H-pyrazol-5-carbonsäure

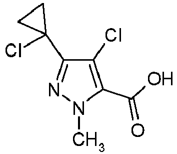

517 mg (1,76 mmol) Ethyl-4-chlor-3-(1-chlorcyclopropyl)-1-methyl-1*H*-pyrazol-5-carboxylat (Reinheit 89%) werden in 10 mL Ethanol p.a. gelöst. Die Lösung wird anschließend mit 3,5 mL (3,5 mmol) 1 N wässriger Natriumhydroxid Lösung versetzt und 16h bei Raumtemperatur gerührt. Das Reaktionsgemisch wird durch den Zusatz von 1 N Salzsäure angesäuert. Die wässrige Phase wird zweimal mit Ethylacetat extrahiert. Die vereinigten organischen Phasen werden mit gesättigter Natriumchlorid Lösung gewaschen, über Natriumsulfat getrocknet und filtriert. Das Lösungsmittel wird unter vermindertem Druck am Rotationsverdampfer entfernt. Man erhält 422 mg (99%) 4-Chlor-3-(1-chlorcyclopropyl)-1-methyl-1*H*-pyrazol-5-carbonsäure.
¹H-NMR (400 MHz, d₆-DMSO): δ = 4,02 (s, 3H), 1,31-1,42 (m, 4H) ppm.
HPLC-MS ^{a)}: logP = 1,90, Masse (m/z) = 235 [M+H]⁺.

### Ethyl-3-(1-fluorcyclopropyl)-1-methyl-1H-pyrazol-5-carboxylat

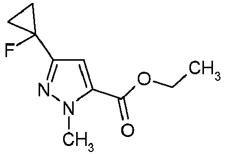

Die Herstellung von Ethyl-3-(1-fluorcyclopropyl)-1-methyl-1H-pyrazol-5-carboxylat erfolgt ausgehend von 1-(1-Fluorcyclopropyl)ethanon in Analogie zu dem in der Synthese von Ethyl-3-(1-chlorcyclopropyl)-1-methyl-1*H*-pyrazol-5-carboxylat beschriebenen Verfahren.
¹H-NMR (300 MHz, d₁-Chloroform) δ = 6,90 (s, 1H), 4,34 (q, 2H), 4,13 (s, 3H), 1,37 (t, 3H) ppm;

### Ethyl-4-chlor-3-(1-fluorcyclopropyl)-1-methyl-1H-pyrazol-5-carboxylat

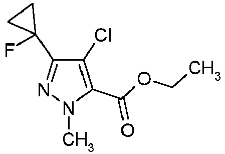

Die Herstellung erfolgte in Analogie zur Herstellung von Ethyl-4-chlor-3-(1-chlorcyclopropyl)-1-methyl-1*H*-pyrazol-5-carboxylat unter Verwendung von Ethyl-3-(1-fluorcyclopropyl)-1-methyl-1H-pyrazol-5-carboxylat und 3 eq *N*-Chlorsuccinimid.
¹H-NMR (400 MHz, d₆-DMSO): δ = 4,36 (q, 2H), 4,06 (s, 3H), 1,38-1,44 (m, 2H), 1,33 (t, 3H), 1,04-1,09 (m , 2H) ppm.
HPLC-MS ^{a)}: logP = 3,07, Masse (m/z) = 247 [M+H]⁺.

### 4-Chlor-3-(1-fluorcyclopropyl)-1-methyl-1H-pyrazol-5-carbonsäure

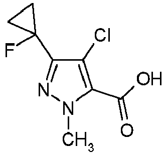

Die Herstellung erfolgte in Analogie zur Herstellung von 4-Chlor-3-(1-chlorcyclopropyl)-1-methyl-1*H-*pyrazol-5-carbonsäure unter Verwendung von Ethyl-3-(1-fluorcyclopropyl)-1-methyl-1H-pyrazol-5-carboxylat und 5,0 eq Natriumhydroxid in Methanol.
¹H-NMR (400 MHz, d₆-DMSO): δ = 4,05 (s, 3H), 1,37-1,43 (m, 2H), 1,05-1,09 (m, 2H) ppm.
HPLC-MS ^{a)}: logP = 3,07, Masse (m/z) = 219 [M+H]⁺.

### Methyl-2-chlor-5-({[1-methyl-3-(pentafluorethyl)-4-(trifluormethyl)-1H-pyrazol-5-yl]carbonyl}amino)benzoat

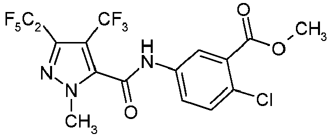

4,0 g (12,8 mmol) 1-Methyl-3-(pentafluorethyl)-4-(trifluormethyl)-1*H*-pyrazol-5-carbonsäure werden in 50 mL Dichlormethan suspendiert. Anschließend werden nacheinander 0,02 mL *N,N*-Dimethylformamid und 3,54 mL (38,4 mmol) Oxalylchlorid dazugegeben. Das Reaktionsgemisch wird erst 30 Minuten bei Raumtemperatur und dann 30 Minuten unter Rückfluß gerührt. Das Lösungsmittel wird unter vermindertem Druck am Rotationsverdampfer entfernt. Das enstandene 1-Methyl-3-(pentafluorethyl)-4-(trifluormethyl)-1H-pyrazol-5-carbonylchlorid wird ohne weitere Aufarbeitung für den nachfolgenden Syntheseschritt eingesetzt.

Eine Suspension von 2,38 g (12,8 mmol) Methyl-5-amino-2-chlorbenzoat und 2,57 g (19,2 mmol) Silber(I)cyanid in 50 mL Dichlormethan p.a. wird mit einer Lösung von 4,24 g (12,8 mmol) 1-Methyl-3-(pentafluorethyl)-4-(trifluormethyl)-1*H*-pyrazol-5-carbonylchlorid in 25 mL Dichlormethan p.a. versetzt und 16 h bei Raumtemperatur gerührt. Die Suspension wird anschließend über Silicagel filtriert und das Produkt mit einem Gemisch aus Cyclohexan und Ethylacetat (1:1) eluiert. Die organische Phase wird nacheinander dreimal mit 6N Salzsäure und zweimal mit gesättigter Natriumchloridlösung gewaschen. Anschließend wird die organische Phase über Natriumsulfat getrocknet, filtriert und am Rotationsverdampfer unter vermindertem Druck eingeengt. Man erhält 5,75 g (93%) Methyl-2-chlor-5-({[1-methyl-3-(pentafluorethyl)-4-(trifluormethyl)-1*H*-pyrazol-5-yl]carbonyl}amino)benzoat
¹H-NMR (400 MHz, d₃-Acetonitril): δ = 9,33 (s, 1H), 8,14 (d, 1H), 7,72 (dd, 1H), 7,54 (d, 1H), 3,98 (s, 3H), 3,90 (s, 3H) ppm.
HPLC-MS ^{a)}: logP = 4,05 Masse (m/z) = 480 [M+H]⁺.

### 2-Chlor-5-({[1-methyl-3-(pentafluorethyl)-4-(trifluormethyl)-1H-pyrazol-5-yl]carbonyl}amino) benzencarbonsäure

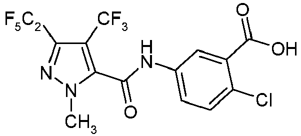

5,75 g (11,9 mmol) Methyl-2-chlor-5-({[1-methyl-3-(pentafluorethyl)-4-(trifluormethyl)-1*H*-pyrazol-5-yl]carbonyl}amino)benzoat werden in 30 mL Methanol p.A. gelöst und dann mit 15,0 mL (30,0 mmol) 2N Natronlauge versetzt. Das Reaktionsgemisch wird 16 Stunden bei Raumtemperatur gerührt. Die Reaktionslösung wird vorsichtig mit 6 N Salzsäure angesäuert und die wässrige Phase anschließend dreimal mit Ethylacetat extrahiert. Die vereinigten organischen Phasen werden einmal mit gesättigter Natriumchloridlösung gewaschen, über Natriumsulfat getrocknet und filtriert. Das Lösungsmittel wird unter vermindertem Druck am Rotationsverdampfer entfernt. Man erhält 5,57 g 2-Chlor-5-({[1-methyl-3-(pentafluorethyl)-4-(trifluormethyl)-1*H*-pyrazol-5-yl]carbonyl}amino) benzencarbonsäure als farblosen Feststoff.
¹H-NMR (400 MHz, d₃-Acetonitril): δ = 9.17 (s, 1H), 8.11 (d, 1H), 7.73 (dd, 1H), 7.52 (d, 1H), 3.98 (s, 3H) ppm.
HPLC-MS ^{a)}: logP = 3,18, Masse (m/z) = 466 [M+H]⁺.

### Ethyl-3,4-bis(trifluormethyl)-1H-pyrazol-5-carboxylat

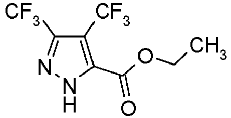

7,57 g (63,0 mmol) Diazoethylacetat werden unter Schutzgas in 200 mL Diethylether vorgelegt und auf -70°C temperiert. Es werden anschließend 20,4 g (126 mmol) Hexafluorbutin in die gekühlte Lösung eingeleitet. Das Reaktionsgemisch wird langsam auf Raumtemperatur erwärmt und 16 Stunden gerührt. Anschließend wird das Lösungsmittel am Rotationsverdampfer entfernt. Man erhält 17,0 g Ethyl-3,4-bis(trifluormethyl)-1*H*-pyrazol-5-carboxylat (98%) als gelbes Öl.
¹H-NMR (400 MHz, d₃-Acetonitril): δ = 4,42 (q, 2H), 1,38 (t, 3H) ppm.
GC-MS: Retentionszeit 3,48 min; Masse (m/z) = 276 [M]⁺.

### 1-Methyl-3,4-bis(trifluormethyl)-1H-pyrazol-5-carbonsäure

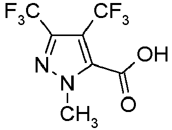

3,0 g (10,9 mmol) Ethyl-3,4-bis(trifluormethyl)-1*H*-pyrazol-5-carboxylat und 4,5 g (32,6 mmol) Kaliumcarbonat werden in 70 mL Aceton suspendiert und mit 1,35 mL Iodmethan (21,7 mmol) versetzt. Das Reaktionsgemisch wird über Nacht bei Raumtemperatur gerührt. Zu der Suspension werden 54 mL (108 mmol) 2 N Natronlauge gegeben. Die Lösung wird anschließend über Nacht bei Raumtemperatur gerührt. Das Reaktionsgemisch wird mit Wasser verdünnt und das Aceton weitesgehend am Rotationsverdampfer unter vermindertem Druck entfernt. Der Rückstand wird mit 1 M Salzsäure auf pH 2-3 eingestellt. Die wässrige Reaktionslösung wird zweimal mit Ethylacetat extrahiert. Die vereinigten organischen Phasen werden über Magnesiumsulfat getrocknet, filtriert und unter vermindertem Druck am Rotationsverdampfer eingeengt. Man erhält 2,7 g 1-Methyl-3,4-bis(trifluormethyl)-1*H*-pyrazol-5-carbonsäure (84%; Reinheit 88%) als braunen Feststoff.
¹H-NMR (400 MHz, d₃-Acetonitril): δ = 4,12 (s, 3H) ppm.
HPLC-MS ^{a)}: logP = 1,47; Masse (m/z) = 263 [M+H]⁺.

### Methyl-2-chlor-5-(methylamino)benzoat

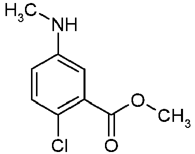

55,0 g (296 mmol) Methyl-2-chlor-5-amino-benzoat und 49,1 g (356 mmol) Kaliumcarbonat werden in 500 mL Acetonitril p.a. suspendiert. Es werden tropfenweise 22,1 mL (356 mmol) Methyliodid zu dem Reaktionsgemisch zu gegeben. Anschließend wird die Suspension 3 Stunden unter Rückfluß gekocht. Nach dem Abkühlen wird das Reaktionsgemisch filtriert. Das Filtrat wird mit Wasser verdünnt. Die wässrige Phase wird zweimal mit Ethylacetat extrahiert. Die vereinigten organischen Phasen werden über Natriumsulfat getrocknet und filtriert. Das Lösungsmittel wird unter vermindertem Druck am Rotationsverdampfer entfernt. Das Rohprdukt wird säulenchromatographisch aufgereinigt. Man erhält 30,0 g (51%) Methyl-2-chlor-5-(methylamino)benzoat.
¹H-NMR (300 MHz, d₁-Chloroform) δ = 7,21 (d, 1H), 7,00 (d, 1H), 6,63 (d, 1H), 3,90 (s, 3H), 2,86 (s, 3H) ppm.

### 5-Amino-2-chlor-N-(1-cyancyclopropyl)benzamid

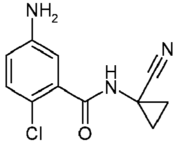

3,20 g (15,9 mmol) 2-Chlor-5-nitrobenzoesäure werden in 50 mL Dichlormethan p.a. vorgelegt und mit 0.06 mL N,N-Dimethylformamid p.a. versetzt. Das Reaktionsgemisch wird anschließend mit 2,08 mL (23,8 mmol) Oxalsäuredichlorid versetzt. Nach 3h bei RT wird das Reaktionsgemisch unter vermindertem Druck am Rotationsverdampfer eingeengt. Das Rohprodukt (2-Chlor-5-nitrobenzoesäurechlorid) wird ohne weitere Aufreinigung weiter umgesetzt.

2.36 g (19.8 mmol) 1-Aminocyclopropancarbonitrilhydrochlorid wird in 70 mL Chloroform p.a. suspendiert. Die Suspension wird unter Eiskühlung mit 6.93 mL (39,7 mmol) N-Ethyl-diisopropylamin versetzt. Zu der gekühlten Mischung wird anschließend eine Lösung von 3,50 g (15,9 mmol) 2-Chlor-5-nitrobenzoesäurechlorid in 5 mL Chloroform p.a. getropft. Das Reaktionsgemisch wird 4 h auf 50°C (Ölbadtemperatur) erwärmt. Anschließend wird das Reaktionsgemisch 12 h bei Raumtemperatur nachgerührt.

Das Reaktionsgemisch wird unter vermindertem Druck am Rotatiosverdampfer eingeengt und der Rückstand in Ethylacetat aufgenommen. Die organische Phase wird zweimal mit 0,5 N Salzsäure gewaschen, über Natriumsulfat getrocknet und filtriert. Das Lösungsmittel wird unter vermindertem Druck am Rotationsderdampfer entfernt. Man erhält 3.70 g (84%) 2-Chlor-*N*-(1-cyancyclopropyl)-5-nitrobenzamid.
¹H-NMR (400 MHz, d₆-DMSO): δ = 9,59 (s, 1H), 8,36 (d, 1H), 8,31 (dd, 1H), 7,85 (d, 1H), 1,55-1,61 (m, 2H), 1,32-1,37 (m, 2H) ppm.
HPLC-MS ^{a)}: logP = 1,52; Masse (m/z) = 266 [M+H]⁺.

3,15 g Eisenpulver wird in 18 mL 5%-iger Essigsäure suspendiert und mit einer Lösung von 3,0 g 2-Chlor-*N*-(1-cyancyclopropyl)-5-nitrobenzamid in einem Gemisch aus 25 mL Ethylacetat und 22,6 mL Eisessig versetzt. Die Innentemperatur wird dabei unter 45°C gehalten. Das Reaktionsgemisch wird 14 Stunden bei Raumtemperatur gerührt und anschließend über Celite filtriert. Das Filtrat wird mit Wasser verdünnt und die wässrige Phase dreimal mit Ethylacetat extrahiert. Die vereinigten organischen Phasen werden zweimal mit gesättigter Natriumchloridlösung gewaschen, über Magnesiumsulfat getrocknet, filtriert und am Rotationsverdampfer unter vermindertem Druck eingeengt. Das Rohprodukt wird einem Gemisch aus drei Teilen Cyclohexan und einem Teil Ethylacetat verrührt und der Feststoff abfiltriert. Man erhält 2,0 g (71%) 5-Amino-2-chlor-*N*-(1-cyancyclopropyl)benzamid.
¹H-NMR (400 MHz, d₆-DMSO): δ = 9,20 (s, 1H), 7,07 (d, 1H), 6,62 (dd, 1H), 6,57 (d, 1H), 1,51-1,57 (m, 2H), 1,17-1,24 (m, 2H) ppm.
HPLC-MS ^{a)}: logP = 0.82; Masse (m/z) = 236 [M+H]⁺.

### 1-Methyl-4-(methylsulfanyl)-3-(pentafluorethyl)-1H-pyrazol-5-carbonsäure

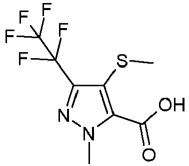

8,0 g (27,7 mmol) 1-Methyl-4-nitro-3-(pentafluorethyl)-1H-pyrazol-5-carbonsäure [Herstellung in Analogie zu J. Med. Chem. 1987, 30, 91-96] werden in 100 mL Dichlormethan gelöst. Die Lösung wird nacheinander mit 50 µL N,N-Dimehtylformamid und 10,5 g (83,0 mmol) Oxalylchlorid versetzt. Nach 0,5h bei Raumtemperatur, wird die Reaktion 0,5h unter Rückfluß erwärmt. Das Reaktiongemisch wird auf Raumtemperatur abgekühlt. Die Lösungsmittel und überschüssiges Oxalylchlorid werden am Rotationsverdampfer unter vermindertem Druck entfernt. Der Rückstand wird in Chloroform p.a. gelöst und langsam zu einer Suspension aus 5,56 g (41,5 mmol) Silber(I)cyanid, 100 mL Chloroform p.a. und 56 mL Methanol p.a. getropft. Die Mischung wird 8h unter Rückfluß erhitzt und anschließend auf Raumtemperatur abgekühlt. Das Reaktionsgemisch wird über eine kurze Kieselgelsäule filtriert und mit Dichlormethan nachgespült. Die Lösungsmittel werden am Rotationsverdampfer unter vermindertem Druck entfernt.

Man erhält 8,5 g Methyl-1-methyl-4-nitro-3-(pentafluorethyl)-1*H*-pyrazol-5-carboxylat. Das Rohprodukt wird ohne weitere Aufreinigung für die nächste Umsetzung verwendet.
¹H-NMR (600 MHz, d₆-DMSO): δ = 4,16 (s, 3H), 3,93 (s, 3H) ppm.
HPLC-MS ^{a)}: logP = 3,18; Masse (m/z) = 304 [M+H]⁺.

8,5 g (28,0 mmol) Methyl-1-methyl-4-nitro-3-(pentafluorethyl)-1*H*-pyrazol-5-carboxylat und 850 mg Palldium auf Kohle (10% Palladium) werden in 100 mL Methanol suspendiert. Nachdem Inertisieren des Autoklavs mit Stickstoff wird das Reaktionsgemisch unter 5 bar Wasserstoffatmosphäre gerührt. Nach 22h bei RT wird das Gemisch über Celite filtriert und das Lösungsmittel unter vermindertem Druck am Rotationsverdampfer entfernt. Das Rohprodukt wird in Dichlormethan aufgenommen und über Natriumsulfat filtriert. Das Dichlormethan wird anschließend unter vermindertem Druck am Rotationsverdampfer entfernt.

Man erhält 6,7 g (86%) Methyl-4-amino-1-methyl-3-(pentafluorethyl)-1*H*-pyrazol-5-carboxylat.
¹H-NMR (600 MHz, d₆-DMSO): δ = 5,32 (s, 2H), 4,07 (s, 3H), 3,86 (s, 3H) ppm.
HPLC-MS ^{a)}: logP = 2,52; Masse (m/z) = 274 [M+H]⁺.

2,0 g (7,32 mmol) Methyl-4-amino-1-methyl-3-(pentafluorethyl)-1*H*-pyrazol-5-carboxylat und 1,38 g (14,6 mmol) Dimethyldisulfid werden in 14 mL Acetonitril p.a. gelöst. Zu dieser Mischung wird langsam eine Lösung von 1,26 g (11,0 mmol) *tert*-Butylnitrit in 5 mL Acetonitril p.a. getropft. Nach Ende der Zugabe wird das Reaktionsgemisch noch 1h bei Raumtemperatur gerührt. Das Reaktionsgemisch wird anschließend auf 1 N Salzsäure gegossen. Die wässrige Phase wird dreimal mit Ethylacetat extrahiert. Die vereinigten organischen Phasen werden zweimal mit gesättigter Natriumchloridlösung gewaschen, über Magnesiumsulfat getrocknet und filtriert. Die Lösungsmittel werden am Rotationsverdampfer unter vermindertem Druck entfernt.

Man erhält 2,0 g (72%) Methyl-1-methyl-4-(methylsulfanyl)-3-(pentafluorethyl)-1H-pyrazol-5-carboxylat als 8:2 Gemisch aus dem gewünschten Produkt und dem Nebenprodukt Methyl-1-methyl-3-(pentafluorethyl)-1H-pyrazol-5-carboxylat.
¹H-NMR (400 MHz, d₆-DMSO): δ = 4,12 (s, 3H), 3,94 (s, 3H), 2,34 (s, 3H) ppm.
HPLC-MS ^{a)}: logP = 3,51; Masse (m/z) = 305 [M+H]⁺.

3,0 g Methyl-1-methyl-4-(methylsulfanyl)-3-(pentafluorethyl)-1H-pyrazol-5-carboxylat werden in 16 mL Methanol p.a. gelöst. Die Lösung wird anschließend mit 16,5 mL 2N wässriger Natronlauge versetzt und 16h bei Raumtemperatur gerührt. Das Reaktionsgemisch wird mit Ethylacetat verdünnt und dann mit 100 mL 1N Salzäure gewaschen. Die saure wässrige Phase wird zweimal mit zweimal mit Ethylacetat extrahiert. Die vereinigten organjschen Phasen werden mit gesättigter Natriumchloridlösung gewaschen, über Natriumsulfat getrocknet und filtriert. Die Lösungsmittel werden am Rotationsverdampfer unter vermindertem Druck entfernt.

Man erhält 2,5 g (90%) 1-Methyl-4-(methylsulfanyl)-3-(pentafluorethyl)-1H-pyrazol-5-carbonsäure als ein ca. 8:2 Gemisch aus dem gewünschten Produtk und dem Nebenprodukt 1-Methyl-3-(pentafluorethyl)-1H-pyrazol-5-carbonsäure.
¹H-NMR (400 MHz, d₆-DMSO): δ = 4,12 (s, 3H), 3,94 (s, 3H), 2,34 (s, 3H) ppm.
HPLC-MS ^{a)}: logP = 3,51; Masse (m/z) = 305 [M+H]⁺.

### Biologische Beispiele

### A. Wirksamkeit der Verbindungen

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen Zeit wird die Wirkung in % bestimmt. Dabei bedeutet 100 %, dass alle Käferlarven abgetötet wurden; 0 % bedeutet, dass keine Käferlarven abgetötet wurden.
Bei diesem Test zeigen z. B. die folgenden Verbindungen der Herstellungsbeispiele Wirkung von 100 % bei einer Aufwandmenge von 500g/ha : 1, 2, 3, 4, 5, 6, 7, 8, 9, 10

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigt z. B. die folgende Verbindung der Herstellungsbeispiele Wirkung von 83 % bei einer Aufwandmenge von 500g/ha : 7

Bei diesem Test zeigen z. B. die folgenden Verbindungen der Herstellungsbeispiele Wirkung von 100 % bei einer Aufwandmenge von 500g/ha : 2, 4, 5, 6, 8, 9, 10

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90 % bei einer Aufwandmenge von 500g/ha : 5, 9

Bei diesem Test zeigen z. B. die folgenden Verbindungen der Herstellungsbeispiele Wirkung von 100 % bei einer Aufwandmenge von 500g/ha : 2, 4, 6, 8, 10

### Tetranychus - test, OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindung der Herstellungsbeispiele Wirkung von 90 % bei einer Aufwandmenge von 500g/ha : 10

Bei diesem Test zeigen z. B. die folgenden Verbindungen der Herstellungsbeispiele Wirkung von 100 % bei einer Aufwandmenge von 500g/ha : 1, 2, 3, 4, 5, 6, 7, 8, 9.

### Ctenocephalides felis oral (CTECFE)

| | | |
|---|---|---|
| Lösungsmittel: | 1 | Gewichtsteil Dimethylsulfoxid |

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt.

Ca. 20 nüchterne adulte Flöhe *(Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Nach 2 Tagen wird die Abtötung in % bestimmt. Dabei bedeutet 100 %, dass alle Flöhe abgetötet wurden; 0 % bedeutet, dass kein Floh abgetötet wurde.

Bei diesem Test zeigt z. B. die folgende Verbindung der Herstellungsbeispiele Wirkung von 80 % bei einer Aufwandmenge von 100 ppm : 7

Bei diesem Test zeigen z. B. die folgenden Verbindungen der Herstellungsbeispiele Wirkung von 100 % bei einer Aufwandmenge von 100 ppm : 2, 6, 8, 9, 10

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100 % bei einer Aufwandmenge von 100 ppm: 6, 7, 8, 9, 10

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit *Musca domestica Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 100 % bei einer Aufwandmenge von 100 ppm : 6, 7, 8, 10

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100 % bei einer Aufwandmenge von 20µg / Tier: 2, 6, 7, 8, 9, 10

### Boophilus microplus - Test (DIP)

### Testtiere: adulte gesogene Weibchen von Boophilus microplus Stamm Parkhurst - (SP- resistent)

| | |
|---|---|
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 Zecken werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in

Kunststoffschalen überführt und in einem klimatisierten Raum aufbewahrt. Die Wirkungskontrolle erfolgt nach 7 Tagen auf Ablage fertiler Eier. Eier, deren Fertilität nicht äußerlich sichtbar ist, werden in Glasröhrchen bis zum Larvenschlupf im Klimaschrank aufbewahrt. Eine Wirkung von 100 % bedeutet, dass keine Zecke fertile Eier gelegt hat.

Bei diesem Test zeigen z.B. die folgenden Verbindungen der Herstellungsbeispiele eine Wirkung von 100 % bei einer Aufwandmenge von 100 ppm : 6, 8, 9, 10

### Amblyomma hebaraeum -Test (AMBYHE)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckenymphen (*Amblyomma hebraeum)* werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine Zecken abgetötet wurden.

Bei diesem Test zeigt z. B. die folgende Verbindung der Herstellungsbeispiele eine Wirkung von 100 % bei einer Aufwandmenge von 100 ppm : 10

### B. Biologische Vergleichsversuche

### Spodoptera frugiperda-Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in g/ha).

Maisblattscheiben (Z*ea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele überlegene Wirksamkeit gegenüber dem Stand der Technik: siehe Tabelle

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in g/ha).

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

### Phaedon-Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in g/**ha**).

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

### Tetranychus-Test ; OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in **g/ha**).

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele überlegene Wirksamkeit gegenüber dem Stand der Technik: siehe Tabelle

### Phaedon cochleariae - Sprühtest (PHAECO)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in ppm). Bei erforderlicher Zugabe von Ammoniumsalzen oder/und Penetrationsförderern werden diese jeweils in einer Konzentration von 1000 ppm der Präparatelösung zugefügt.

Kohlblätter *(Brassica oleracea)* werden mit einer Wirkstoffzubereitung der gewünschten Konzentration gespritzt und mit Larven des Meerrettichblattkäfers (*Phaedon cochleariae)* besetzt.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigt z. B. die folgende Verbindung der Herstellungsbeispiele überlegene Wirksamkeit gegenüber dem Stand der Technik: siehe Tabelle

### Plutella xylostella - Sprühtest (PLUTMA)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in ppm). Bei erforderlicher Zugabe von Ammoniumsalzen oder/und Penetrationsförderern werden diese jeweils in einer Konzentration von 1000 ppm der Präparatelösung zugefügt.

Kohlblätter *(Brassica oleracea)* werden mit einer Wirkstoffzubereitung der gewünschten Konzentration gespritzt und mit Raupen der Kohlschabe (*Plutella xylostella*) infiziert.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele überlegene Wirksamkeit gegenüber dem Stand der Technik: siehe Tabelle

### Spodoptera frugiperda - Sprühtest (SPODFR)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in **ppm**). Bei erforderlicher Zugabe von Ammoniumsalzen oder/und Penetrationsförderern werden diese jeweils in einer Konzentration von 1000 ppm der Präparatelösung zugefügt.

Baumwollblätter (*Gossypium hirsutum*) werden mit einer Wirkstoffzubereitung der gewünschten Konzentration gespritzt und mit Raupen des Heerwurms (*Spodoptera frugiperda*) besetzt.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele überlegene Wirksamkeit gegenüber dem Stand der Technik: siehe Tabelle

### Heliothis armigera - Sprühtest (HELIAR)

| | | |
|---|---|---|
| Lösungsmittel: | 7 | Gewichtsteile Dimethylformamid |
| Emulgator: | 2 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in ppm). Bei erforderlicher Zugabe von Ammoniumsalzen oder/und Penetrationsförderern werden diese jeweils in einer Konzentration von 1000 ppm der Präparatelösung zugefügt.

Baumwollpflanzen *(Gossypium hirsutum*) werden mit einer Wirkstoffzubereitung der gewünschten Konzentration gespritzt und nach Abtrocknung mit Raupen des Baumwollkapselwurms (*Heliothis armigera*) besetzt.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele überlegene Wirksamkeit gegenüber dem Stand der Technik: siehe Tabelle

### Tetranychus urticae - Sprühtest, OP-resistent (TETRUR)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration (in **ppm**). Bei erforderlicher Zugabe von Ammoniumsalzen oder/und Penetrationsförderern werden diese jeweils in einer Konzentration von 1000 ppm der Präparatelösung zugefügt.

Bohnenpflanzen *(Phaseolus vulgaris),* die stark von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindungen der Herstellungsbeispiele überlegene Wirksamkeit gegenüber dem Stand der Technik: siehe Tabelle

### Nilaparvata lugens - Sprühtest (NILALU)

| | | |
|---|---|---|
| Lösungsmittel: | 52,5 | GewichtsteileAceton |
| | 7 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Reispflanzen *(Oryza sativa)* werden mit einer Wirkstoffzubereitung der gewünschten Konzentration gespritzt und andchließend mit Larven der Reiszikade *(Nilaparvata lugens*) besetzt.

Nach der gewünschten Zeit wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z. B. die folgenden Verbindungen der Herstellungsbeispiele überlegene Wirksamkeit gegenüber dem Stand der Technik: siehe Tabelle

### Frankliniella occidentalis- Sprühtest (FRANOC)

| | | |
|---|---|---|
| Lösungsmittel: | 52,5 | GewichtsteileAceton |
| | 7 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Buschbohnenblattscheiben *(Phaseolus vulgaris)* werden mit einer Wirkstoffzubereitung der gewünschten Konzentration gespritzt und andchließend mit einer gemischten Thripspopulation *(Frankliniella occidentalis)* infiziert.

Nach der gewünschten Zeit wird die Wirkung in % bestimmt. Dabei bedeutet 100 %, dass alle Thripse abgetötet wurden; 0 % bedeutet, dass keine Thripse abgetötet wurden.

Bei diesem Test zeigen z. B. die folgenden Verbindungen der Herstellungsbeispiele überlegene Wirksamkeit gegenüber dem Stand der Technik: siehe Tabelle

### Liriomyza trifolii - Sprühtest (LIRITR)

| | | |
|---|---|---|
| Lösungsmittel: | 52,5 | Gewichtsteile Aceton |
| | 7 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Bohnenblattscheiben *(Phaseolus vulgaris),* die von Larven der Minierfliege *(Liriomyza trifolii)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Minierfliegen abgetötet wurden; 0 % bedeutet, dass keine Minierfliege abgetötet wurde.

Bei diesem Test zeigen z. B. die folgenden Verbindungen der Herstellungsbeispiele überlegene Wirksamkeit gegenüber dem Stand der Technik: siehe Tabelle

### Ctenocephalides felis oral (CTECFE)

| | | |
|---|---|---|
| Lösungsmittel: | 1 | Gewichtsteil Dimethylsulfoxid |

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt.

Ca. 20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigen z. B. die folgenden Verbindungen der Herstellungsbeispiele überlegene Wirksamkeit gegenüber dem Stand der Technik: siehe Tabelle

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit *Musca domestica*-Adulten besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z. B. die folgenden Verbindungen der Herstellungsbeispiele überlegene Wirksamkeit gegenüber dem Stand der Technik: siehe Tabelle

### Boophilus microplus - Test (DIP)

Testtiere: adulte gesogene Weibchen von *Boophilus microplus* Stamm Parkhurst - (SP- resistent)

| | |
|---|---|
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration (in **ppm**)

Diese Wirkstoffzubereitung wird in Röhrchen pipettiert. 8-10 Zecken werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach der gewünschten Zeit auf Ablage fertiler Eier. Eier, deren Fertilität nicht äußerlich sichtbar ist, werden in Glasröhrchen bis zum Larvenschlupf im Klimaschrank aufbewahrt.Eine Wirkung von 100 % bedeutet, dass keine Zecke fertile Eier gelegt hat.

Bei diesem Test zeigen z. B. die folgenden Verbindungen der Herstellungsbeispiele überlegene Wirksamkeit gegenüber dem Stand der Technik: siehe Tabelle

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration (in **µg/Tier)**.

Die Wirkstofflösung wird in das Abdomen *(Boophilus microplus)* injiziert, die Tiere werden in Schalen überführt und in einem klimatisierten Raum aufbewahrt.

Nach 7 Tagen wird die Wirkung in % bestimmt. Die Wirkungskontrolle erfolgt auf Ablage fertiler Eier. Dabei bedeutet 100%, dass keine Zecke fertile Eier gelegt hat.

Bei diesem Test zeigen z. B. die folgenden Verbindungen der Herstellungsbeispiele überlegene Wirksamkeit gegenüber dem Stand der Technik: siehe Tabelle

| Substanz | Struktur | Tierart | Konzentration | % Wirkung dat | |
|---|---|---|---|---|---|
| Bsp-Nr. Ik-136 bekannt aus WO 2010/051926 A2 | 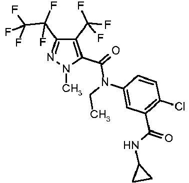 | MYZUPE | 20 g/ha | 0 | 6dat |
| | | TETRUR | 20 g/ha | 0 | 6dat |
| | | PHAECO | 0,8 ppm | 0 | 7dat |
| | | NILALU | 20 g/ha | 0 | 7dat |
| | | CTECFE | 0,8 ppm | 30 | 2dat |
| | | BOOPMI | 0,032 µg/Tier | 0 | 7dat |
| | | | 20 ppm | 0 | 7dat |
| Bsp.-Nr. 6 erfindungsgemäß | 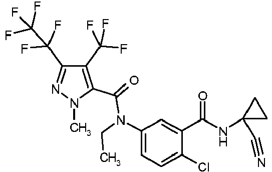 | MYZUPE | 20 g/ha | 70 | 6dat |
| | | TETRUR | 20 g/ha | 100 | 6dat |
| | | PHAECO | 0,8 ppm | 100 | 7dat |
| | | NILALU | 20 g/ha | 90 | 7dat |
| | | CTECFE | 0,8 ppm | 80 | 2dat |
| | | BOOPMI | 0,032µg/Tier | 70 | 7dat |
| | | | 20 ppm | 100 | 7dat |
| Substanz | Struktur | Tierart | Konzentration | % Wirkung dat | |
| Bsp-Nr. Ik-132 bekannt aus WO 2010/051926 A2 | 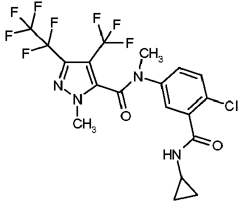 | MYZUPE | 100 g/ha | 0 | 6dat |
| | | PHAECO | 0,8 ppm | 5 | 7dat |
| | | PLUTMA | 20 ppm | 5 | 7dat |
| | | SPODFR | 20 ppm | 50 | 7dat |
| | | TETRUR | 4 ppm | 30 | 7dat |
| | | CTECFE | 0,8 ppm | 30 | 2dat |
| | | BOOPMI | 0,032µg/Tier | 0 | 7dat |
| | | | 20 ppm | 30 | 7dat |
| Bsp.-Nr. 8 erfindungsgemäß | 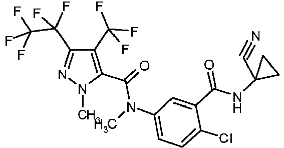 | MYZUPE | 100 g/ha | 100 | 6dat |
| | | PHAECO | 0,8 ppm | 100 | 7dat |
| | | PLUTMA | 20 ppm | 100 | 7dat |
| | | SPODFR | 20 ppm | 100 | 7dat |
| | | TETRUR | 4 ppm | 95 | 7dat |
| | | CTECFE | 0,8 ppm | 90 | 2dat |
| | | BOOPMI | 0,032µg/Tier | 100 | 7dat |
| | | | 20 ppm | 100 | 7dat |
| Substanz | Struktur | Tierart | Konzentration | % Wirkung dat | |
| Bsp-Nr. Ik-1 bekannt aus WO 2010/051926 A2 | 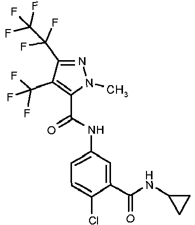 | SPODFR | 20 ppm | 20 | 7dat |
| | | HELIAR | 20 ppm | 45 | 7dat |
| | | TETRUR | 4 ppm | 40 | 7dat |
| | | FRANOC | 20 g/ha | 0 | 7dat |
| | | NILALU | 500 g/ha | 0 | 7dat |
| | | CTECFE | 0,8 ppm | 0 | 2dat |
| Bsp-Nr. Ik-296 bekannt aus WO 2010/051926 A2 | 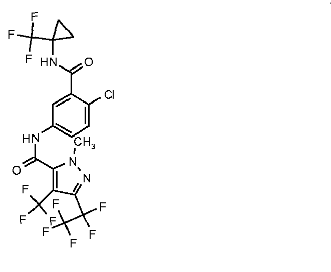 | MYZUPE | 100 g/ha | 0 | 6dat |
| | | FRANOC | 500 g/ha | 60 | 7dat |
| | | LIRITR | 500 g/ha | 0 | 7dat |
| | | NILALU | 500 g/ha | 0 | 7dat |
| Bsp-Nr. Ik-47 bekannt aus WO 2010/051926 A2 | 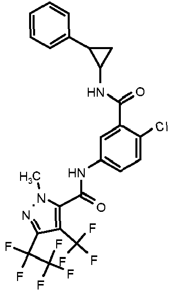 | MYZUPE | 500 g/ha | 0 | 6dat |
| | | PHAECO | 20 g/ha | 0 | 7dat |
| | | PLUTMA | 20 ppm | 0 | 7dat |
| | | SPODFR | 20 ppm | 0 | 7dat |
| | | HELIAR | 20 ppm | 0 | 7dat |
| | | TETRUR | 20 ppm | 30 | 7dat |
| | | FRANOC | 500 g/ha | 0 | 7dat |
| | | NILALU | 500 g/ha | 0 | 7dat |
| | | CTECFE | 20 ppm | 50 | 2dat |
| | | MUSCDO | 100 ppm | 0 | 2dat |
| | | BOOPMI | 0,8 µg/Tier | 50 | 7dat |
| Bsp-Nr. Ik-286 bekannt aus WO 2010/051926 A2 | 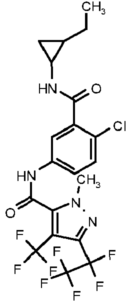 | MYZUPE | 20 g/ha | 0 | 6dat |
| | | PHAECO | 100 g/ha | 0 | 7dat |
| | | CTECFE | 4 ppm | 0 | 2dat |
| | | BOOPMI | 100 ppm | 40 | 7dat |
| | | | 0,16 µg/Tier | 20 | 7dat |
| Bsp-Nr. Ik-279 bekannt aus WO 2010/051926 A2 | 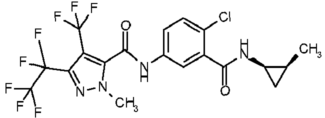 | MYZUPE | 20 g/ha | 0 | 6dat |
| | | PHAECO | 20 g/ha | 0 | 7dat |
| | | SPODFR | 100 g/ha | 50 | 7dat |
| | | TETRUR | 4 ppm | 0 | 7dat |
| Bsp-Nr. Ik-280 bekannt aus WO 2010/051926 A2 | 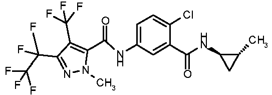 | PLUTMA | 20 ppm | 65 | 7dat |
| | | PHAECO | 4 ppm | 0 | 7dat |
| | | SPODFR | 20 ppm | 40 | 7dat |
| | | HELIAR | 100 ppm | 0 | 7dat |
| | | NILALU | 100 g/ha | 0 | 7dat |
| Bsp.-Nr. 10 erfindungsgemäß | 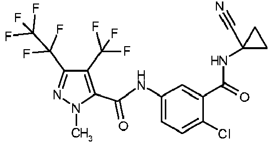 | MYZUPE | 500 g/ha | 100 | 6dat |
| | | | 100 g/ha | 100 | 6dat |
| | | | 20 g/ha | 80 | 6dat |
| | | PHAECO | 100 g/ha | 100 | 7dat |
| | | | 20 g/ha | 100 | 7dat |
| | | | 4 ppm | 100 | 7dat |
| | | SPODFR | 100 g/ha | 100 | 7dat |
| | | | 20 ppm | 100 | 7dat |
| | | PLUTMA | 20 ppm | 100 | 7dat |
| | | HELIAR | 100 ppm | 100 | 7dat |
| | | | 20 ppm | 100 | 7dat |
| | | TETRUR | 20 ppm | 100 | 7dat |
| | | | 4 ppm | 100 | 7dat |
| | | FRANOC | 500 g/ha | 100 | 7dat |
| | | | 20 g/ha | 90 | 7dat |
| | | LIRITR | 500 g/ha | 100 | 7dat |
| | | NILALU | 500 g/ha | 100 | 7dat |
| | | | 100 g/ha | 100 | 7dat |
| | | CTECFE | 20 ppm | 100 | 2dat |
| | | | 4 ppm | 98 | 2dat |
| | | | 0,8 ppm | 90 | 2dat |
| | | MUSCDO | 100 ppm | 100 | 2dat |
| | | BOOPMI | 0,8 µg/Tier | 100 | 7dat |
| | | | 0,16 µg/Tier | 80 | 7dat |
| | | | 100 ppm | 100 | 7dat |
| Substanz | Struktur | Tierart | Konzentration | % Wirkung dat | |
| Bsp-Nr. Ik-175 bekannt aus WO 2010/051926 A2 | 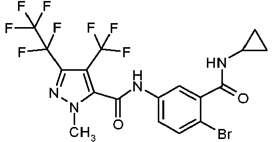 | MYZUPE | 100 g/ha | 0 | 6dat |
| | | PHAECO | 4 ppm | 10 | 7dat |
| | | NILALU | 500 g/ha | 0 | 7dat |
| Bsp.-Nr. 14 erfindungsgemäß | 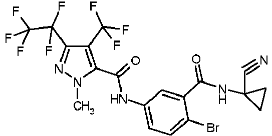 | MYZUPE | 100 g/ha | 100 | 6dat |
| | | PHAECO | 4 ppm | 100 | 7dat |
| | | NILALU | 500 g/ha | 80 | 4dat |
| Substanz | Struktur | Tierart | Konzentration | % Wirkung dat | |
| Bsp-Nr. Ik-155 bekannt aus WO 2010/051926 A2 | 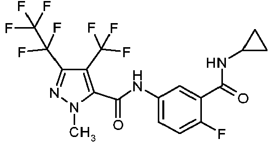 | MYZUPE | 100 g/ha | 0 | 6dat |
| | | TETRUR | 100 g/ha | 0 | 6dat |
| Bsp.-Nr. 14 erfindungsgemäß | 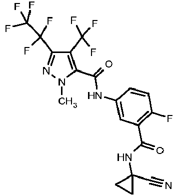 | MYZUPE | 100 g/ha | 100 | 6dat |
| | | TETRUR | 100 g/ha | 100 | 6dat |

## Patentansprüche

1. Verbindungen der allgemeinen Formel (I), 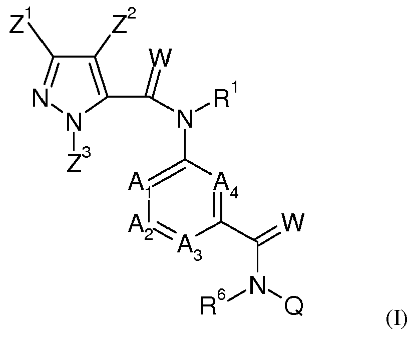 in denen
R¹ für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyan-C₁-C₂-alkyl, Aryl(C₁-C₃)-alkyl, Heteroaryl(C₁-C₃)-alkyl,
die chemische Gruppierung
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
², R³, R⁴ und R⁵ Rnabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₁-C₆alkylamino, *N*-C₁-C₆-Alkylaminocarbonyl, *N*-C₃-C₆-Cycloalkylaminocarbonyl oder (C₁-C₃-Alkoxy)carbonyl, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
W für Sauerstoff oder Schwefel steht;
R⁶ für Wasserstoff, gegebenenfalls substituiertes C₁-C₆-Alkyl, Aryl(C₁-C₃)-alkyl, Heteroaryl(C₁-C₃)-alkyl, C₂-C₆-Alkenyl, C₂-C₆-Alkinyl, C₃-C₆-Cycloalkyl, (C₁-C₃-Alkyl)-C₃-C₆-cycloalkyl und (C₃-C₆-Cycloalkyl)-C₁-C₃-alkyl, C₁-C₆-Alkylcarbonyl, C₁-C₆-Alkoxycarbonyl steht;
Q für 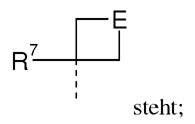
E für eine Bindung, -CH₂-, S, SO, SO₂, -S-CH₂-, -SO-CH₂-, -SO₂-CH₂-, -CH₂-S-CH₂-,-CH₂-SO-CH₂-, -CH₂-SO₂-CH₂-, -S-CH₂-CH₂-, -SO-CH₂-CH₂-, -SO₂-CH₂-CH₂-, -NR⁶-CH₂-, -CH₂-NR⁶-CH₂- steht;
R⁷ für Cyano und C(=S)NH₂ steht;
Z¹ für ein gegebenenfalls substituiertes C₁-C₆-Halogenalkyl und C₃-C₆-Cycloalkyl, C₃-C₆-Halogencycloalkyl, und
Z² für Halogen, Cyano, Nitro oder ein gegebenenfalls substituiertes C₁-C₆-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, und
Z³ für Wasserstoff oder ein gegebenenfalls substituiertes C₁-C₆-Alkyl, C₁-C₄-Alkenyl, C₁-C₄-Alkinyl, C₁-C₆-Halogenalkyl stehen.

2. Verbindungen der allgemeinen Formel (I) gemäß Anspruch 1 in denen
R¹ für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemische Gruppierung
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R² und R⁵ unabhängig voneinander für Wasserstoff, Methyl, Fluor und Chlor stehen und
R³ und R⁴ unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlordifluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl stehen; wobei
W für Sauerstoff steht,
R⁶ für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
Q für 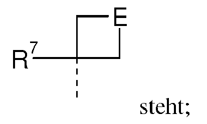
E für eine Bindung und -CH₂- steht;
R⁷ für Cyano und C(=S)NH₂ steht;
Z¹ für Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyancyclopropyl, 1-Trifluormehtyl-cyclopropyl, Cyclobutyl und 2,2-Difluor-1-methyl-cyclopropyl, und
Z² für Halogen, Cyan, Nitro, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylthio, Methylsulfinyl, Methylsulfonyl, Ethylthio, Ethylsulfinyl, Ethylsulfonyl, Trifluormethylthio, Trifluormethylsulfinyl, Trifluormethylsulfonyl und
Z³ für Wasserstoff, Methyl, Ethyl, n-Propyl, Isopropyl, n-Butyl, Isobutyl, s-Butyl, t-Butyl, Ethenyl, 1-Propenyl, 2-Propenyl, Ethinyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl stehen.

3. Verbindungen der allgemeinen Formel (I) gemäß einem der Ansprüche 1 bis 2, in denen
Z¹ für Trifluormethyl, 1-Chlor-cyclopropyl, 1-Fluor-cyclopropyl oder Pentafluorethyl steht,
Z² für Trifluormethyl, Nitro, Methylthio, Methylsulfinyl, Methylsulfonyl, Fluor, Chlor, Brom oder Iod steht,
Z³ für Methyl, Ethyl, n-Propyl oder Wasserstoff steht,
R¹ für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht,
A¹ A² und A⁴ für CH steht,
A₃ für CR⁴ und
R⁴ für Fluor, Chlor, Brom oder Iod steht
R⁶ für Wasserstoff, Methyl, Ethyl, n-Propyl, Isopropyl, n-Butyl, Isobutyl, s-Butyl, t-Butyl steht,
W für Sauerstoff steht und
Q für 1-Cyancyclopropyl steht.

4. Verbindungen der allgemeinen Formel (I) gemäß einem der Ansprüche 1 bis 3, in denen
Z¹ für Trifluormethyl, 1-Chlor-cyclopropyl, 1-Fluor-cyclopropyl oder Pentafluorethyl steht,
Z² für Trifluormethyl, Chlor oder steht,
Z³ für Methyl steht,
R¹ für Wasserstoff, Methyl, Ethyl, steht,
A¹ A² und A⁴ für CH stehen,
A₃ für CR⁴ und
R⁴ für Chlor steht
R⁶ für Wasserstoff, Methyl, Ethyl steht,
W für Sauerstoff steht und
Q für 1-Cyancyclopropyl steht.

5. Verwendung von Verbindungen der allgemeinen Formel (I) gemäß einem der Ansprüche 1 bis 4 zur Bekämpfung von Insekten, Spinnentieren und Nematoden zum Schutz von Pflanzen.

6. Pharmazeutische Zusammensetzungen, enthaltend wenigstens eine Verbindung gemäß einem der Ansprüche 1 bis 4.

7. Verbindungen gemäß einem der Ansprüche 1 bis 4 zur Verwendung als Arzneimittel.

8. Verfahren zur Herstellung von Pflanzenschutzmitteln enthaltend Verbindungen der allgemeinen Formel (I) gemäß einem der Ansprüche 1 bis 4 sowie übliche Streckmittel und/oder oberflächenaktive Substanzen.

9. Verfahren zum Bekämpfen von Schädlingen auf Pflanzen oder Saatgut, **dadurch gekennzeichnet, dass** man eine Verbindung der allgemeinen Formel (I) gemäß einem der Ansprüche 1 bis 4 auf die Schädlinge und/oder ihren Lebensraum einwirken lässt.

10. Verwendung von Verbindungen der allgemeinen Formel (I) gemäß einem der Ansprüche 1 bis 4 zum Schutz des Vermehrungsmaterials von Pflanzen, insbesondere von Saatgut.

11. Verbindungen der allgemeinen Formeln (IVa) und (IVb), 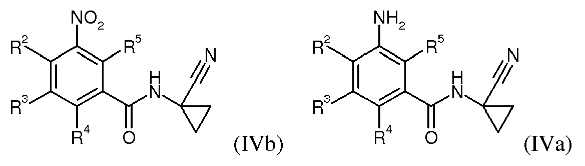 in denen
R², R³, R⁴ und R⁵ unabhängig voneinander für Wasserstoff, Halogen, Cyan, Nitro, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-C₁-C₆alkylamino, *N*-C₁-C₆-Alkylaminocarbonyl, *N*-C₃-C₆-Cycloalkylaminocarbonyl oder (C₁-C₃-Alkoxy)carbonyl stehen.

## Claims

1. Compounds of the general formula (I) 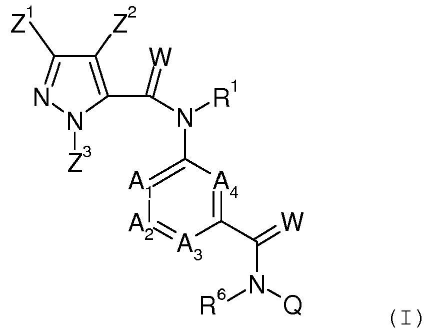 in which
R¹ represents hydrogen, optionally substituted C₁-C₆-alkyl, C₂-C₆-alkenyl, C₂-C₆-alkynyl, C₃-C₇-cycloalkyl, C₁-C₆-alkylcarbonyl, C₁-C₆-alkoxycarbonyl, cyano-C₁-C₂-alkyl, aryl-(C₁-C₃)-alkyl, heteroaryl-(C₁-C₃)-alkyl,
the chemical grouping
A₁ represents CR² or nitrogen,
A₂ represents CR³ or nitrogen,
A₃ represents CR⁴ or nitrogen and
A₄ represents CR⁵ or nitrogen,
but where not more than three of the chemical groupings A₁ to A₄ simultaneously represent nitrogen;
R², R³, R⁴ and R⁵ independently of one another represent hydrogen, halogen, CN, NO₂, optionally substituted C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylamino, *N*,*N*-di-C₁-C₆alkylamino, *N*-C₁-C₆-alkylaminocarbonyl, *N*-C₃-C₆-cycloalkylaminocarbonyl or (C₁-C₃-alkoxy)carbonyl;
if none of the groupings A₂ and A₃ represents nitrogen, R³ and R⁴ together with the carbon atom to which they are attached may form a 5- or 6-membered ring which contains 0, 1 or 2 nitrogen atoms and/or 0 or 1 oxygen atom and/or 0 or 1 sulphur atom, or
if none of the groupings A₁ and A₂ represents nitrogen, R² and R³ together with the carbon atom to which they are attached may form a 6-membered ring which contains 0, 1 or 2 nitrogen atoms;
W represents oxygen or sulphur;
R⁶ represents hydrogen, optionally substituted C₁-C₆-alkyl, aryl-(C₁-C₃)-alkyl, heteroaryl-(C₁-C₃)-alkyl, C₂-C₆-alkenyl, C₂-C₆-alkynyl, C₃-C₆-cycloalkyl, (C₁-C₃-alkyl) -C₃-C₆-cycloalkyl and (C₃-C₆-cycloalkyl) -C₁-C₃-alkyl, C₁-C₆-alkylcarbonyl, C₁-C₆-alkoxycarbonyl;
Q represents 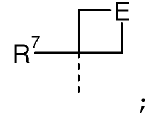
E represents a bond, -CH₂-, S, SO, SO₂, -S-CH₂-,-SO-CH₂-, -SO₂₋CH2-_{,} -CH₂-S-CH₂-, -CH₂-SO-CH₂-,-CH₂SO₂-CH₂-, -S-CH₂-CH₂-, -SO-CH₂-CH₂-, -SO₂-CH₂-CH₂-, -NR⁶-CH₂-, -CH₂-NR⁶-CH₂-;
R⁷ represents cyano or C(=S)NH₂;
Z¹ represents optionally substituted C₁-C₆-haloalkyl or C₃-C₆-cycloalkyl, C₃-C₆-halocycloalkyl, and
Z² represents halogen, cyano, nitro or optionally substituted C₁-C₆-haloalkyl, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, and
Z³ represents hydrogen or optionally substituted C₁-C₆-alkyl, C₁-C₄-alkenyl, C₁-C₄-alkynyl, C₁-C₆-haloalkyl.

2. Compounds of the general formula (I) according to Claim 1 in which
R¹ represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl;
the chemical grouping
A₁ represents CR² or nitrogen,
A₂ represents CR³ or nitrogen,
A₃ represents CR⁴ or nitrogen and
A₄ represents CR⁵ or nitrogen, but where not more than three of the chemical groupings A₁ to A₄ simultaneously represent nitrogen;
R² and R⁵ independently of one another represent hydrogen, methyl, fluorine or chlorine and
R³ and R⁴ independently of one another represent hydrogen, fluorine, chlorine, bromine, CN, NO₂, methyl, ethyl, fluoromethyl, difluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, methylsulphonyl, methylsulphinyl, trifluoromethylsulphonyl, trifluoromethylsulphinyl; where
W represents oxygen,
R⁶ represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl;
Q represents 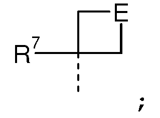
E represents a bond or -CH₂-;
R⁷ represents cyano or C(=S)NH₂;
Z¹ represents difluoromethyl, trichloromethyl, chlorodifluoromethyl, dichlorofluoromethyl, trifluoromethyl, bromodichloromethyl, chloromethyl, bromomethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-t-butyl, heptafluoro-n-propyl, heptafluoroisopropyl, nonafluoro-n-butyl, cyclopropyl, 1-chlorocyclopropyl, 1-fluorocyclopropyl, 1-bromocyclopropyl, 1-cyanocyclopropyl, 1-trifluoromethylcyclopropyl, cyclobutyl or 2,2-difluoro-1-methylcyclopropyl, and
Z² represents halogen, cyano, nitro, difluoromethyl, trichloromethyl, chlorodifluoromethyl, dichlorofluoromethyl, trifluoromethyl, bromodichloromethyl, chloromethyl, bromomethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-t-butyl, heptafluoro-n-propyl, heptafluoroisopropyl, nonafluoro-n-butyl, methylthio, methylsulphinyl, methylsulphonyl, ethylthio, ethylsulphinyl, ethylsulphonyl, trifluoromethylthio, trifluoromethylsulphinyl, trifluoromethylsulphonyl, and
Z³ represents hydrogen, methyl, ethyl, n-propyl, isopropyl, n-butyl, isobutyl, s-butyl, t-butyl, ethenyl, 1-propenyl, 2-propenyl, ethynyl, 1-propynyl, 1-butynyl, difluoromethyl, trichloromethyl, chlorodifluoromethyl, dichlorofluoromethyl, trifluoromethyl, chloromethyl, bromomethyl, 1-fluoroethyl, 1-fluoro-1-methylethyl, 2-fluoroethyl, 2,2-difluoroethyl, 2,2,2-trifluoroethyl.

3. Compounds of the general formula (I) according to Claim 1 or 2 in which
Z¹ represents trifluoromethyl, 1-chlorocyclopropyl, 1-fluorocyclopropyl or pentafluoroethyl,
Z² represents trifluoromethyl, nitro, methylthio, methylsulphinyl, methylsulphonyl, fluorine, chlorine, bromine or iodine,
Z³ represents methyl, ethyl, n-propyl or hydrogen,
R¹ represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl,
A¹, A² and A⁴ represent CH,
A₃ represents CR⁴ and
R⁴ represents fluorine, chlorine, bromine or iodine,
R⁶ represents hydrogen, methyl, ethyl, n-propyl, isopropyl, n-butyl, isobutyl, s-butyl, t-butyl,
W represents oxygen and
Q represents 1-cyanocyclopropyl.

4. Compounds of the general formula (I) according to any of Claims 1 to 3 in which
Z¹ represents trifluoromethyl, 1-chlorocyclopropyl, 1-fluorocyclopropyl or pentafluoroethyl,
Z² represents trifluoromethyl, chlorine or,
Z³ represents methyl,
R¹ represents hydrogen, methyl, ethyl,
A¹, A² and A⁴ represent CH,
A₃ represents CR⁴ and
R⁴ represents chlorine,
R⁶ represents hydrogen, methyl, ethyl,
W represents oxygen and
Q represents 1-cyanocyclopropyl.

5. Use of compounds of the general formula (I) according to any of Claims 1 to 4 for controlling insects, arachnids and nematodes in order to protect plants.

6. Pharmaceutical compositions comprising at least one compound according to any of Claims 1 to 4.

7. Compounds according to any of Claims 1 to 4 for use as medicaments.

8. Process for preparing crop protection compositions comprising compounds of the general formula (I) according to any of Claims 1 to 4 and customary extenders and/or surfactants.

9. Method for controlling pests on plants or seed, **characterized in that** a compound of the general formula (I) according to any of Claims 1 to 4 is allowed to act on the pests and/or their habitat.

10. Use of compounds of the general formula (I) according to any of Claims 1 to 4 for protecting the propagation material of plants, in particular seed.

11. Compounds of the general formulae (IVa) and (IVb), 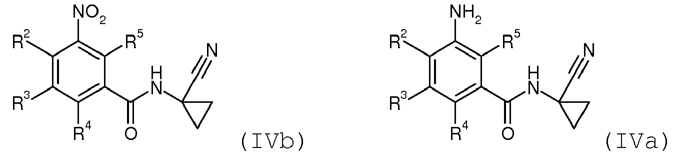 in which
R², R³, R⁴ and R⁵ independently of one another represent hydrogen, halogen, cyano, nitro, optionally substituted C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylamino, *N*,*N*-di-C₁-C₆-alkylamino, *N*-C₁-C₆-alkylaminocarbonyl, *N*-C₃-C₆-cycloalkylaminocarbonyl or (C₁-C₃-alkoxy)carbonyl.

## Revendications

1. Composés de formule générale (I) 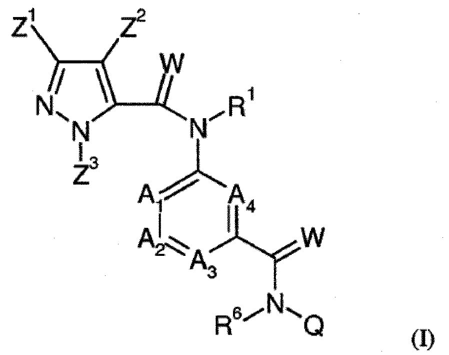 dans lesquels
R¹ représente hydrogène, alkyle en C₁-C₆ éventuellement substitué, alcényle en C₂-C₆, alcynyle en C₂-C₆, cycloalkyle en C₃-C₇, alkylcarbonyle en C₁-C₆, alcoxycarbonyle en C₁-C₆, cyanoalkyle en C₁-C₂, aryl-alkyle en C₁-C₃, hétéroaryl-alkyle en C₁-C₃,
le groupement chimique
A₁ représente CR² ou azote,
A₂ représente CR³ ou azote,
A₃ représente CR⁴ ou azote,
A₄ représente CR⁵ ou azote,
pas plus de trois des groupements chimiques A₁ à A₄ représentant toutefois simultanément l'azote ;
R², R³, R⁴ et R⁵ représentent indépendamment les uns des autres hydrogène, halogène, CN, NO₂ alkyle en C₁-C₆ éventuellement substitué, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylamino en C₁-C₆, *N*,*N*-di-alkylamino en C₁-C₆, *N*-alkylaminocarbonyle en C₁-C₆, *N*-cycloalkylaminocarbonyle en C₃-C₆ ou (alcoxy en C₁-C₃)carbonyle ;
lorsqu'aucun des groupements A₂ et A₃ ne représente l'azote, R³ et R⁴ pouvant former ensemble avec l'atome de carbone auquel ils sont reliés un cycle à 5 ou 6 éléments, qui contient 0, 1 ou 2 atomes d'azote et/ou 0 ou 1 atome d'oxygène et/ou 0 ou 1 atome de soufre, ou lorsqu'aucun des groupements A₁ et A₂ ne représente l'azote, R² et R³ pouvant former ensemble avec l'atome de carbone auquel ils sont reliés un cycle à 6 éléments, qui contient 0, 1 ou 2 atomes d'azote ;
W représente oxygène ou soufre ;
R⁶ représente hydrogène, alkyle en C₁-C₆ éventuellement substitué, aryl-alkyle en C₁-C₃, hétéroaryl-alkyle en C₁-C₃, alcényle en C₂-C₆, alcynyle en C₂-C₆, cycloalkyle en C₃-C₆, (alkyle en C₁-C₃)-cycloalkyle en C₃-C₆ et (cycloalkyle en C₃-C₆)-alkyle en C₁-C₃, alkylcarbonyle en C₁-C₆, alcoxycarbonyle en C₁-C₆ ; 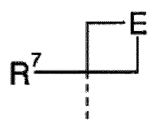
Q représente ;
E représente une liaison, -CH₂-, S, SO, SO₂, -S-CH₂-, -SO-CH₂-, -SO₂-CH₂-, -CH₂-S-CH₂-, -CH₂-SO-CH₂-, -CH₂-SO₂-CH₂-, -S-CH₂-CH₂-, -SO-CH₂-CH₂-, -SO₂-CH₂-CH₂- -NR⁶-CH₂-, -CH₂-NR⁶-CH₂- ;
R⁷ représente cyano et C(=S)NH₂ ;
Z¹ représente halogénoalkyle en C₁-C₆ éventuellement substitué et cycloalkyle en C₃-C₆, halogénocycloalkyle en C₃-C₆, et
Z² représente halogène, cyano, nitro ou un halogénoalkyle en C₁-C₆ éventuellement substitué, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, et
Z³ représente hydrogène ou un alkyle en C₁-C₆ éventuellement substitué, alcényle en C₁-C₄, alcynyle en C₁-C₄, halogénoalkyle en C₁-C₆.

2. Composés de formule générale (I) selon la revendication 1, dans lesquels
R¹ représente hydrogène, méthyle, éthyle, n-propyle, isopropyle, n-butyle, isobutyle, s-butyle, t-butyle, méthoxyméthyle, éthoxyméthyle, propoxyméthyle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 4-chloro-pyrid-3-yl-méthyle ;
le groupement chimique
A₁ représente CR² ou azote,
A₂ représente CR³ ou azote,
A₃ représente CR⁴ ou azote,
A₄ représente CR⁵ ou azote,
pas plus de trois des groupements chimiques A₁ à A₄ représentant toutefois simultanément l'azote ;
R² et R⁵ représentent indépendamment l'un de l'autre hydrogène, méthyle, fluor et chlore, et
R³ et R⁴ représentent indépendamment l'un de l'autre hydrogène, fluor, chlore, brome, CN, NO₂, méthyle, éthyle, fluorométhyle, difluorométhyle, trifluorométhyle, 2,2,2-triflluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, méthylsulfonyle, méthylsulfinyle, trifluorométhylsulfonyle, trifluorométhylsulfinyle ;
W représente oxygène,
R⁶ représente hydrogène, méthyle, éthyle, n-propyle, isopropyle, n-butyle, isobutyle, s-butyle, t-butyle, méthoxyméthyle, éthoxyméthyle, propoxyméthyle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 4-chloro-pyrid-3-yl-méthyle ; 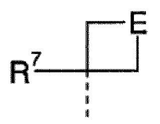
Q représente ;
E représente une liaison et -CH₂- ;
R⁷ représente cyano et C(=S)NH₂ ;
Z¹ représente difluorométhyle, trichlorométhyle, chlorodifluorométhyle, dichlorofluorométhyle, trifluorométhyle, bromodichlorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle pentafluoro-t-butyle, heptafluoro-n-propyle, heptafluoro-isopropyle, nonafluoro-n-butyle, cyclopropyle, 1-chlorocyclopropyle, 1-fluorocyclopropyle, 1-bromocyclopropyle, 1-cyano-cyclopropyle, 1-trifluorométhyl-cyclopropyle, cyclobutyle et 2,2-difluoro-1-méthyl-cyclopropyle, et
Z² représente halogène, cyano, nitro, difluorométhyle, trichlorométhyle, chlorodifluorométhyle, dichlorofluorométhyle, trifluorométhyle, bromodichlorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-t-butyle, heptafluoro-n-propyle, heptafluoro-isopropyle, nonafluoro-n-butyle, méthylthio, méthylsulfinyle, méthylsulfonyle, éthylthio, éthylsulfinyle, éthylsulfonyle, trifluorométhylthio, trifluorométhylsulfinyle, trifluorométhylsulfonyle, et
Z³ représente hydrogène, méthyle, éthyle, n-propyle, isopropyle, n-butyle, isobutyle, s-butyle, t-butyle, éthényle, 1-propényle, 2-propényle, éthynyle, 1-propynyle, 1-butynyle, difluorométhyle, trichlorométhyle, chlorodifluorométhyle, dichlorofluorométhyle, trifluorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle.

3. Composés de formule générale (I) selon l'une quelconque des revendications 1 à 2, dans lesquels Z¹ représente trifluorométhyle, 1-chloro-cyclopropyle, 1-fluoro-cyclopropyle ou pentafluoroéthyle,
Z² représente trifluorométhyle, nitro, méthylthio, méthylsulfinyle, méthylsulfonyle, fluor, chlore, brome ou iode,
Z³ représente méthyle, éthyle, n-propyle ou hydrogène,
R¹ représente hydrogène, méthyle, éthyle, n-propyle, isopropyle, n-butyle, i-butyle, s-butyle, t-butyle, méthoxyméthyle, éthoxyméthyle, propoxyméthyle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 4-chloro-pyrid-3-yl-méthyle,
A¹, A² et A⁴ représentent CH,
A₃ représente CR⁴ et
R⁴ représente fluor, chlore, brome ou iode,
R⁶ représente hydrogène, méthyle, éthyle, n-propyle, isopropyle, n-butyle, isobutyle, s-butyle, t-butyle,
W représente oxygène et
Q représente 1-cyanocyclopropyle.

4. Composés de formule générale (I) selon l'une quelconque des revendications 1 à 3, dans lesquels Z¹ représente trifluorométhyle, 1-chloro-cyclopropyle, 1-fluoro-cyclopropyle ou pentafluoroéthyle,
Z² représente trifluorométhyle, chlore ou,
Z³ représente méthyle,
R¹ représente hydrogène, méthyle, éthyle,
A¹, A² et A⁴ représentent CH,
A₃ représente CR⁴ et
R⁴ représente chlore,
R⁶ représente hydrogène, méthyle, éthyle,
W représente oxygène et
Q représente 1-cyanocyclopropyle.

5. Utilisation de composés de formule générale (I) selon l'une quelconque des revendications 1 à 4 pour lutter contre les insectes, les araignées et les nématodes pour la protection de plantes.

6. Compositions pharmaceutiques, contenant au moins un composé selon l'une quelconque des revendications 1 à 4.

7. Composés selon l'une quelconque des revendications 1 à 4, destinés à une utilisation en tant que médicament.

8. Procédé de fabrication d'agents phytosanitaires contenant des composés de formule générale (I) selon l'une quelconque des revendications 1 à 4, ainsi que des extendeurs et/ou substances tensioactives usuels.

9. Procédé de lutte contre des animaux nuisibles sur des plantes ou des graines, **caractérisé en ce qu'**un composé de formule générale (I) selon l'une quelconque des revendications 1 à 4 est laissé agir sur les animaux nuisibles et/ou leur habitat.

10. Utilisation de composés de formule générale (I) selon l'une quelconque des revendications 1 à 4 pour la protection du matériel de reproduction de plantes, notamment de graines.

11. Composés de formules générales (IVa) et (IVb) 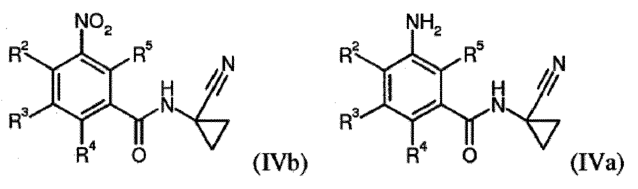 dans lesquels
R², R³, R⁴ et R⁵ représentent indépendamment les uns des autres hydrogène, halogène, cyano, nitro, alkyle en C₁-C₆ éventuellement substitué, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylamino en C₁-C₆, *N*,*N*-di-alkylamino en C₁-C₆, *N*-alkylaminocarbonyle en C₁-C₆, *N*-cycloalkylaminocarbonyle en C₃-C₆ ou (alcoxy en C₁-C₃)carbonyle.
